(19) Europäisches Patentamt
European Patent Office
Office européen des brevets

(11) Veröffentlichungsnummer: **0 145 956**
**A1**

(12) # EUROPÄISCHE PATENTANMELDUNG

(21) Anmeldenummer: **84113706.0**

(22) Anmeldetag: **13.11.84**

(51) Int. Cl.[4]: **C 07 D 211/90**, C 07 D 401/06, C 07 D 401/12, C 07 D 405/12, C 07 D 417/12, A 61 K 31/44

(30) Priorität: **16.11.83 CH 6154/83**
**13.07.84 CH 3425/84**

(43) Veröffentlichungstag der Anmeldung: **26.06.85**
**Patentblatt 85/26**

(84) Benannte Vertragsstaaten: **AT BE CH DE FR GB IT LI LU NL SE**

(71) Anmelder: **CIBA-GEIGY AG, Postfach, CH-4002 Basel (CH)**

(72) Erfinder: **Kamber, Bruno, Dr., Sonnenweg 9, CH-4144 Arlesheim (CH)**
Erfinder: **Leutert, Thomas, Dr., Quidumweg 3, CH-4143 Dornach (CH)**
Erfinder: **Kühnis, Hans, Dr., Lange Gasse 26, CH-4052 Basel (CH)**
Erfinder: **Eichenberger, Kurt, Dr., Neuberweg 36, CH-4106 Therwil (CH)**

(74) Vertreter: **Zumstein, Fritz jun., Dr. et al, Dr. F. Zumstein sen. Dr. E. Assmann Dr. R. Koenigsberger Dipl.-Ing. F. Klingseisen Dr. F. Zumstein jun. Bräuhausstrasse 4, D-8000 München 2 (DE)**

(54) **Neue Amid-Verbindungen.**

(57) Verbindung der Formel I

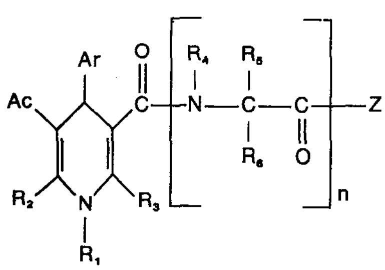

(I),

worin n für 1, 2 oder 3 steht, Ar einen carbocyclischen oder heterocyclischen Arylrest bedeutet, Ac den Acylrest einer Säure darstellt, Z für einen Rest $-OR_7$ oder $-NR_8R_9$ steht, $R_1$ Wasserstoff, unsubstituiertes oder substituiertes Niederalkyl, einen carbocyclischen oder heterocyclischen Arylrest oder freies, veräthertes oder verestertes Hydroxy bedeutet, $R_2$ und $R_3$ unabhängig voneinander für Wasserstoff, unsubstituiertes oder substituiertes Niederalkyl, Formyl oder funktionell abgewandeltes Formyl, Carboxy oder funktionell abgewandeltes Carboxy, einen carbocyclischen oder heterocyclischen Arylrest oder unsubstituiertes, mono- oder disubstituiertes Amino stehen, $R_4$ Wasserstoff oder Niederalkyl bedeutet, $R_5$ und $R_6$ unabhängig voneinander Wasserstoff, unsubstituiertes oder substituiertes Niederalkyl oder einen carbocyclischen oder heterocyclischen Arylrest darstellen, $R_7$, $R_8$ und $R_9$ unabhängig voneinander Wasserstoff, unsubstituiertes oder substituiertes Alkyl oder einen carbocyclischen oder heterocyclischen Arylrest bedeuten, worin $R_1$ und $R_2$ zusammen oder $R_1$ und $R_3$ zusammen unsubstituiertes oder substituiertes Niederalkylen, worin ein Kohlenstoffatom gegebenenfalls durch ein Heteroatom ersetzt ist, bedeuten können, worin $R_4$ und $R_5$ zusammen, ebenso wie $R_5$ und $R_6$ zusammen und/oder $R_8$ und $R_9$ zusammen, unabhängig voneinander unsubstituiertes oder substituiertes Niederalkylen, worin ein Kohlenstoffatom durch ein Heteroatom ersetzt sein kann, bedeuten können, optische Isomere von Verbindungen der Formel I, Mischungen dieser optischen Isomere und Salze von solchen Verbindungen, die eine salzbildende Gruppierung aufweisen, zeichnen sich durch cardiovaskuläre, insbesondere blutdrucksenkende Eigenschaften aus. Sie werden in an sich bekannter Weise hergestellt.

EP 0 145 956 A1

0145956

CIBA-GEIGY AG
Basel (Schweiz)

4-14657/1+2/+

## Neue Amid-Verbindungen

Die Erfindung betrifft neue Amid-Verbindungen und deren Salze, Verfahren zu ihrer Herstellung, pharmazeutische Präparate, die solche Verbindungen enthalten, und ihre Verwendung zur Herstellung von pharmazeutischen Präparaten oder als pharmakologisch wirksame Verbindungen.

Die Erfindung betrifft insbesondere substituierte 3-Carbamoyl-1,4-Dihydropyridin-Verbindungen der Formel I

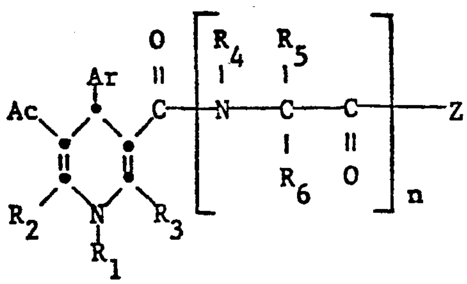

(I),

worin n für 1, 2 oder 3 steht, Ar einen carbocyclischen oder heterocyclischen Arylrest bedeutet, Ac den Acylrest einer Säure darstellt, Z für einen Rest $-OR_7$ oder $-NR_8R_9$ steht, $R_1$ Wasserstoff, unsubstituiertes oder substituiertes Niederalkyl, einen carbocyclischen oder heterocyclischen Arylrest oder freies, veräthertes oder verestertes Hydroxy bedeutet, $R_2$ und $R_3$ unabhängig voneinander für Wasserstoff, unsubstituiertes oder substituiertes Niederalkyl, Formyl oder funktionell abgewandeltes Formyl, Carboxy oder funktionell abgewandeltes Carboxy, einen carbocyclischen oder heterocyclischen Arylrest oder unsubstituiertes, mono- oder disubstituiertes Amino stehen, $R_4$ Wasserstoff oder Niederalkyl bedeutet, $R_5$ und $R_6$

unabhängig voneinander Wasserstoff, unsubstituiertes oder substituiertes Niederalkyl oder einen carbocyclischen oder heterocyclischen Arylrest darstellen, $R_7$, $R_8$ und $R_9$ unabhängig voneinander Wasserstoff, unsubstituiertes oder substituiertes Alkyl oder einen carbocyclischen oder heterocyclischen Arylrest bedeuten, worin $R_1$ und $R_2$ zusammen oder $R_1$ und $R_3$ zusammen unsubstituiertes oder substituiertes Niederalkylen, worin ein Kohlenstoffatom gegebenenfalls durch ein Heteroatom ersetzt ist, bedeuten können, worin $R_4$ und $R_5$ zusammen, ebenso wie $R_5$ und $R_6$ zusammen und/oder $R_8$ und $R_9$ zusammen, unabhängig voneinander unsubstituiertes oder substituiertes Niederalkylen, worin ein Kohlenstoffatom durch ein Heteroatom ersetzt sein kann, bedeuten können, optische Isomere von Verbindungen der Formel I, Mischungen dieser optischen Isomere und Salze von solchen Verbindungen, die eine salzbildende Gruppierung aufweisen, Verfahren zur Herstellung dieser Verbindungen, solche Verbindungen enthaltende pharmazeutische Mittel und ihre Verwendung zur Herstellung von pharmazeutischen Präparaten oder als pharmakologisch wirksame Verbindungen.

Die vor-, sowie nachstehend verwendeten Definitionen haben, sofern nicht besonders definiert, die folgenden Bedeutungen:

Der Ausdruck "nieder" bedeutet, dass entsprechend definierte Gruppen oder Verbindungen bis und mit 7, vorzugsweise bis und mit 4 Kohlenstoffatome enthalten.

Substituierte Reste können einen oder mehrere, gleiche oder verschiedene Substituenten enthalten; diese können irgendeine geeignete Stellung substituieren.

In der obigen Formel I steht n in erster Linie für 1, kann aber auch 2 oder 3 bedeuten. Ist n 2 oder 3, so sind die dann mehrfach auftretenden Reste $R_4$, $R_5$ und $R_6$ jeweils unabhängig voneinander.

Ein carbocyclischer oder heterocyclischer Arylrest ist in erster Linie ein entsprechender monocyclischer Rest, kann jedoch auch ein bi- oder polycyclischer, carbocyclischer oder heterocyclischer Rest mit aromatischen Eigenschaften sein.

Carbocyclische Reste dieser Art sind insbesondere Phenyl, ferner Naphthyl, z.B. 1- oder 2-Naphthyl.

Heterocyclische Arylreste sind vorzugsweise entsprechende monocyclische Reste, können jedoch auch entsprechende bi- oder polycyclische Reste sein, wobei letztere aus mehreren heterocyclischen Ringen, oder aus einem oder mehreren heterocyclischen Ringen mit einem oder mehreren ankondensierten carbocyclischen Ringen, insbesondere einem oder mehreren ankondensierten Benzoringen bestehen können. Die üblicherweise vorhandenen heterocyclischen Reste, die vorzugsweise aus fünf oder sechs Ringgliedern bestehen, können bis zu vier, gleiche oder verschiedene Hetero-, insbesondere Stickstoff-, Sauerstoff- und/oder Schwefelatome, vorzugsweise ein, zwei, drei oder vier Stickstoffatome, ein Sauerstoff- oder Schwefelatom, oder ein oder zwei Stickstoffatome zusammen mit einem Sauerstoff- oder Schwefelatom als Ringglieder enthalten. Sie sind mit dem 4-Ringkohlenstoffatom des 1,4-Dihydro-pyridinrings üblicherweise über ein Ringkohlenstoffatom gebunden.

Monocyclische fünfgliedrige Heteroarylreste sind z.B. entsprechende monoaza-, diaza-, triaza-, tetraza-, monooxa-, monothia-, oxaza-, oxadiaza-, thiaza- oder thiadiazacyclische Reste, wie Pyrryl-, Pyrazolyl-, Imidazolyl-, Triazolyl-, Tetrazolyl-, Furyl-, Thienyl-, Isoxazolyl-, Oxazolyl-, Oxadiazolyl-, Isothiazolyl-, Thiazolyl- oder Thiadiazolylreste, während monocyclische, sechsgliedrige Heteroarylreste z.B. entsprechende monoaza-, diaza- oder triazacyclische Reste, wie

Pyridyl-, Pyridazinyl-, Pyrimidinyl-, Pyrazinyl- oder Triazinylreste sind. Bicyclische Heteroarylreste sind insbesondere monocyclische Heteroarylreste mit ankondensiertem Benzoring; der Heteroring kann fünf- oder sechsgliedrig sein, wobei der fünfgliedrige Heteroarylrest z.B. einen monoaza-, diaza-, monooxa-, monothia-, oxaza-, thiaza-, oxadiaza- oder thiadiazacyclischen Rest, und der sechsgliedrige Heteroarylrest z.B. einen monoaza- oder einen diazacyclischen Heteroarylrest darstellt. Solche bicyclischen Reste sind z.B. Indolyl-, Isoindolyl-, Benzimidazolyl-, Benzofuranyl-, Benzothienyl-, Benzthiazolyl-, Benzoxadiazolyl-, Benzthiadiazolyl-, Chinolinyl- oder Isochinolinylreste.

Die carbocyclischen und heterocyclischen Arylreste Ar können unsubstituiert oder substituiert sein, wobei in erster Linie Ringkohlenstoffatome, aber auch Ringstickstoffatome substituiert sein können. Substituenten von Ringkohlenstoffatomen sind u.a. gegebenenfalls substituierte Kohlenwasserstoffreste, wie entsprechende aliphatische, cycloaliphatische, aromatische oder araliphatische Kohlenwasserstoffreste, wie Niederalkyl, Niederalkenyl, Niederalkinyl, Niederalkylen, Cycloalkyl, Cycloalkylniederalkyl, Phenyl, Phenylniederalkyl, Phenylniederalkylthio und/oder Phenylniederalkyloxy, wobei Substituenten von solchen Kohlenwasserstoffresten, insbesondere von Niederalkyl, Phenyl, Phenylniederalkyl, Phenylniederalkylthio und/oder Phenylniederalkoxy, z.B. gegebenenfalls verätherte oder veresterte Hydroxygruppen, wie Hydroxy, Niederalkoxy, Niederalkenyloxy, Niederalkinyloxy, Niederalkylendioxy, Niederalkanoyloxy oder Halogen, und/oder gegebenenfalls funktionell abgewandeltes Carboxy, wie Carboxy, verestertes Carboxy, z.B. Niederalkoxycarbonyl, amidiertes Carboxy, wie Carbamoyl, N-Niederalkyl-carbamoyl oder N,N-Diniederalkyl-carbamoyl, oder Cyan sein können. Cyclische Substituenten, insbesondere Phenyl, können zudem auch Niederalkyl, das gegebenenfalls, z.B. wie angegeben,substituiert sein kann, als Substituenten enthalten. Weitere Substituenten von Arylresten Ar sind z.B. gegebenenfalls verätherte oder veresterte Hydroxygruppen, wie Hydroxy, Niederalkoxy, Halogeniederalkoxy, Niederalkenyloxy, Halogenniederalkenyloxy, Niederalkinyloxy, Niederalkylendioxy, Niederalkanoyloxy oder Halogen Nitro, gegebenenfalls substituiertes Amino, wie Amino, Niederalkylamino, Diniederalkylamino, N-Niederalkyl-N-phenylniederalkylamino, Niederalkylenamino, Oxaniederalkylenamino, Thianiederalkylenamino oder Azaniederalkylenamino, wobei der Aza-Stickstoff unsubstituiert oder z.B. durch gegebenenfalls, z.B. wie oben beschrieben, substituiertes Niederalkyl, Phenyl oder Phenylniederalkyl substituiert sein kann, oder Acylamino, z.B. Niederalkanoylamino, Azido, Acyl, wie Niederalkanoyl, oder gegebenenfalls funktionell abgewandeltes Carboxy, wie Carboxy, verestertes Carboxy, z.B. Niederalkoxycarbonyl oder amidiertes Carboxy, wie Carbamoyl, N-Niederalkyl-carbamoyl oder N,N-Diniederalkyl-carbamoyl, oder Cyan, gegebenenfalls funktionell abgewandeltes Sulfo, wie Sulfo oder Aminosulfonyl, und/oder veräthertes Mercapto, das gegebenenfalls oxidiert sein kann, wie Niederalkylthio, Niederalkylsulfinyl oder Niederalkylsulfonyl. Substituenten von Ringstickstoffatomen sind in erster Linie die obengenannten, gegebenenfalls substituierten Kohlenwasserstoffreste, wie Niederalkyl oder Niederalkoxycarbonyl, ferner Hydroxy oder Oxido.

Heterocyclische Arylreste Ar können z.B. je nach Art der Substituenten in verschiedenartigen tautomeren Formen vorliegen.

Partiell gesättigte fünf- oder sechsgliedrige Monoaza-, Diaza- oder Triaza-Heteroarylreste sind z.B. Dihydropyrrolyl, Dihydroimidazolyl, Dihydropyridinyl, Dihydropyrimidinyl oder Tetrahydrotriazinyl. Beispiele für solche Reste mit Oxo-Substituenten sind 2-Oxo-1,2-dihydro-1-pyrrolinyl, 2-Oxo-1,2-dihydro-4-pyrimidinyl oder 5,6-Dioxo-1,4,5,6-Tetrahydro-1,2,4-Triazin-3-yl. Ein ganz gesättigter Heteroarylrest entspricht Heterocyclyl. Fünf- oder sechsgliedriges Monoaza- oder Diazaheterocyclyl ist z.B. Pyrrolidinyl, Piperidinyl, Imidazolidinyl oder Piperazinyl. Beispiele für solche Reste mit Oxo-Substituenten sind 2-Pyrrolidinon-1-yl, 2-Piperidinon-1-yl und vor allem 2-Imidazolidinon-1-yl.

Ein Acylrest Ac kann den entsprechenden Rest einer Carbonsäure, insbesondere Niederalkanoyl, ferner unsubstituiertes oder substituiertes, z.B. Niederalkyl, Niederalkoxy, Nitro und/oder Halogen enthaltendes Benzoyl, oder einer organischen Sulfonsäure,insbesondere Niederalkylsulfonyl oder unsubstituiertes oder substituiertes, z.B. Niederalkyl, Niederalkoxy, Nitro und/oder Halogen enthaltendes Phenylsulfonyl darstellen. Acylreste Ac stehen jedoch in erster Linie für Acylreste von Monoestern, ferner auch von Monoamiden der Kohlensäure, wie vor allem unsubstituiertes oder substituiertes, z.B. freies oder veräthertes Hydroxy, wie Niederalkoxy, oder unsubstituiertes oder substituiertes Amino, wie z.B. oben beschriebenes Amino, und in erster Linie disubstituiertes Amino, wie Diniederalkylamino, N-Niederalkyl-N-phenylniederalkyl-amino, oder gegebenenfalls durch Sauerstoff, Schwefel oder unsubstituierten oder, z.B. durch Niederalkyl, substituierten Stickstoff unterbrochenes Niederalkylenamino, oder einen unsubstituierten oder z.B. durch Niederalkyl, Niederalkoxy, Nitro und/oder Halogen substituierten Phenyl-, Thienyl-, Furyl-, Pyrryl- oder Pyridylrest enthaltendes Niederalkoxycarbonyl, ferner Niederalkenyloxy- oder Niederalkinyloxycarbonyl, aber auch N-unsubstituiertes oder N-mono- oder N,N-disubstituiertes Carbamoyl, wie N-Niederalkylcarbamoyl, N,N-Diniederalkylcarbamoyl, N-Hydroxycarbamoyl, oder gegebenenfalls im Niederalkylenteil durch Sauerstoff, Schwefel oder unsubstituierten oder, z.B. durch Niederalkyl, substituierten Stickstoff unterbrochenes N,N-Niederalkylen-carbamoyl. Weiterhin umfassen Acylreste Ac auch Acylreste von cyclischen Kohlensäurederivaten, wie z.B. 5-Tetrazolyl oder unsubstituiertes oder durch Niederalkyl oder Phenyl substituiertes 4,5-Dihydro-2-oxazolyl oder 5,6-Dihydro-4H-1,3-oxazin-2-yl.

Alkyl enthält vorzugsweise 1 bis 12 Kohlenstoffatome, z.B. n-Decyl, und ist in erster Linie Niederalkyl. Niederalkyl ist z.B. Methyl, Aethyl, n-Propyl, Isopropyl, n-Butyl, Isobutyl, sek-Butyl oder tert-Butyl, ferner n-Pentyl, Isopentyl, Neopentyl, n-Hexyl, Isohexyl oder n-Heptyl.

Ein substituierter Niederalkylrest $R_1$ enthält als Substituenten z.B. unsubstituiertes oder insbesondere mono- oder disubstituiertes Amino,

wie Niederalkylamino, Diniederalkylamino, N-Niederalkyl-N-phenyl-niederalkyl-amino oder gegebenenfalls durch Sauerstoff, Schwefel oder unsubstituierten oder, z.B. durch Niederalkyl oder Niederalkanoyl, substituierten Stickstoff unterbrochenes Niederalkylenamino, wobei ein solcher Substituent vorzugsweise durch mindestens 2 Kohlenstoffatome vom Ringstickstoffatom getrennt ist. Er kann ferner z.B. durch Carboxy oder funktionell abgewandeltes Carboxy, z.B. Carboxymethyl, oder durch Niederalkoxy, wobei solches Niederalkoxy bevorzugt ist, das seinerseits durch Niederalkoxy substituiert ist, z.B. 2-Methoxyäthoxymethyl, oder durch Phenyl, das seinerseits gegebenenfalls Niederalkyl, Niederalkoxy, Halogen und/oder Nitro als Substituenten enthält, z.B. Benzyl, oder durch N-Pyrrolyl, N-Imidazolyl, N-Pyrazolyl, N-Indolyl oder N-Isoindolyl, z.B. N-Imidazolylmethyl, substituiert sein.

Funktionell abgewandeltes Carboxy ist z.B. verestertes Carboxy, z.B. Niederalkoxycarbonyl, oder amidiertes Carboxy, wie z.B. Carbamoyl, N-Niederalkyl-carbamoyl oder N,N-Diniederalkyl-carbamoyl, oder Cyan. Funktionell abgewandeltes Carboxy als Bedeutung von $R_2$ und $R_3$ ist vorzugsweise Cyan.

Bei einem Amino-niederalkylrest $R_5$, der durch funktionell abgewandeltes Carboxy substituiert ist, ist letzteres vorzugsweise Carbamoyl oder C-Amidino [$-C(=NH)-NH_2$].

Phenylniederalkyl ist z.B. Benzyl oder 1- oder 2-Phenyläthyl. Es kann im Phenylring z.B. durch freies oder veräthertes Hydroxy, Niederalkyl, Nitro, Amino, Niederalkylamino, Diniederalkylamino, Niederalkanoylamino und/oder Halogen substituiert sein.

Niederalkylen enthält z.B. zwei bis sieben, insbesondere drei bis fünf Kettenkohlenstoffatome und ist u.a. 1,3-Propylen oder 1,4-Butylen. Niederalkylen, worin ein Kohlenstoffatom durch ein Heteroatom ersetzt ist, ist z.B. Oxa-, Thia- oder Azaniederalkylen. Niederalkylen, Aza-, Oxa- oder Thianiederalkylen, gebildet aus den Resten $R_8$ und $R_9$ zusammen, ist vorzugsweise $C_2$-$C_7$-Niederalkylen, $C_3$-$C_4$-Aza-, $C_3$-$C_4$-Oxa- oder

$C_3$-$C_4$-Thianiederalkylen, insbesondere $C_4$-$C_5$-Niederalkylen, $C_4$-Oxa- oder $C_4$-Azaniederalkylen, in erster Linie 1,5-Pentylen, 3-Oxa- oder 3-Aza-1,5-Pentylen und vor allem 3-Aza-1,5-Pentylen.

Niederalkylen, Aza-, Oxa- oder Thianiederalkylen, gebildet aus den Resten $R_5$ und $R_6$ zusammen, bedeutet vorzugsweise Niederalkylen mit 2 bis 5 Kettenkohlenstoffatomen, Aza-, Oxa- oder Thianiederalkylen mit jeweils 3 oder 4 Kettenkohlenstoffatomen, insbesondere $C_2$-$C_5$-Niederalkylen oder $C_4$-Azaniederalkylen und in erster Linie 1,5-Pentylen.

Niederalkylen, Aza-, Oxa- oder Thianiederalkylen, gebildet aus den Resten $R_4$ und $R_5$ zusammen, ist vorzugsweise $C_3$-$C_5$-Niederalkylen, $C_2$-$C_4$-Oxa- oder $C_2$-$C_4$-Thianiederalkylen, insbesondere 1,3-Propylen, 2-Oxa- oder 2-Thia-1,3-Propylen und in erster Linie 1,3-Propylen.

Niederalkylen, worin ein Kohlenstoffatom gegebenenfalls durch ein Heteroatom ersetzt ist, gebildet aus den Resten $R_1$ und $R_2$ zusammen oder $R_1$ und $R_3$ zusammen, bedeutet vorzugsweise $C_3$-$C_5$-Niederalkylen, worin das mit dem C2- bzw. C6-Kohlenstoffatom des 1,4-Dihydropyridinrings direkt verbundene Kohlenstoffatom gegebenenfalls durch ein Sauerstoff-, Schwefel- oder ein Stickstoffatom, das durch Wasserstoff oder Niederalkyl substituiert ist, ersetzt ist, insbesondere $C_3$-$C_5$-Niederalkylen oder 3-Oxa-, 3-Thia- oder 3-Aza-1,3-Propylen - die Numerierung der C-Atome beginnt dabei am Stickstoffatom -, und vor allem 1,2-Aethylen, 1,3-Propylen oder 1,4-Butylen.

Niederalkylen-, Oxa-, Thia- und Azaniederalkylen-Reste können z.B. durch Hydroxy, Niederalkoxy, Amino, Niederalkylamino, Diniederalkylamino, Halogen, Carboxy und/oder funktionell abgewandeltes Carboxy, am Aza-Stickstoff auch durch Niederalkanoyl, Phenyl oder Phenylniederalkyl oder insbesondere durch Niederalkyl, substituiert sein. Reste wie z.B. $C_3$-$C_4$-Aza-, $C_4$-Oxa- oder $C_3$-$C_4$-Thianiederalkylen enthalten die angegebene Zahl von Kohlenstoffatomen und zusätzlich das angeführte Heteroatom.

Naphthyl kann z.B. 1- oder 2-Naphthyl sein.

Pyrryl ist z.B. 2- oder 3-Pyrryl, Pyrazolyl z.B. 3- oder 4-Pyrazolyl, Imidazolyl z.B. 2- oder 4-Imidazolyl, Triazolyl z.B. 1,3,5-1H-Triazol-2-yl oder 1,3,4-Triazol-2-yl, und Tetrazolyl,z.B. 1,2,3,4-1H-Tetrazol-5-yl, während Furyl 2- oder 3-Furyl und Thienyl 2- oder 3-Thienyl bedeuten. Isoxazolyl ist z.B. 3-Isoxazolyl, Oxazolyl z.B. 2- oder 4-Oxazolyl, Oxadiazolyl z.B. 1,3,4-Oxadiazol-2-yl, Isothiazolyl z.B. 3-Isothiazolyl, Thiazolyl 2- oder 4-Thiazolyl, und Thiadiazolyl z.B. 1,3,4-Thiadiazol-2-yl. Benzoxadiazolyl und Benzthiadiazolyl sind vorzugsweise 2,1,3-Benzoxadiazol-4-yl und 2,1,3-Benzthiadiazol-4-yl.

Pyridyl ist 2-, 3- oder 4-Pyridyl, Pyridazinyl z.B. 3-Pyridazinyl, Pyrimidinyl ist 2-, 4- oder 5-Pyrimidinyl, Pyrazinyl ist 2-Pyrazinyl, und Triazinyl ist z.B. 1,3,5-Triazin-2-yl.

Indolyl ist z.B. 2-, 3- oder 5-Indolyl, Isoindolyl z.B. 1-Isoindolyl, Benzimidazolyl z.B. 2- oder 5-Benzimidazolyl, Benzofuranyl z.B. 2- oder 3-Benzofuranyl, Benzothienyl z.B. 3-Benzothienyl, Benzthiazolyl, z.B. 2-Benzthiazolyl, Chinolinyl z.B. 2- oder 4-Chinolinyl, und Isochinolinyl z.B. 1-Isochinolinyl.

Niederalkenyl ist z.B. Allyl oder Methallyl, und Niederalkinyl z.B. Propargyl.

Cycloalkyl weist vorzugsweise 5 bis 7 Ringkohlenstoffatome auf und ist z.B. Cyclopentyl oder Cyclohexyl, während Cycloalkylniederalkyl z.B. Cyclopropylmethyl, Cyclopentylmethyl oder Cyclohexylmethyl sein kann.

Veräthertes Hydroxy ist in erster Linie Niederalkoxy, ferner z.B. Phenylniederalkoxy, Aryloxy oder Heteroaryloxy.

Acyloxy ist z.B. durch einen der oben definierten Acylreste Ac substituiertes Hydroxy, vorzugsweise Benzoyloxy, das gegebenenfalls durch Hydroxy, Niederalkoxy, Halogen, Niederalkyl und/oder Nitro substituiert ist, und in erster Linie Niederalkanoyloxy.

Niederalkoxy steht z.B. für Methoxy, Aethoxy, n-Propyloxy, Isopropyloxy, n-Butyloxy, Isobutyloxy oder tert-Butyloxy.

In einem Niederalkoxyniederalkoxyrest ist die terminale Niederalkoxygruppe vorzugsweise durch mehr als ein Kohlenstoffatom vom Verknüpfungskohlenstoffatom getrennt; solche Reste sind z.B. 2-Methoxy-äthoxy oder 2-Aethoxy-äthoxy.

Niederalkenyloxy ist z.B. Allyloxy oder Methallyloxy, und Halogenniederalkenyloxy, das ein oder mehrere Halogenatome enthalten kann, wobei letztere vorzugsweise eine Atomnummer bis und mit 35 aufweisen und besonders für Fluor und Chlor stehen, ist z.B. 1,2-Dichlor-vinyloxy.

In einem Halogen-niederalkoxyrest können ein oder mehrere Halogenatome, die vorzugsweise eine Atomnummer bis und mit 35 aufweisen und besonders für Fluor oder Chlor stehen, vorhanden sein; solche Reste sind z.B. 1,1,2-Trifluor-2-chlor-äthoxy oder vorzugsweise Difluormethoxy.

Niederalkinyloxy ist z.B. Propargyloxy, während Niederalkylendioxy z.B. Methylendioxy oder Aethylendioxy bedeutet.

Niederalkanoyloxy ist z.B. Acetyloxy, Propionyloxy oder Pivaloyloxy.

Halogensubstituiertes Niederalkyl ist z.B. Trifluormethyl, 1,1,2-Trifluor-2-chlor-äthyl oder Chlormethyl.

Halogen hat vorzugsweise eine Atomnummer bis und mit 35 und ist insbesondern Fluor oder Chlor, ferner Brom, kann jedoch auch Iod sein.

Niederalkoxycarbonyl ist z.B. Methoxycarbonyl, Aethoxycarbonyl, n-Propyloxycarbonyl, Isopropyloxycarbonyl, n-Butyloxycarbonyl, Isobutyloxycarbonyl oder tert—Butyloxycarbonyl.

N-Niederalkyl-carbamoyl ist z.B. N-Methyl-carbamoyl oder N-Aethylcarbamoyl, während N,N-Diniederalkyl-carbamoyl z.B. N,N-Dimethylcarbamoyl oder N,N-Diäthyl-carbamoyl ist.

Niederalkylamino ist z.B. N-Methylamino, N-Aethylamino, N-n-Propylamino oder N-Isopropylamino.

Diniederalkylamino ist z.B. N,N-Dimethylamino, N-Aethyl-N-methylamino oder N,N-Diäthylamino, während N-Niederalkyl-N-phenylniederalkylamino z.B. N-Benzyl-N-methylamino oder N-Methyl-N-(2-phenyläthyl)-amino darstellt.

Niederalkylenamino enthält z.B. 2 bis 7, vorzugsweise 4 oder 5 Ringkohlenstoffatome und ist z.B. Pyrrolidino oder Piperidino, während Oxaniederalkylenamino z.B. Morpholino, wie 4-Morpholino (3-Oxa-1,5-pentylenamino), Thianiederalkylenamino, z.B. Thiomorpholino, wie 4-Thiomorpholino, und gegebenenfalls azasubstituiertes Azaniederalkylenamino, z.B. Piperazino, wie 1-Piperazino (3-Aza-1,5-pentylenamino), 4-Methylpiperazino, 4-Phenyl-piperazino, 4-Benzyl-piperazino oder 4-(2-Phenyläthyl)-piperazino darstellen kann.

Acylamino ist z.B. durch einen der oben definierten Acylreste Ac substituiertes Amino, vorzugsweise Benzoylamino, das gegebenenfalls durch Hydroxy, Niederalkoxy, Halogen, Niederalkyl und/oder Nitro substituiert ist, und in erster Linie Niederalkanoylamino.

Niederalkanoylamino ist z.B. Acetylamino oder Propionylamino.

Acyl als solches ist z.B. einer der oben definierten Acylreste Ac, vorzugsweise Benzoyl, das gegebenenfalls wie für Benzoylamino angegeben substituiert ist, und in erster Linie Niederalkanoyl, z.B. Formyl, Acetyl, Propionyl oder Pivaloyl . Weitere bevorzugte Acylreste sind

Furanylcarbonyl, Thienylcarbonyl, Pyrrylcarbonyl oder Pyridinylcarbonyl, und besonders Furan-2-yl-carbonyl.

Veräthertes Mercapto ist in erster Linie Niederalkylthio aber auch z.B. Phenylthio. Oxidiertes veräthertes Mercapto ist vorzugsweise Niederalkylsulfinyl oder Niederalkylsulfonyl. Niederalkylthio ist z.B. Methylthio, Aethylthio, n-Propylthio oder Isopropylthio, während Niederalkylsulfinyl z.B. Methylsulfinyl, und Niederalkylsulfonyl z.B. Methylsulfonyl oder Aethylsulfonyl bedeuten.

In einer Aminoniederalkylgruppe $R_1$ ist Amino vorzugsweise durch mindestens 2 Kohlenstoffatome vom Verknüpfungskohlenstoffatom getrennt; solche Reste sind in erster Linie 2-disubstituiertes Amino-niederalkyl, wie 2-Diniederalkylaminoäthyl, z.B. 2-Dimethylaminoäthyl oder 2-Diäthylaminoäthyl, 2-Niederalkylenaminoäthyl, z.B. 2-Pyrrolidino-äthyl oder 2-Piperidino-äthyl, 2-(4-Morpholino)-äthyl, oder 2-(4-Niederalkylpiperazino)-äthyl, z.B. 2-(4-Methylpiperazino)-äthyl.

In einem substituierten Niederalkoxycarbonyl ist der Substituent üblicherweise durch mindestens 2, vorzugsweise durch 2 oder 3 Kohlenstoffatome vom Sauerstoff getrennt. Solche Reste sind z.B. Hydroxyniederalkoxycarbonyl, wie 2-Hydroxyäthoxycarbonyl oder 2,3-Dihydroxypropyloxycarbonyl, Niederalkoxy-niederalkoxycarbonyl, z.B. 2-Methoxyäthoxycarbonyl, Diniederalkylamino-niederalkoxycarbonyl, z.B. 2-Dimethylaminoäthoxycarbonyl, 2-Diäthylaminoäthoxycarbonyl oder 3-Dimethylaminopropyloxycarbonyl, Niederalkylenamino-niederalkoxycarbonyl, z.B. 2-Pyrrolidinoäthoxycarbonyl oder 2-Piperidinoäthoxycarbonyl, Morpholino-niederalkoxycarbonyl, z.B. 2-(4-Morpholino)-äthoxycarbonyl, oder 4-Niederalkyl-piperazino-niederalkoxycarbonyl, z.B. 2-(4-Methylpiperazino)-äthoxy carbonyl.

Phenylniederalkoxycarbonyl ist z.B. Benzyloxycarbonyl oder 2-Phenyläthoxy-carbonyl.

N,N-Niederalkylen-carbamoyl ist z.B. Pyrrolidinocarbonyl oder Piperidinocarbonyl, wobei entsprechende Reste, in welchen der Niederalkylenteil durch Sauerstoff, Schwefel oder unsubstituierten oder substituierten Stickstoff unterbrochen ist, z.B. 4-Morpholinocarbonyl, 4-Thiomorpholino-carbonyl, 1-Piperazinocarbonyl oder 4-Methyl-1-piperazino-carbonyl bedeuten.

Salze von erfindungsgemässen Verbindungen sind in erster Linie pharmazeutisch verwendbare, nicht-toxische Salze, wie diejenigen von Verbindungen der Formel I mit sauren Gruppen, z.B. mit einer freien Carboxy- oder Sulfogruppe. Solche Salze sind in erster Linie Metall- oder Ammoniumsalze, wie Alkalimetall- und Erdalkalimetall-, z.B. Natrium-, Kalium-, Magnesium- oder Calciumsalze, sowie Ammoniumsalze mit Ammoniak oder geeigneten organischen Aminen, wobei in erster Linie aliphatische, cycloaliphatische, cycloaliphatisch-aliphatische oder araliphatische primäre, sekundäre oder tertiäre Mono-, Di- oder Polyamine, sowie heterocyclische Basen für die Salzbildung in Frage kommen, wie Niederalkylamine, z.B. Triäthylamin, Hydroxyniederalkylamine, z.B. 2-Hydroxyäthylamin, Bis-(2-hydroxyäthyl)-amin oder Tris-(2-hydroxyäthyl)-amin, basische aliphatische Ester von Carbonsäuren, z.B. 4-Aminobenzoesäure-2-diäthylaminoäthylester, Niederalkylenamine, z.B. 1-Aethyl-piperidin, Cycloalkylamine, z.B. Dicyclohexylamin, oder Benzylamine, z.B. N,N'-Dibenzyl-äthylendiamin, ferner Basen vom Pyridintyp, z.B. Pyridin, Collidin oder Chinolin. Verbindungen der Formel I mit basischen Gruppen können Säureadditionssalze, z.B. mit anorganischen Säuren, wie Salzsäure, Schwefelsäure oder Phosphorsäure, oder mit geeigneten organischen Carbon- oder Sulfonsäuren, z.B. Trifluoressigsäure, sowie mit Aminosäuren, wie Arginin und Lysin, bilden. Bei Anwesenheit von mehreren sauren oder basischen Gruppen können Mono- oder Polysalze gebildet werden. Verbindungen der Formel I mit einer sauren, z.B. freien Carboxygruppe, und einer freien basischen, z.B. einer Aminogruppe, können auch in Form von inneren Salzen, d.h. in zwitterionischer Form vorliegen, oder es kann ein Teil des Moleküls als inneres Salz, und ein anderer Teil als normales Salz vorliegen.

Zur Isolierung oder Reinigung können auch pharmazeutisch ungeeignete Salze Verwendung finden. Zur therapeutischen Anwendung gelangen nur die pharmazeutisch verwendbaren, nicht toxischen Salze, die deshalb bevorzugt werden.

Die Verbindungen der Formel I und Salze von solchen Verbindungen mit salzbildenden Eigenschaften besitzen wertvolle pharmakologische Eigenschaften. Sie zeigen vor allem starke antihypertensive und hypotensive Wirkungen, die sich beispielsweise an der wachen, renalhypertonischen Ratte in Anlehnung an die von Byrom und Wilson, J.Physiol. (London), Bd. 93, S. 301 (1938), Gerold et al., Helv. Physiol. Pharmacol. Acta, Bd. 24, S. 58 (1966), und Goldblatt et al., J.Exptl. Med., Bd. 59, S. 347 (1934) beschriebene Testanordnung in Dosen ab etwa 0.3 mg/kg p.o. nachweisen lassen. Die blutdrucksenkende Wirkung kann z.B. auch an der narkotisierten Katze bei intravenöser Verabreichung von Dosen ab etwa 0.001 mg/kg gezeigt werden. Ferner besitzen die Verbindungen langdauernde pharmakologische Wirkungen, z.B. eine langdauernde hypotensive bzw. vasodilatierende Wirkung, die sich z.B. am wachen Hund und am narkotisierten Hund bei intravenöser Verabreichung von Dosen ab etwa 0.01 mg/kg nachweisen lassen. Die Verbindungen der Formel I zeichnen sich auch ferner dadurch aus, dass sie negativ inotrope und negativ chronotrope Effekte aufweisen; erstere lassen sich beispielsweise in vitro durch eine Hemmung der Kontraktionskraft des isolierten, linken, mit konstanter Frequenz stimulierten Meerschweinchenvorhofs ab einer Konzentration von etwa 10 nmol/l und letztere am rechten, spontan schlagenden Meerschweinchenvorhof ab einer Konzentration von etwa 1 nmol/l nachweisen. Dabei zeichnen sich die Verbindungen der Formel I dadurch aus, dass sie relativ zur negativ chronotropen Wirkung nur schwache negative inotrope Effekte aufweisen. Weiterhin bewirken die Verbindungen der Formel I, wie bei Calciumantagonisten allgemein üblich, beispielsweise am narkotisierten Hund infolge der reflektorischen Cardio-Stimulation einen Anstieg der Herzfrequenz, der jedoch verzögert eintritt und begrenzt ist. Bei der narkotisierten Ratte wird die Herzfrequenz durch die Verabreichung von Verbindungen der Formel I trotz eines Anstiegs der Aktivität des Sympathikus verringert.

Die Verbindungen der Formel I wirken weiterhin coronardilatierend, wie sich z.B. am isolierten, perfundierten Meerschweinchenherzen nach Langendorff in Konzentrationen ab etwa 0.1 nmol/l nachweisen lässt. Dabei ist hervorzuheben, dass die Verbindungen der Formel I relativ zur coronardilatierenden Wirkung nur schwache negativ inotrope Effekte aufweisen. Die Affinität der Verbindungen zu Calciumkanälen kann im Nitrendipin-Rezeptorbindungstest in vitro nachgewiesen werden. $^3$H-Nitrendipin wird spezifisch von Membran-Präparaten des Meerschweinchen-Herzens gebunden. Die $IC_{50}$-Werte, welche die Konzentration der Testverbindung angeben, die nötig ist, um die spezifische Bindung von $^3$H-Nitrendipin um 50% zu verringern, liegen bei den erfindungsgemässen Verbindungen bei etwa 0.1 nmol/l oder darüber. Ferner weisen elektrophysiologische Untersuchungen an Verbindungen der Formel I, z.B. die Registrierung der interzellulären Ableitung des Aktionspotentials am isolierten Papillarmuskel des Meerschweinchenherzens, auf eine starke Hemmung des $Ca^{2+}$-Ionen-Einstroms hin. So tritt z.B. am teilweise durch $K^+$-Ionen depolarisierten Papillarmuskel-Präparat in Konzentrationen von 10 nmol/l oder weniger eine Hemmung des "slow potentials" um 50% ein. Bei Untersuchungen am nicht-depolarisierten Präparat verlängern Verbindungen der Formel I bemerkenswerterweise die Dauer des Aktionspotentials in Konzentrationen ab 10 nmol/l, möglicherweise infolge einer zusätzlichen Wirkung auf den Kaliumausstrom. Weiterhin wird auch in vitro am isoliert perfundierten Mesenterialgefässbett der Ratte bevorzugt die durch Kalium- oder Calcium-Ionen (ab etwa 0.1 nmol/l) induzierte gegenüber der durch Noradrenalin (ab etwa 10 nmol/l) induzierten Vasokonstriktion durch Verbindungen der Formel I gehemmt. Die Verbindungen der Formel I zeichnen sich ferner durch verhältnismässig raschen Eintritt der pharmakologischen Wirkungen, chemische Beständigkeit und, im Vergleich zu den cardiovaskulären Wirkungen, eine relativ niedrige Toxizität aus.

Die Verbindungen der Formel I und Salze von solchen Verbindungen mit salzbildenden Eigenschaften können daher z.B. als Antihypertensiva und Coronardilatatoren zur Behandlung von cardiovasculären Krankheitszuständen, wie vor allem Angina pectoris und Hypertonie, ferner z.B. Arrhythmien, cerebralen und peripheren Durchblutungsstörungen, pulmonaler Hypertonie, Migräne, Gefässspasmen oder Herzinsuffizienz und weiteren Indikationen, wie z.B. Arteriosklerose, Spasmen der glatten Muskulatur, wie Bronchien, Uterus, Darm etc., oder allgemeinen Funktionsstörungen des Gehirns Verwendung finden. Die neuen Verbindungen sind aber auch wertvolle Zwischenprodukte zur Herstellung anderer, insbesondere pharmazeutisch wirksamer Verbindungen.

Die Erfindung betrifft insbesondere Verbindungen der Formel I, worin n für 1, 2 oder 3 steht, Ar für einen mono- oder bicyclischen, carbocyclischen Arylrest oder für einen fünf- oder sechsgliedrigen, ein bis und mit vier Ringstickstoffatome, ein Ringsauerstoff- oder Ringschwefelatom, oder ein oder zwei Ringstickstoffatome zusammen mit einem Ringsauerstoff oder einem Ringschwefelatom als Ringglieder enthaltenden, monocyclischen Heteroarylrest, der gegebenenfalls einen ankondensierten Benzoring enthält, steht, und insbesondere Phenyl, Naphthyl, Pyrryl, Pyrazolyl, Imidazolyl, Triazolyl, Tetrazolyl, Furyl, Thienyl, Isoxazolyl, Oxazolyl, Oxadiazolyl, Isothiazolyl, Thiazolyl, Thiadiazolyl, Pyridyl, Pyridazinyl, Pyrimidinyl, Pyrazinyl, Triazinyl, Indolyl, Isoindolyl, Benzoxadiazolyl, Benzthiadiazolyl, Benzimidazolyl, Benzofuranyl, Benzothienyl, Chinolinyl oder Isochinolinyl bedeutet, wobei in diesen Resten Ringkohlenstoffatome gegebenenfalls durch Niederalkyl, Niederalkenyl, Niederalkinyl, Niederalkylen, Cycloalkyl, Phenyl, Phenylniederalkyl, Phenylniederalkoxy und/oder Phenylniederalkylthio, wobei Niederalkyl, Phenyl, Phenylniederalkyl, Phenylniederalkoxy und/oder Phenylniederalkylthio gegebenenfalls Hydroxy, Niederalkoxy, Halogenniederalkoxy, Niederalkenyloxy, Niederalkinyloxy, Niederalkylendioxy, Niederalkanoyloxy, Halogen, Carboxy, Niederalkoxycarbonyl, Carbamoyl, N-Niederalkylcarbamoyl, N,N-Diniederalkyl-carbamoyl und/oder Cyan, die cyclischen Reste auch Niederalkyl, das seinerseits wie angegeben substituiert sein kann, als Substituenten enthalten können, und/oder durch Hydroxy, Niederalkoxy, Halogenniederalkoxy, Niederalkenyloxy, Niederalkinyloxy, Niederalkylendioxy, Niederalkanoyloxy, Halogen,

Nitro, Amino, Niederalkylamino, Diniederalkylamino, N-Niederalkyl-N-phenylniederalkylamino, Niederalkylenamino, Oxaniederalkylenamino, Thianiederalkylenamino und/oder Azaniederalkylenamino, worin das Azastickstoffatom durch Niederalkyl, Phenyl oder Phenylniederalkyl substituiert sein kann, wobei diese Substituenten Hydroxy, Niederalkoxy, Halogenniederalkoxy, Niederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkylcarbamoyl und/oder Cyan als Substituenten enthalten können, und/oder durch Niederalkanoylamino, Azido, Carboxy, Niederalkoxycarbonyl, Carbamoyl, N-Niederalkylcarbamoyl, N,N-Diniederalkyl-carbamoyl, Cyan, Sulfo, Aminosulfonyl, Niederalkylthio, Niederalkylsulfinyl und/oder Niederalkylsulfonyl, und/oder Ringstickstoffatome gegebenenfalls durch Niederalkoxycarbonyl oder durch Niederalkyl, das als Substituenten gegebenenfalls Hydroxy, Niederalkoxy, Halogenniederalkoxy, Niederalkenyloxy, Niederalkinyloxy, Niederalkylendioxy, Niederalkanoyloxy, Halogen, Carboxy, Niederalkoxycarbonyl, Carbamoyl, N-Niederalkylcarbamoyl, N,N-Diniederalkyl-carbamoyl oder Cyan enthalten kann, oder durch Hydroxy oder Oxido substituiert sein können, der Rest Ac für Niederalkanoyl, unsubstituiertes oder durch Niederalkyl, Niederalkoxy, Nitro und/oder Halogen substituiertes Benzoyl, Niederalkylsulfonyl, unsubstituiertes oder durch Niederalkyl, Niederalkoxy, Nitro und/oder Halogen substituiertes Phenylsulfonyl, Niederalkoxycarbonyl, Hydroxyniederalkoxycarbonyl, Niederalkoxyniederalkoxycarbonyl, Aminoniederalkoxycarbonyl, Niederalkylaminoniederalkoxycarbonyl, N,N-Diniederalkylaminoniederalkoxycarbonyl, N-Niederalkyl-N-phenylniederalkyl-aminoniederalkoxycarbonyl, N,N-Niederalkylenaminoniederalkoxycarbonyl, Morpholinoniederalkoxycarbonyl, Thiomorpholinoniederalkoxycarbonyl, Piperazinoniederalkoxycarbonyl, 4-Niederalkyl-piperazino-niederalkoxycarbonyl, unsubstituiertes oder durch Niederalkyl, Niederalkoxy, Nitro und/oder Halogen substituiertes Phenyl-, Thienyl-, Furyl-, Pyrryl- oder Pyridyl-niederalkoxycarbonyl, Niederalkenyloxy- oder Niederalkinyloxycarbonyl, Carbamoyl, N-Niederalkyl-carbamoyl, N,N-Diniederalkylcarbamoyl, N-Hydroxycarbamoyl, N,N-Niederalkylencarbamoyl, Morpholinocarbonyl,

Thiomorpholinocarbonyl, Piperazinocarbonyl, 4-Niederalkylpiperazinocarbonyl, 5-Tetrazolyl, unsubstituiertes oder durch Niederalkyl oder Phenyl substituiertes 4,5-Dihydro-2-oxazolyl oder 5,6-Dihydro-4H-1,3-oxazin-2-yl steht, Z einen Rest $-OR_7$ oder $-NR_8R_9$ bedeutet, $R_1$ Wasserstoff; Niederalkyl, das gegebenenfalls durch Niederalkylamino, Diniederalkylamino, Niederalkylenamino, Morpholino, Thiomorpholino, Piperazino, welches an einem Stickstoffatom gegebenenfalls Niederalkyl oder Niederalkanoyl als Substituenten trägt, Carboxy, funktionell abgewandeltes Carboxy, Niederalkoxy, Niederalkoxy-niederalkoxy oder Phenyl, das seinerseits gegebenenfalls Niederalkyl, Niederalkoxy, Halogen und/oder Nitro als Substituenten enthält, oder N-Pyrrolyl, N-Imidazolyl, N-Pyrazolyl, N-Indolyl oder N-Isoindolyl substituiert ist; Phenyl, das gegebenenfalls wie ein Phenyl-niederalkyl-Rest $R_1$ substituiert ist; Hydroxy, Niederalkoxy oder Phenylniederalkoxy darstellt, $R_2$ und $R_3$ unabhängig voneinander Wasserstoff, Niederalkyl, das gegebenenfalls durch Hydroxy, Niederalkoxy, das gegebenenfalls Amino , Niederalkylamino, Diniederalkylamino oder Acylamino als Substituenten enthält, Acyloxy oder Phenyl substituiert ist, Formyl, Diniederalkylacetal oder -dithioacetal, Niederalkylenacetal oder -dithioacetal, funktionell abgewandeltes Carboxy; Phenyl, das gegebenenfalls Niederalkyl, Hydroxy, Niederalkoxy, Niederalkylendioxy, Halogen, Nitro, Amino, Niederalkylamino, Diniederalkylamino, Niederalkanoylamino, Carboxy, funktionell abgewandeltes Carboxy und/oder Niederalkylthio als Substituenten enthält; Pyrryl, Furyl, Thienyl oder Pyridyl, wobei diese Reste gegebenenfalls wie ein Phenylrest $R_2$ oder $R_3$ substituiert sind; Amino, Niederalkylamino oder Diniederalkylamino bedeuten, $R_4$ Wasserstoff oder Niederalkyl bedeutet, $R_5$ Wasserstoff, unsubstituiertes oder durch freies oder veräthertes Hydroxy, freies oder veräthertes Mercapto, das gegebenenfalls oxidiert sein kann, durch Carboxy, funktionell abgewandeltes Carboxy, durch Amino, das gegebenenfalls seinerseits Niederalkyl, Carboxy, funktionell abgewandeltes Carboxy oder Acyl als Substituenten enthält, durch einen mono- oder bicyclischen carbocyclischen Arylrest oder einen fünf- oder sechsgliedrigen Heteroarylrest gemäss der obigen Definition für Ar substituiertes Niederalkyl, unsubstituiertes oder durch freies oder veräthertes Hydroxy, Niederalkyl, Nitro, Amino, Niederalkylamino, Diniederalkylamino, Niederalkanoylamino und/oder Halogen substituiertes Phenyl oder einen gegebenenfalls in gleicher Weise substituierten monocyclischen fünf- oder sechsgliedrigen Heteroarylrest gemäss der obigen Definition für Ar bedeutet, $R_6$ Wasserstoff, Niederalkyl, unsubstituiertes oder durch freies oder veräthertes Hydroxy, Niederalkyl, Nitro, Amino, Niederalkylamino, Diniederalkylamino, Niederalkanoylamino und/oder Halogen substituiertes Phenylniederalkyl, unsubstituiertes oder wie ein Phenylniederalkyl-Rest $R_6$ substituiertes Phenyl oder einen gegebenenfalls in gleicher Weise substituierten monocyclischen fünf- oder sechsgliedrigen Heteroarylrest bedeutet, und $R_7$, $R_8$ und $R_9$ unabhängig voneinander Wasserstoff; Alkyl, das gegebenenfalls durch Amino, Niederalkylamino, Diniederalkylamino, Niederalkylenamino, Oxa-, Thia- oder Azaniederalkylenamino, worin der Aza-Stickstoff gegebenenfalls Niederalkyl oder Niederalkanoyl als Substituenten trägt, Acylamino, freies oder veräthertes Hydroxy oder einen carbocyclischen Arylrest oder Heteroarylrest wie oben für die Gruppe Ar definiert substituiert ist; oder einen carbocyclischen Arylrest oder Heteroarylrest wie oben für die Gruppe Ar definiert bedeuten, worin $R_1$ und $R_2$ zusammen oder $R_1$ und $R_3$ zusammen $C_3$-$C_5$-Niederalkylen bedeuten können, in dem das mit dem C2- bzw. C6-Kohlenstoffatom des 1,4-Dihydropyridinrings direkt verbundene Kohlenstoffatom gegebenenfalls durch ein Sauerstoff-, Schwefel- oder ein Stickstoffatom, das durch Wasserstoff oder Niederalkyl substituiert ist, ersetzt ist, worin $R_4$ und $R_5$ zusammen unsubstituiertes oder durch freies oder veräthertes Hydroxy, Amino, Niederalkylamino, Diniederalkylamino und/oder Halogen, am Aza-Stickstoff auch durch Niederalkyl, substituiertes Niederalkylen, Oxa-, Thia- oder Azaniederalkylen bedeuten können, worin $R_5$ und $R_6$ zusammen Niederalkylen, Aza-, Oxa- oder Thianiederalkylen bedeuten können, wobei diese Reste gegebenenfalls an Kohlenstoffatomen durch freies oder veräthertes Hydroxy, Amino, Niederalkylamino, Diniederalkylamino, Halogen, Carboxy und/oder funktionell abgewandeltes Carboxy und am Aza-Stickstoff gegebenenfalls durch Niederalkyl substituiert sind, und worin $R_8$ und $R_9$ zusammen Niederalkylen, Aza-,

Oxa- oder Thianiederalkylen bedeuten können, wobei diese Reste gegebenenfalls an Kohlenstoffatomen durch freies oder veräthertes Hydroxy, Amino, Niederalkylamino, Diniederalkylamino, Halogen, Carboxy, funktionell abgewandeltes Carboxy oder einen fünf- oder sechsgliedrigen Monoaza-, Diaza- oder Triaza-Heteroarylrest, der gegebenenfalls ganz oder partiell gesättigt und gegebenenfalls an Kohlenstoffatomen durch Oxo substituiert ist und an den Azastickstoffatomen gegebenenfalls durch Niederalkyl oder Phenyl, welches seinerseits Niederalkyl, Halogen, Niederalkoxy und/oder Nitro als Substituenten enthalten kann, substituiert ist; und am Aza-Stickstoff gegebenenfalls durch Niederalkyl, das seinerseits gegebenenfalls einen carbocyclischen Arylrest oder Heteroarylrest wie oben für die Gruppe Ar definiert und/-oder freies oder veräthertes Hydroxy, Acyloxy, Amino, Niederalkylamino und/oder Diniederalkylamino als Substituenten enthält; durch Acyl oder Phenyl, welches seinerseits Niederalkyl, Halogen, Niederalkoxy und/oder Nitro als Substituenten enthalten kann, substituiert sind, optische Isomere von Verbindungen der Formel I, Mischungen dieser optischen Isomere und Salze von solchen Verbindungen mit salzbildenden Gruppen.

Die Erfindung betrifft in erster Linie Verbindungen der Formel I, worin n für 1, 2 oder 3 steht, Ar Phenyl das gegebenenfalls durch Niederalkyl, Phenyl, Phenylniederalkyl, Phenylniederalkoxy und/oder Phenylniederalkylthio, wobei solche Reste ihrerseits Hydroxy, Niederalkoxy, Niederalkylendioxy, Halogen, Carboxy, Niederalkoxycarbonyl und/oder Cyan, die cyclischen Reste auch Niederalkyl als Substituenten enthalten können, und/oder durch Hydroxy, Niederalkoxy, Halogen-niederalkoxy, Niederalkylendioxy, Halogen, Nitro, Amino, Niederalkylamino, Diniederalkylamino, Niederalkanoylamino, Carboxy, Niederalkoxycarbonyl, Carbamoyl, Cyan, Sulfo, Aminosulfonyl, Niederalkylthio, Niederalkylsulfinyl und/oder Niederalkylsulfonyl substituiert ist, oder Pyrryl, Furyl, Thienyl, Pyridyl, Benzoxadiazolyl oder Benzthiadiazolyl, wobei diese Reste gegebenenfalls wie ein Phenylrest Ar substituiert sind, und insbesondere Niederalkyl, Niederalkoxy, Halogen und/oder gegebenenfalls durch Niederalkyl, Niederalkoxy, Halogen und/oder Nitro substituiertes Phenyl als Substituenten enthalten, bedeutet, der Rest Ac für Niederalkanoyl, Niederalkylsulfonyl, Niederalkoxycarbonyl, Hydroxy-niederalkoxycarbonyl, Niederalkoxyniederalkoxycarbonyl, N,N-Diniederalkylamino-niederalkoxycarbonyl, N-Niederalkyl-N-phenylniederalkyl-amino-niederalkoxycarbonyl, N,N-Niederalkylenamino-niederalkoxycarbonyl, (4-Morpholino)-niederalkoxycarbonyl, Niederalkenyloxy- oder Niederalkinyloxycarbonyl, Carbamoyl, N-Niederalkyl-carbamoyl, N,N-Diniederalkyl-carbamoyl, N,N-Niederalkylencarbamoyl, 4-Morpholinocarbonyl oder 4-Niederalkyl-1-piperazino-carbonyl steht, Z einen Rest $-OR_7$ oder $-NR_8R_9$ bedeutet, $R_1$ für Wasserstoff, Niederalkyl, Diniederalkylaminoniederalkyl, Niederalkylenaminoniederalkyl, (4-Morpholino)-niederalkyl, Carboxy-niederalkyl, Niederalkoxycarbonyl-niederalkyl oder Niederalkoxyniederalkoxy-niederalkyl, wobei Diniederalkylamino, Niederalkylenamino bzw. 4-Morpholino durch mindestens zwei Kohlenstoffatome vom Ringstickstoffatom getrennt sind, oder für Phenylniederalkyl, Phenyl, Hydroxy oder Niederalkoxy steht, $R_2$ und $R_3$ unabhängig voneinander Niederalkyl, das gegebenenfalls durch Hydroxy, Niederalkoxy, das gegebenenfalls Amino, Niederalkylamino oder Diniederalkylamino als Substituenten enthält, Niederalkanoyloxy oder Phenyl substituiert ist, Formyl, Diniederalkylacetal oder -dithioacetal, Niederalkylenacetal oder -dithioacetal, Cyan, Phenyl, Thienyl oder Amino bedeuten, $R_4$ Wasserstoff oder Niederalkyl bedeutet, $R_5$ Wasserstoff, unsubstituiertes oder durch Hydroxy, Niederalkoxy, Mercapto, Niederalkylthio, Carboxy, Carbamoyl, Amino, Niederalkanoylamino, Carbamoylamino, Guanidino, durch Phenyl, welches seinerseits durch Hydroxy und/oder Halogen substituiert sein kann, Imidazolyl oder Indolyl substituiertes Niederalkyl, unsubstituiertes oder durch Hydroxy, Niederalkoxy, Niederalkyl, Nitro, Amino, Niederalkylamino, Diniederalkylamino und/oder Halogen substituiertes Phenyl oder unsubstituiertes oder in gleicher Weise wie für einen Phenylrest $R_5$ angegeben substituiertes Pyrryl, Pyrazolyl, Imidazolyl, Triazolyl, Tetrazolyl, Furyl, Thienyl, Isoxazolyl, Oxazolyl, Oxadiazolyl, Isothiazolyl, Thiazolyl, Thiadiazolyl, Pyridyl, Pyridazinyl, Pyrimidinyl, Pyrazinyl oder Triazinyl bedeutet, $R_6$ Wasserstoff, Niederalkyl, Phenylniederalkyl, unsubstituiertes oder durch Hydroxy, Niederalkoxy, Niederalkyl, Nitro, Amino, Niederalkylamino, Diniederalkylamino und/oder Halogen substituiertes Phenylniederalkyl oder einen unsubstituierten oder in gleicher Weise wie für einen Phenylniederalkylrest $R_6$ angegeben substituierten Rest aus der Gruppe Phenyl, Pyrryl, Pyrazolyl, Imidazolyl, Triazolyl, Tetrazolyl, Furyl, Thienyl, Isoxazolyl, Oxazolyl, Oxadiazolyl, Isothiazolyl, Thiazolyl, Thiadiazolyl, Pyridyl, Pyridazinyl, Pyrimidinyl, Pyrazinyl und Triazinyl bedeutet, und $R_7$, $R_8$ und $R_9$ unabhängig voneinander Wasserstoff, Niederalkyl oder durch Amino, Niederalkylamino, Diniederalkylamino, Niederalkylenamino, Oxa-, Thia- oder Azaniederalkylenamino, worin der Aza-Stickstoff gegebenenfalls durch Niederalkyl substituiert ist, Niederalkanoylamino, Hydroxy, Niederalkoxy oder durch Phenyl, welches unsubstituiert oder seinerseits durch Amino, Niederalkylamino, Diniederalkylamino, Niederalkylenamino, Hydroxy, Niederalkoxy, Halogen und/oder Nitro substituiert sein kann, oder durch Pyrryl, Furyl, Thienyl oder Pyridyl, wobei diese Gruppen in gleicher Weise wie Phenyl substituiert sein können, substituiertes Niederalkyl oder Phenyl, Pyrryl, Furyl, Thienyl oder Pyridyl, wobei diese Gruppen in gleicher Weise wie ein Phenyl-niederalkyl-Rest $R_7$, $R_8$ oder $R_9$ substituiert sein können, bedeuten, worin $R_1$ und $R_2$ zusammen oder $R_1$ und $R_3$ zusammen $C_3$-$C_5$-Niederalkylen bedeuten können, in dem das mit dem C2 bzw. C6-Kohlenstoffatom des 1,4-Dihydropyridinrings direkt verbundene Kohlenstoffatom gegebenenfalls durch ein Sauerstoff-, Schwefel- oder ein Stickstoffatom, welches durch Wasserstoff oder Niederalkyl substituiert ist, ersetzt ist, worin $R_4$ und $R_5$ zusammen unsubstituiertes oder hydroxysubstituiertes $C_3$-$C_5$-Niederalkylen, $C_2$-$C_4$-Oxa- oder $C_2$-$C_4$-Thianiederalkylen bedeuten können, worin $R_5$ und $R_6$ zusammen Niederalkylen mit 2 bis 5 Kettenkohlenstoffatomen oder Azaniederalkylen mit 3 oder 4 Kettenkohlenstoffatomen bedeuten können, wobei diese Reste unsubstituiert oder durch Hydroxy, Niederalkoxy, Amino, Niederalkylamino oder Diniederalkylamino substituiert sein können, und worin $R_8$ und $R_9$ zusammen $C_2$-$C_7$-Niederalkylen, $C_3$-$C_4$-Aza-, $C_3$-$C_4$-Oxa- oder $C_3$-$C_4$-Thianiederalkylen bedeuten können, wobei diese Reste gegebenenfalls an Kohlenstoffatomen durch Hydroxy, Niederalkoxy, Amino, Niederalkylamino, Diniederalkylamino oder fünf- oder sechsgliedriges Monoaza- oder Diazaheterocyclyl, das gegebenenfalls an Kohlenstoffatomen durch Oxo substituiert ist und an den Azastickstoffatomen gegebenenfalls durch Niederalkyl oder Phenyl, welches seinerseits Niederalkyl, Halogen, Niederalkoxy und/oder Nitro als Substituenten enthalten kann, substituiert ist; und am Aza-Stickstoff gegebenenfalls durch Niederalkyl, das seinerseits durch Phenyl, Pyrryl, Furyl, Thienyl und/oder Pyridyl, wobei diese Reste selbst gegebenenfalls Niederalkyl, Hydroxy, Niederalkoxy, Halogen und/oder Nitro als Substituenten enthalten, und oder durch Hydroxy, Niederalkoxy, Niederalkanoyloxy, Amino, Niederalkylamino und/oder Diniederalkylamino substituiert sein kann; durch Benzoyl, Niederalkanoyl, Furanylcarbonyl, Thienylcarbonyl, Pyrrylcarbonyl, Pyridinylcarbonyl oder Phenyl, welches seinerseits Niederalkyl, Halogen, Niederalkoxy und/oder Nitro als Substituenten enthalten kann, substituiert sind, optische Isomere von Verbindungen der Formel I, Mischungen dieser optischen Isomere und Salze von solchen Verbindungen mit salzbildenden Gruppen.

Die Erfindung betrifft ganz besonders Verbindungen der Formel I, worin n für 1 oder 2 steht, Ar für Phenyl, Thienyl, Furyl oder Benzoxadiazolyl steht, wobei diese Gruppen gegebenenfalls durch Niederalkyl, Niederalkoxy, Halogenniederalkoxy, Phenylniederalkoxy, Phenylniederalkylthio, Niederalkylendioxy, Halogen, Trifluormethyl, Nitro, Niederalkanoylamino und/-oder Cyan mono-, di- oder trisubstituiert sind, der Rest Ac Niederalkanoyl, Niederalkylsulfonyl, Niederalkoxycarbonyl, 2-Niederalkoxy-niederalkoxycarbonyl, N,N-Diniederalkylamino-niederalkoxycarbonyl, N-Niederalkyl-N-phenylniederalkyl-amino-niederalkoxycarbonyl, Carbamoyl, N-Niederalkyl-carbamoyl, N,N-Diniederalkyl-carbamoyl, N,N-Niederalkylen-carbamoyl oder 4-Morpholinocarbonyl bedeutet, Z für einen Rest $-OR_7$ oder $-NR_8R_9$ steht, $R_1$ Wasserstoff, Niederalkyl, 2-(Diniederalkylamino)-niederalkyl, 2-(Niederalkylenamino)-niederalkyl, 2-(4-Morpholino)-niederalkyl, Carboxyniederalkyl oder Niederalkoxyniederalkoxy-niederalkyl bedeutet, $R_2$ und $R_3$ unabhängig voneinander Niederalkyl, Hydroxyniederalkyl, Cyan oder Amino bedeuten, $R_4$ Wasserstoff oder Niederalkyl darstellt, $R_5$ Wasserstoff, unsubstituiertes oder durch Hydroxy, Niederalkoxy, Mercapto, Niederalkylthio, Carboxy, Carbamoyl, Amino, Carbamoylamino, Guanidino, Phenyl, welches seinerseits durch Hydroxy substituiert sein kann, oder Imidazol-4-yl oder

Indol-3-yl substituiertes Niederalkyl oder Phenyl, Thiazolyl, Imidazolyl, Furyl, Thienyl, Pyridyl oder Pyrimidinyl, wobei diese Reste gegebenenfalls durch Hydroxy, Niederalkoxy und/oder Amino substituiert sind, bedeutet, $R_6$ Wasserstoff, Niederalkyl, Phenylniederalkyl, welches unsubstituiert oder durch Hydroxy, Niederalkoxy und/oder Amino substituiert ist, oder Phenyl, Thiazolyl, Imidazolyl, Furyl, Thienyl, Pyridyl oder Pyrimidinyl, wobei diese Reste gegebenenfalls wie ein Phenylniederalkyl-Rest $R_6$ substituiert sind, bedeutet, und $R_7$, $R_8$ und $R_9$ unabhängig voneinander Wasserstoff oder unsubstituiertes oder durch Amino, Niederalkylamino, Diniederalkylamino, Niederalkylenamino, Oxa- oder Azaniederalkylenamino, worin der Aza-Stickstoff gegebenenfalls durch Niederalkyl substituiert ist, Hydroxy, Niederalkoxy, Phenyl, welches unsubstituiert oder seinerseits durch Amino, Hydroxy, Niederalkoxy und/oder Halogen substituiert sein kann, oder durch Thienyl oder Pyridyl, wobei diese Gruppen in gleicher Weise wie Phenyl substituiert sein können, substituiertes Niederalkyl bedeuten, worin $R_4$ und $R_5$ zusammen $C_3$-$C_4$-Niederalkylen bedeuten können, worin $R_5$ und $R_6$ zusammen $C_2$-$C_5$-Niederalkylen oder $C_4$-Azaniederalkylen bedeuten können und worin $R_8$ und $R_9$ zusammen $C_4$-$C_5$-Niederalkylen, das gegebenenfalls durch 2-Imidazolidinon-1-yl, welches in 3-Stellung Phenyl oder Niederalkyl als Substituenten enthalten kann, substituiert ist, $C_4$-Oxa- oder $C_4$-Azaniederalkylen bedeuten können, wobei der Aza-Stickstoff durch Niederalkyl, das seinerseits unsubstituiert oder durch Phenyl, welches Niederalkyl, Niederalkoxy, Halogen und/oder Nitro als Substituenten enthalten kann, Thienyl und/oder Pyridyl mono- oder disubstituiert ist, oder durch Benzoyl, Furanylcarbonyl, Phenyl oder Niederalkoxyphenyl substituiert sein kann, optische Isomere von Verbindungen der Formel I, Mischungen dieser optischen Isomere und pharmazeutisch verwendbare Salze von solchen Verbindungen mit salzbildenden Gruppen.

Die Erfindung betrifft hauptsächlich Verbindungen der Formel I, worin n für 1 oder 2 steht, Ar für unsubstituiertes oder durch Niederalkyl, Niederalkoxy, Halogenniederalkoxy, Benzyloxy, Benzylthio, Halogen, Trifluormethyl, Nitro und/oder Cyan mono- oder disubstituiertes Phenyl, 2- oder 3-Thienyl, aber auch für 2,1,3-Benzoxadiazol-4-yl steht,

der Rest Ac Niederalkylsulfonyl oder Niederalkoxycarbonyl darstellt, Z für einen Rest $-OR_7$ oder $-NR_8R_9$ steht, $R_1$ Wasserstoff, ferner 2-(4-Morpholino)-äthyl bedeutet, $R_2$ Niederalkyl, Hydroxymethyl, Cyan oder Amino bedeutet, $R_3$ für Niederalkyl steht, $R_4$ Wasserstoff oder Niederalkyl darstellt, $R_5$ Wasserstoff, unsubstituiertes oder durch Phenyl, welches seinerseits durch Hydroxy substituiert sein kann, substituiertes Niederalkyl, Phenyl oder unsubstituiertes oder Amino-substituiertes Thiazolyl bedeutet, $R_6$ Wasserstoff, Niederalkyl oder Phenyl bedeutet, $R_7$ für Wasserstoff, Niederalkyl oder Niederalkyl, das durch Phenyl, Amino, Niederalkylamino, Diniederalkylamino, 4-Morpholino, 1-Piperazino, welches seinerseits am 4-Stickstoff Niederalkyl als Substituenten enthalten kann, oder Niederalkoxy substituiert ist, steht, $R_8$ und $R_9$ unabhängig voneinander Wasserstoff, unsubstituiertes oder durch Niederalkoxy, Phenyl oder Pyridyl substituiertes Niederalkyl bedeuten, worin $R_4$ und $R_5$ zusammen 1,3-Propylen bedeuten können, worin $R_5$ und $R_6$ zusammen für 1,5-Pentylen stehen können, und worin $R_8$ und $R_9$ zusammen 1,5-Pentylen, 3-(3-Phenyl-2-imidazolidinon-1-yl)-1,5-pentylen, 3-(2-Imidazolidinon-1-yl)-1,5-pentylen oder 3-Oxa- oder 3-Aza-1,5-pentylen bedeuten können, wobei der Aza-Stickstoff gegebenenfalls durch Niederalkyl, Benzyl, Diphenylmethyl, wobei die Reste Benzyl oder Diphenylmethyl gegebenenfalls Halogen, Niederalkyl und/oder Niederalkoxy als Substituenten enthalten, Benzoyl, Furanylcarbonyl, Phenyl oder Niederalkoxyphenyl, substituiert ist, optische Isomere von Verbindungen der Formel I, Mischungen dieser optischen Isomere und pharmazeutisch verwendbare Salze von solchen Verbindungen mit salzbildenden Gruppen.

Die Erfindung betrifft vor allem Verbindungen der Formel I, worin n für 1 steht, Ar 2- oder 3-Nitro-phenyl, 2- oder 3-Difluormethoxy-phenyl, 2- oder 3-Methyl-phenyl, 2- oder 3-Trifluormethyl-phenyl, 2,3-Dimethyl-phenyl,

2,3-Dichlor-phenyl, 2- oder 3-Benzyloxy-phenyl oder 2- oder 3-Benzylthiophenyl, bedeutet, Ac Niederalkoxycarbonyl darstellt, Z für einen Rest $-OR_7$ oder $-NR_8R_9$ steht, $R_1$ Wasserstoff ist, $R_2$ und $R_3$ unabhängig voneinander Niederalkyl bedeuten, $R_4$ Wasserstoff ist, $R_5$ Niederalkyl oder Phenyl bedeutet, $R_6$ Wasserstoff darstellt, $R_7$ unsubstituiertes oder durch 4-Morpholino, 1-Piperazino oder 4-Niederalkyl-1-Piperazino substituiertes Niederalkyl bedeutet, $R_8$ und $R_9$ unabhängig voneinander für Wasserstoff, unsubstituiertes oder durch Phenyl oder Pyridyl substituiertes Niederalkyl stehen, und worin $R_8$ und $R_9$ zusammen 3-Aza-1,5-Pentylen, in dem der Aza-Stickstoff gegebenenfalls durch Benzyl oder Diphenylmethyl substituiert ist, bedeuten können, optische Isomere von Verbindungen der Formel I, Mischungen dieser optischen Isomere und pharmazeutisch verwendbare Salze von solchen Verbindungen mit salzbildenden Gruppen.

Bevorzugt sind die Verbindungen der Formel I, worin Ar 2- oder 3-Nitrophenyl, 2- oder 3-Difluormethoxy-phenyl, 2- oder 3-Methyl-phenyl, 2- oder 3-Trifluormethyl-phenyl, 2,3-Dimethyl-phenyl, 2,3-Dichlorphenyl, 2- oder 3-Benzyloxy-phenyl oder 2- oder 3-Benzylthio-phenyl bedeutet, ferner aber auch 2,1,3-Benzoxadiazol-4-yl. Von besonderer Bedeutung sind die Verbindungen der Formel I, worin Ar 2- oder 3-Nitrophenyl, und ganz besonders 3-Nitro-phenyl, bedeutet. Eine weitere bevorzugte Ausführungsform der Erfindung sind die Verbindungen der Formel I, worin Ac Niederalkoxycarbonyl, 2-Niederalkoxy-niederalkoxycarbonyl,Niederalkylsulfonyl, Carbamoyl, N-Alkyl- oder N,N-Dialkylcarbamoyl, im besonderen Niederalkoxycarbonyl, 2-Niederalkoxy-niederalkoxycarbonyl, Carbamoyl, N-Alkyl- oder N,N-Dialkylcarbamoyl, und vor allem Niederalkoxycarbonyl bedeutet. Hervorzuheben sind die Verbindungen der Formel I, worin Z für einen Rest $-NR_8R_9$ steht. Bevorzugt sind die Verbindungen der Formel I, worin Z einen Rest $-OR_7$ bedeutet. Bevorzugt sind ferner die Verbindungen der Formel I, worin $R_1$ Wasserstoff ist. Bevorzugt sind auch die Verbindungen der Formel I, worin $R_2$ und/oder $R_3$ Niederalkyl, Hydroxyniederalkyl, insbesondere Hydroxymethyl, Cyan oder Amino bedeuten. Hervorzuheben sind die Verbindungen der Formel I, worin einer der Reste $R_2$ und $R_3$ für Niederalkyl steht und der andere die für $R_2$ und $R_3$ unter Formel I angegebenen Reste, insbesondere Niederalkyl, Hydroxyniederalkyl,

Cyan oder Amino, bedeutet. Von besonderem Interesse sind die Verbindungen der Formel I, worin $R_2$ Niederalkyl, insbesondere Methyl oder Aethyl, und vor allem Methyl bedeutet. Bevorzugt sind ferner die Verbindungen der Formel I, worin $R_3$ Niederalkyl, insbesondere Methyl oder Aethyl, und vor allem Methyl bedeutet. Von besonderer Bedeutung sind die Verbindungen der Formel I, worin $R_4$ Wasserstoff ist. Bevorzugt sind die Verbindungen der Formel I, worin $R_5$ Niederalkyl oder Phenyl, insbesondere $C_3$-$C_4$-Niederalkyl oder Phenyl, vor allem Isopropyl, sek.-Butyl (1-Methylpropyl) oder Phenyl, und in erster Linie Isopropyl bedeutet. Hervorzuheben sind ferner die Verbindungen der Formel I, worin $R_6$ für Wasserstoff steht. Weiterhin sind die Verbindungen der Formel I bevorzugt, worin $R_7$ unsubstituiertes oder durch 4-Morpholino oder 4-Niederalkyl-1-Piperazino substituiertes Niederalkyl, und insbesondere $C_2$-$C_4$-Niederalkyl bedeutet. Hervorzuheben sind ferner Verbindungen der Formel I, worin $R_8$ und $R_9$ zusammen 3-Aza-1,5-Pentylen bedeuten, wobei der Aza-Stickstoff durch Benzyl oder Diphenylmethyl substituiert ist.

Die Erfindung betrifft insbesondere die in den Beispielen beschriebenen spezifischen Verbindungen. Sie betrifft ferner auch alle optischen Isomeren sowie beliebige Mischungen von optischen Isomeren, z.B. Gemische bestehend aus allen optischen Isomeren, der in den Beispielen beschriebenen spezifischen Verbindungen.

Die Verbindungen der Formel I und Salze von solchen Verbindungen mit salzbildenden Eigenschaften können in an sich bekannter Weise hergestellt werden, indem man z.B.

a) eine Verbindung der Formel II

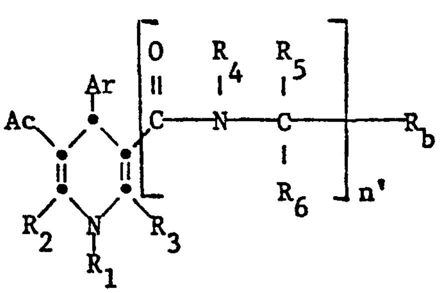

(II),

worin n' für 0, 1 oder 2 steht und $R_b$ eine gegebenenfalls aktivierte Carboxygruppe bedeutet, mit der Massgabe, dass $R_b$ von einem Rest

C(=O)—Z verschieden ist, wenn n' für 1 oder 2 steht, mit einer Verbindung der Formel III

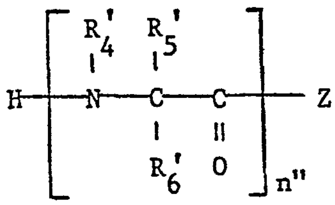

(III) ,

worin n" für 1, 2 oder 3 steht, und $R'_4$, $R'_5$ und $R'_6$ die unter Formel I für $R_4$, $R_5$ und $R_6$ angegebene Bedeutung haben, jedoch von den Resten $R_4$, $R_5$ bzw. $R_6$ in Formel II verschieden sein können, umsetzt, mit der Massgabe, dass die Summe aus n' und n" nicht grösser als 3 ist, oder

a') in einer Verbindung der Formel IIa

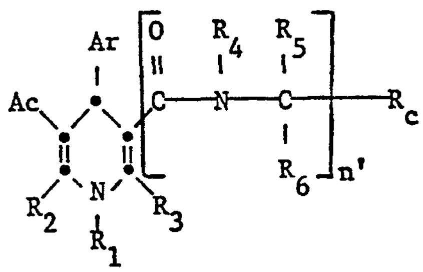

(IIa),

worin n' für 1, 2 oder 3 steht und $R_c$ eine in den Rest C(=O)-Z überführbare Gruppe ist, diese in den Rest C(=O)-Z überführt, oder

b) eine Verbindung der Formel IV, oder ein reaktionsfähiges Derivat davon, mit einer Verbindung der Formel V, oder einem Tautomeren davon, und mit einer Verbindung der Formel VI umsetzt,

(IV)

(VI)

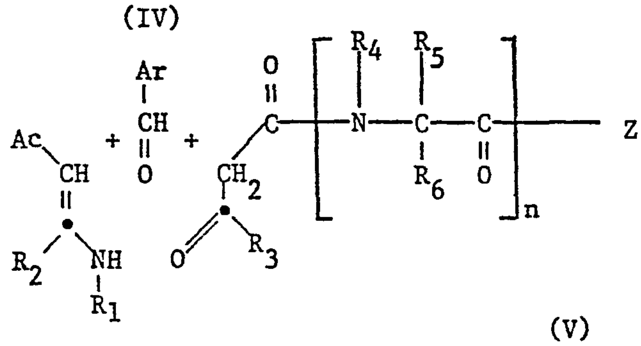

(V)

oder

b') völlig analog zu Verfahren b), eine Verbindung der Formel Va mit einer Verbindung der Formel VI umsetzt,

(VI) 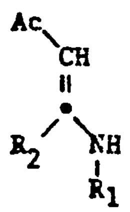

+

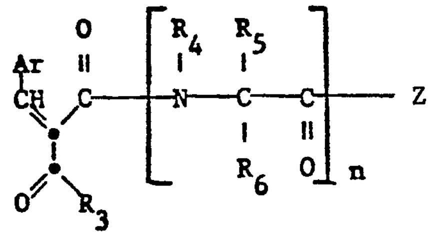 (Va)

oder

c) eine Verbindung der Formel VII

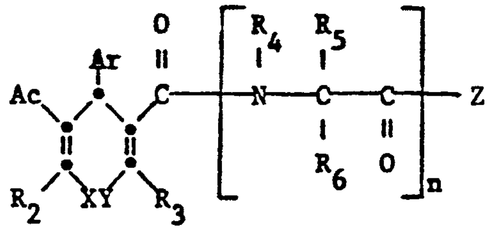 (VII) ,

worin einer der Reste X und Y für die Gruppe der Formel $-NH-R_1$ steht und der andere Hydroxy ist oder beide die Gruppe der Formel $-NH-R_1$ bedeuten, oder ein Tautomeres davon oder ein entsprechendes Tautomerengemisch ringschliesst, oder

d) eine Verbindung der Formel Ar-CHO (IV) oder ein reaktionsfähiges funktionelles Derivat davon mit einer Verbindung der Formel VIII

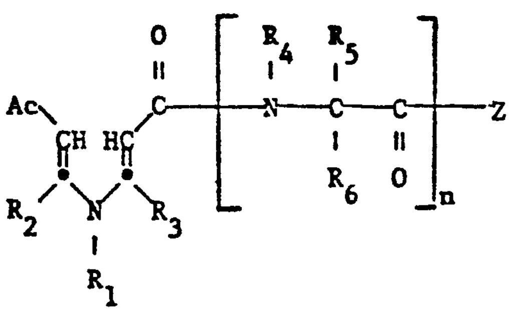 (VIII)

oder einem Tautomeren davon oder einem entsprechenden Tautomerengemisch umsetzt, oder

e) in einer Verbindung der Formel IX

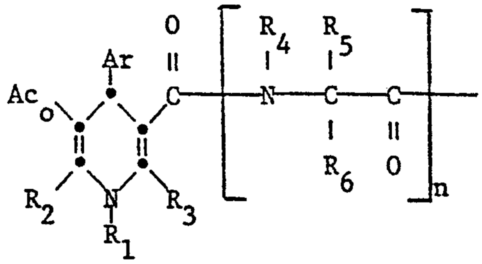

(IX) ,

worin $Ac_o$ einen in die Gruppe Ac überführbaren Rest darstellt, diesen in die Gruppe Ac überführt,

wobei in den obigen Ausgangsstoffen der Formeln II bis IX, IIa und Va, die, sofern sie salzbildende Eigenschaften aufweisen, auch in Form ihrer Salze verwendet werden können, die Symbole n, Ar, Ac, $R_1$, $R_2$, $R_3$, $R_4$, $R_5$, $R_6$ und Z die unter Formel I gegebenen Bedeutungen haben, und, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt, und/oder, wenn erwünscht, ein erhaltenes Gemisch von Isomeren oder Racematen in die einzelnen Isomere oder Racemate auftrennt, und/oder, wenn erwünscht, erhaltene Racemate in die optischen Antipoden aufspaltet.

In einer Verbindung der Formel II ist $R_b$ z.B. eine freie Carboxygruppe oder eine zum Zweck der Knüpfung einer Amid- bzw. Peptidbindung aktivierte Carboxygruppe.

Zur Durchführung der Verfahrensvariante a) kann die Carboxygruppe beispielsweise durch Ueberführung in ein Säureazid, -anhydrid, -imidazolid, -isoxazolid oder einen aktivierten Ester, wie einen der weiter unten genannten, oder durch Reaktion mit einem Carbodiimid,

wie N,N'-Dicyclohexylcarbodiimid, gegebenenfalls unter Zusatz von N-Hydroxysuccinimid, einem unsubstituierten oder z.B. durch Halogen, Methyl oder Methoxy substituierten 1-Hydroxybenzotriazol oder 4-Hydroxybenzo-1,2,3-triazin-3-oxid (u.a., vgl. DE-OS 1 917 690, DE-OS 1 937 656, DE-OS 2 202 613), oder insbesondere von N-Hydroxy-5-norbornen-2,3-dicarboximid, oder mit N,N'-Carbonyldiimidazol aktiviert werden. Ferner kann sie auch dadurch aktiviert werden, dass man sie zunächst z.B. mit Trimethylsilylchlorid zum entsprechenden Trimethylsilylester und dann mit einem geeigneten Halogenierungsmittel, z.B. Thionylchlorid, in das entsprechende Säurehalogenid, insbesondere Säurechlorid, überführt.

Zur Bildung von aktivierten Estern, die oben erwähnt sind, eignen sich z.B. gegebenenfalls durch elektronenanziehende Substituenten substituierte Phenole und Thiophenole wie Phenol, Thiophenol, Thiokresol, p-Nitrothiophenol, 2,4,5- und 2,4,6-Trichlorphenol, Penta(fluor oder chlor)phenol, o- und p-Nitrophenol, 2,4-Dinitrophenol, p-Cyanphenol, weiter z.B. N-Hydroxysuccinimid, N-Hydroxyphthalimid und N-Hydroxypiperidin.

Zur Durchführung des Verfahrens a) werden je eine Verbindung der Formel II und III in an sich bekannter Weise einer Kondensationsreaktion unterworfen.

Als die gebräuchlichste Methode zur Knüpfung von Amid- bzw. Peptidbindungen ist die Carbodiimidmethode zu nennen, ferner auch die Azidmethode, die Methode der aktivierten Ester und die Anhydridmethode, sowie die Merrifield-Methode, die Methode der N-Carboxyanhydride oder N-Thiocarboxyanhydride und die Säurechloridmethode.

Bei einer besonders bevorzugten Herstellung der Verbindungen der Formel I verwendet man als Kupplungsmethode die Carbodiimid-Methode mit N,N'-Dicyclohexylcarbodiimid in Anwesenheit von 1-Hydroxybenzotriazol. Dabei wird eine Verbindung der Formel II, worin $R_b$ eine freie Carboxygruppe ist, durch 1-Hydroxy-benzotriazol zunächst intermediär in den entsprechenden Benzotriazol-1-yl-ester überführt,

welcher dann unter Einwirkung des oben genannten Carbodiimids mit einer Verbindung der Formel III, vorzugsweise einer solchen, worin Z $OR_7$ und $R_7$ gegebenenfalls substituiertes Niederalkyl ist oder worin Z $NR_8R_9$ bedeutet, zu einer Verbindung der Formel I umgesetzt wird.

Die Ausgangsstoffe der Formel II und der Formel III können unabhängig voneinander als Racemat, Racematgemisch, als optisches Isomeres oder als Mischung mehrerer optischer Isomere eingesetzt werden. Zum Beispiel in Chem. Pharm. Bull. 28, 2809-2812 (1980) ist die Herstellung von optischen Isomeren der Formel II aus einer racemischen Verbindung der Formel II beschrieben.

Sehr viele Ausgangsstoffe der Formel III - Aminosäuren, Di- und Tripeptide und deren C-terminale gegebenenfalls substituierte Niederalkylester - sind bekannt, sowohl als Racemate, Racematgemische als auch als optische Isomere, und weitere sind analog zu den bekannten herstellbar.

Ausgangsstoffe der Formel II, worin n' für 0 steht, sind bekannt, z.B. von der Europäischen Patentanmeldung 11.706 her, und weitere analog zu den bekannten herstellbar. Ausgangsstoffe der Formel II, worin n' für 1 oder 2 steht, lassen sich z.B. dadurch herstellen, dass man Verbindungen der Formel II, worin n' für 0 steht, mit einer Verbindung der Formel IIIa

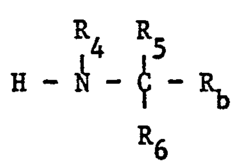

(IIIa),

bzw. einer Verbindung der Formel IIIb

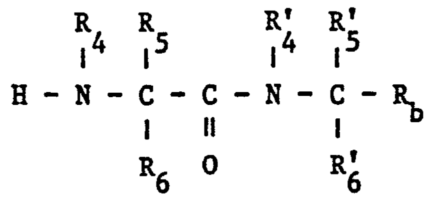

(IIIb),

worin $R_b$ die unter Formel II und $R_4$, $R_5$ und $R_6$ die unter Formel I angegebene Bedeutung haben und $R'_4$, $R'_5$ und $R'_6$ die unter Formel I für $R_4$, $R_5$ und $R_6$ angegebene Bedeutung haben, jedoch von $R_4$, $R_5$ bzw. $R_6$ verschieden sein können, in gleicher Weise umsetzt wie oben für die Herstellung von Verbindungen der Formel I aus jeweils einer Verbindung der Formel II und III beschrieben. Verbindungen der Formel IIIa und IIIb sind bekannt; sie sind entweder mit Verbindungen der Formel III, worin Z $OR_7$ bedeutet und $R_7$ für Wasserstoff steht und n" 1 bzw. 2 bedeutet, identisch oder lassen sich aus eben diesen Verbindungen nach den gleichen Aktivierungsmethoden herstellen wie sie oben für Verbindungen der Formel II beschrieben sind.

Die Verfahrensvariante a') umfasst unter anderem die Ueberführung von Verbindungen der Formel IIa, worin $R_c$ z.B. eine geschützte und/oder funktionell abgewandelte Carboxygruppe bedeutet, in Verbindungen der Formel I. Unter einer geschützten Carboxygruppe ist eine durch eine Schutzgruppe, wie sie üblicherweise in der Peptidchemie zur Anwendung kommt, geschützte Carboxygruppe zu verstehen. Solche Schutzgruppen sind leicht, das heisst ohne dass unerwünschte Nebenreaktionen stattfinden, beispielsweise solvolytisch, reduktiv, photolytisch oder auch unter physiologischen Bedingungen, abspaltbar, wobei in der Regel Verbindungen der Formel I erhalten werden, in denen Z $OR_7$ bedeutet und $R_7$ Wasserstoff ist.

Schutzgruppen dieser Art, sowie ihre Abspaltung sind beispielsweise beschrieben in "Protective Groups in Organic Chemistry", Plenum Press, London, New York, 1973, ferner in "The Peptides", Vol. I, Schröder and Lübke, Academic Press, London, New York, 1965, sowie

in "Methoden der organischen Chemie", Houben-Weyl, 4. Auflage, Bd. 15/1, Georg Thieme Verlag, Stuttgart, 1974.

So werden Carboxygruppen beispielsweise durch Hydrazidbildung oder durch Veresterung geschützt. Zur Veresterung geeignet sind z.B. niedere substituierte Alkanole wie Cyanmethanol, 2,2,2-Trichloräthanol oder Benzoylmethanol. Eine besonders vorteilhafte Kategorie der substituierten Alkanole sind Aethanole, die in β-Stellung eine trisubstituierte Silylgruppe, wie Triphenylsilyl-, Dimethyl-butyl-silyl- oder vor allem Trimethylsilylgruppe, tragen. Wie z.B. im belgischen Patent Nr. 851.576 beschrieben ist, eignen sich diese Alkohole zum Schutze der Carboxygruppen deshalb besonders gut, weil die entsprechenden β-Silyläthylester, z.B. β-(Trimethylsilyl)-äthylester, zwar die Stabilität üblicher Alkylester besitzen, sich jedoch unter milden Bedingungen durch Einwirkung von Fluoridionen-abgebenden Reagentien, z.B. Fluoriden quatärnärer organischer Basen, wie Tetraäthylammoniumfluorid, selektiv abspalten lassen. Sie können aber auch, gleich wie die üblichen substituierten Alkylester, durch alkalische Hydrolyse, z.B. mittels Alkalimetallhydroxiden, -carbonaten oder -bicarbonaten, abgespalten werden.

Ist in einer Verbindung der Formel IIa (bzw. I) $R_c$ eine funktionell abgewandelte Carboxygruppe, also z.B. Cyan, ein Imidoester, insbesondere ein Imidoniederalkylester, eine gegebenenfalls substituierte Carbamoylgruppe oder ein Säurehalogenid, so lässt sich diese in an sich bekannter Weise, insbesondere durch Hydrolyse in alkalischem oder saurem Medium, in eine Verbindung der Formel I überführen, worin Z $OR_7$ bedeutet und $R_7$ für Wasserstoff steht, wobei man im ersteren Fall auch direkt ein Salz erhalten kann. Eine Verbindung der Formel IIa, worin $R_c$ Cyan ist, lässt sich weiterhin in üblicher Weise, z.B. durch Addition von gegebenenfalls substituierten Niederalkanolen in Gegenwart einer wasserfreien Säure, wie Chlorwasserstoff, und nachträglicher vorsichtiger Hydrolyse des entstandenen Imidoesters zu Verbindungen der Formel I, worin $R_7$ gegebenenfalls substituiertes Niederalkyl ist, überführen; gleiches gelingt auch durch die Umsetzung einer Verbindung der Formel IIa, worin $R_c$ ein Säurehalogenid ist, mit gegebenenfalls substituierten Niederalkanolen.

In dieser Verfahrensvariante können jedoch auch Ausgangsstoffe der Formel IIa, in denen $R_c$ z.B. Formyl oder ein reaktionsfähiges Derivat davon - wie unten definiert -, Hydroxymethyl oder Methyl ist, verwendet werden, welche nach üblichen Oxidationsmethoden in Verbindungen der Formel I, vorzugsweise in solche, worin Z $OR_7$ bedeutet und $R_7$ für Wasserstoff steht, überführt werden können.

Insbesondere bei der Durchführung der Verfahrenvariante a') muss darauf geachtet werden, dass unerwünschte Nebenreaktionen, welche die Umwandlung zusätzlicher Gruppierungen im Molekül zur Folge haben können, nicht eintreten.

Ausgangsstoffe der Formel IIa, worin n' für 1, 2 oder 3 steht, lassen sich z.B. in analoger Weise herstellen wie oben für die Verbindungen der Formel II, worin n' für 1 oder 2 steht, beschrieben, nämlich durch Umsetzung einer Verbindung der Formel II, worin n' für 0 oder 1 steht, mit Verbindungen, die sich von solchen der Formel IIIa und IIIb durch das Vorliegen von $R_c$ anstelle von $R_b$ unterscheiden.

Reaktionsfähige funktionelle Derivate des in der Verfahrensvariante b) als Ausgangsstoff verwendeten Aldehyds der Formel IV, ebenso wie von Formylgruppen allgemein, sind u.a. die entsprechenden Acetale, wie Diniederalkyl-, z.B. Dimethyl- oder Diäthylacetale, Acylale wie Diniederalkanoylacylale, z.B. Diacetylacylale, oder insbesondere die entsprechenden Dihalogenmethyl-, z.B. Dichlormethyl- oder Dibrommethyl-Verbindungen, ferner Additionsverbindungen, wie solche mit Wasser oder einem Alkalimetall-, z.B. Kaliumhydrogensulfit.

Eine Variante dieses Verfahrens besteht darin, zunächst eine Verbindung der Formel IV mit einer Verbindung der Formel V umzusetzen, z.B. in Gegenwart einer organischen oder anorganischen Base, z.B. einer katalytischen Menge Piperidin, oder vorzugsweise in Gegenwart einer anorganischen oder organischen Säure, z.B. Mineralsäure wie etwa Salzsäure, oder z.B. p-Toluolsulfonsäure, und das sich bildende Zwischenprodukt der Formel Va

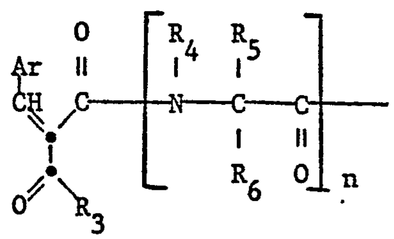

(Va),

nach seiner Isolierung oder ohne es zu isolieren, mit einer Verbindung der Formel VI in Reaktion treten zu lassen.

Ausgangsprodukte der Formel IV und VI sind bekannt, und weitere analog zu den bekannten herstellbar. Das Ausgangsmaterial der Formel V lässt sich in an sich bekannter Weise z.B. dadurch gewinnen, dass man eine Verbindung der Formel III z.B. mit Diketen gemäss Pharm. Acta. Helv. 38, 616 (1963) umsetzt.

Ueblicherweise werden die in der Verfahrensvariante c) verwendeten Ausgangsstoffe der Formel VII in situ gebildet, und der verfahrensgemässe Ringschluss findet unter den Reaktionsbedingungen für die Herstellung des Ausgangsmaterials statt. So kann man die Ausgangsstoffe der Formel VII und unter den Reaktionsbedingungen üblicherweise auch die entsprechenden Endprodukte der Formel I erhalten, indem man
ca) eine Verbindung der Formel IV oder ein oben definiertes reaktionsfähiges funktionelles Derivat davon mit einer Verbindung der Formel

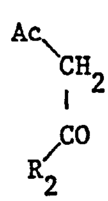

(VIb),

einer Verbindung der Formel V und einer Verbindung der Formel $R_1$-$NH_2$ (X) umsetzt, oder cb) eine Verbindung der Formel IV oder ein reaktionsfähiges funktionelles Derivat davon mit einer Verbindung der Formel

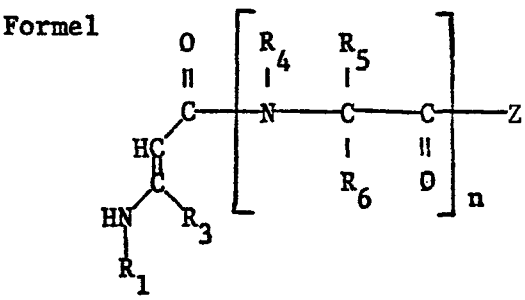

(Vb),

und einer Verbindung der Formel VIb oder VI umsetzt, oder cc) eine Verbindung der Formel X mit einer Verbindung der Formel

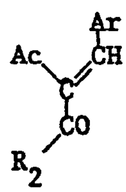

(VIa)

und einer Verbindung der Formel V umsetzt, oder cd) eine Verbindung der Formel X mit einer Verbindung der Formel Va und einer Verbindung der Formel VIb oder VI umsetzt, oder ce) eine Verbindung der Formel X mit einer Verbindung der Formel

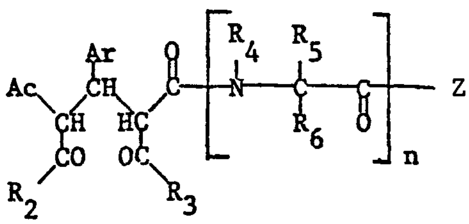

(XI)

umsetzt, oder cf) eine Verbindung der Formel VI mit einer Verbindung der Formel Va oder der Formel

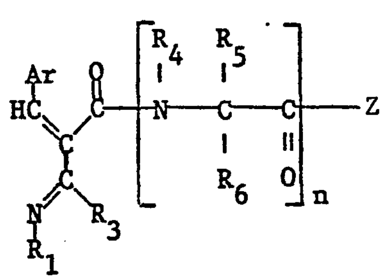

(Vc)

umsetzt, oder cg) eine Verbindung der Formel Vb mit einer Verbindung der Formel VIa oder mit einer Verbindung der Formel

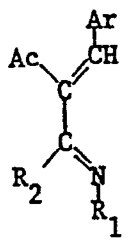

(VIc)

umsetzt. Dabei können mit Ausnahme der Verbindungen der Formeln IV und X die Verbindungen der Formeln V, Va-c, VI, VIa-c, VII und XI in Form von Tautomeren oder in Form von Tautomerengemischen verwendet werden; Ausgangsstoffe der obigen Formeln mit salzbildenden Eigenschaften können auch in Form von Salzen verwendet werden. Ferner haben in den obgenannten Verbindungen die Symbole n, Ar, Ac, $R_1$, $R_2$, $R_3$, $R_4$, $R_5$, $R_6$ und Z die unter Formel I angegebene Bedeutung.

Eine Verbindung der Formel X kann auch in Form eines, diese Verbindung in situ abgebenden Mittels, z.B. Ammoniak in Form eines Ammoniumsalzes, wie Ammoniumacetat oder Ammoniumhydrogencarbonat, oder einer Leichtmetall-, z.B. Alkalimetallverbindung, wie Natriumamid oder Lithium-N-methylamid, verwendet werden.

Bei der Ringschlussreaktion c), sowie den Kondensationsreaktionen ca) bis cg) zur Herstellung des üblicherweise in situ gebildeten Ausgangsmaterials für die Ringschlussreaktion, ebenso wie auch bei Verfahrensvariante b), handelt es sich um Varianten der Dihydropyridinsynthese von Hantzsch. Bei der Variante ca) werden insgesamt drei Moleküle Wasser abgespalten; bei weiteren Varianten tritt teilweise an die Stelle der Wasserabspaltung eine Additionsreaktion, d.h. die Wasserabspaltung erfolgt schon bei der Herstellung von einem oder von zwei Ausgangsstoffen. Bei der Umsetzung von Verbindungen der Formel IV mit Verbindungen der Formel Vb und VI gemäss Variante cb), von Verbindungen der Formel VI mit Verbindungen der Formel Vc gemäss Variante cf), oder von Verbindungen der Formel Vb mit Verbindungen der Formel VIc gemäss Variante cg) wird zusätzlich zu bzw. anstelle von Wasser eine Verbindung der Formel X abgespalten.

Falls nach der Variante ca) Verbindungen der Formel I hergestellt werden sollen, können unerwünschte Nebenprodukte, z.B. vom Typ der 1,4-Dihydro-pyridin-3,5-dicarbonsäurederivate, wie entsprechende Diester, entstehen. Durch nicht-gleichzeitiges Zusammengeben der Reaktionsteilnehmer kann die Bildung solcher Nebenprodukte indessen herabgesetzt werden, indem man einen bestimmten Reaktionsverlauf fördert, der in situ gemäss einer anderen Variante verläuft, da entsprechend der gestaffelten Zugabe der Reaktionskomponenten z.B. zunächst eine Verbindung der allgemeinen Formel VI oder der Formel Vb entstehen kann.

Die verfahrensgemässen Ringschluss- bzw. Kondensationsreaktionen werden in an sich bekannter Weise durchgeführt, falls notwendig, in Gegenwart eines Kondensationsmittels, insbesondere eines basischen Kondensationsmittels, wie eines Ueberschusses einer basischen Reaktionskomponente oder einer zusätzlichen, z.B. organischen Base, wie Piperidin oder Aethyl-diisopropyl-amin, oder eines Metallalkoholats, wie Alkalimetall-niederalkanolats, oder, falls eine Verbindung der Formel X als eine solche mit einem Leichtmetall, etwa als Natriumamid, vorliegt, in Gegenwart saurer Mittel, etwa einer organischen Carbonsäure, z.B. Essigsäure, und/oder eines geeigneten Dehydratisierungs- oder Wasser-aufnehmenden Mittels, ferner üblicherweise in Gegenwart eines inerten organischen Lösungsmittels und bei Reaktionstemperaturen im Bereich von etwa Raumtemperatur bis etwa 150°C, insbesondere bei Siedetemperatur des Lösungsmittels. Gegebenenfalls erfolgt die Umsetzung in einer Inertgas-, z.B. Stickstoffatmosphäre, und/oder, z.B. bei Verwendung eines niedrigsiedenden Lösungsmittels und/oder eines Ausgangsstoffes der Formel X, im geschlossenen Gefäss unter erhöhtem Druck.

Die in den Verfahrensvarianten verwendeten Ausgangsstoffe sind bekannt oder können nach an sich bekannten Verfahren hergestellt werden.
So lässt sich beispielsweise eine Verbindung der Formel Va aus je einer Verbindung der Formeln V und IV herstellen, eine Verbindung der

Formel VIa aus je einer der Formeln VIb und IV, eine Verbindung der Formel Vb aus je einer der Formeln V und X, eine Verbindung der Formel Vc aus je einer der Formeln Vb und IV, eine Verbindung der Formel VIc aus je einer der Formeln VI und IV und eine solche der Formel XI aus je einer der Formeln VIb und Va.

Die Verfahrensvariante d) wird in an sich bekannter Weise durchgeführt. Reaktionsfähige funktionelle Derivate des Aldehyds der Formel IV sind oben beschrieben. Die Reaktion wird in Ab-, vorzugsweise in Anwesenheit eines Lösungs- oder Verdünnungsmittels oder eines entsprechenden Gemisches und/oder eines Kondensationsmittels durchgeführt, wobei man unter Kühlen, bei Raumtemperatur, oder vorzugsweise unter Erwärmen, z.B. in einem Temperaturbereich von etwa 0°C bis etwa 200°C, vorzugsweise von etwa 40°C bis etwa 150°C, und, falls notwendig, in einem geschlossenen Gefäss, gegebenenfalls unter Druck, und/oder unter einer Inertgasatmosphäre arbeitet.

Das Ausgangsmaterial der Formel VIII kann in an sich bekannter Weise hergestellt werden; z.B. kann man durch Reaktion einer Verbindung der Formel V, vorzugsweise einer solchen, worin Z von einer Hydroxygruppe verschieden ist, mit einer Verbindung der Formel VI direkt zum Ausgangsmaterial der Formel VIII gelangen.

Die in der Verfahrensvariante e) verwendeten Ausgangsstoffe der Formel IX können entsprechend dem in ihnen enthaltenen Rest $Ac_o$ beispielsweise Carbonsäuren ($Ac_o$ ist Carboxy), Carbonsäureanhydride, insbesondere gemischte Anhydride, wie Säurehalogenide, z.B. -chloride oder -bromide ($Ac_o$ ist Halogencarbonyl, z.B. Chlor- oder Bromcarbonyl), weiter aktivierte Ester, z.B. Cyanmethyl- oder Pentachlorphenylester ($Ac_o$ ist Cyanmethoxycarbonyl oder Pentachlorphenyloxycarbonyl), sein. Solche Ausgangsstoffe können, gegebenenfalls in Gegenwart von Kondensationsmitteln, durch Behandeln mit einem Alkohol, wie einem unsubstituierten oder substituierten Niederalkanol, oder einem reaktionsfähigen Derivat davon, z.B. einem entsprechenden Alkoholat, wie Alkalimetallalkoholat, freie Carbonsäuren auch durch Umsetzen mit geeigneten Diazo-Verbindungen, wie unsubstituierten oder substituierten Diazoniederalkanen, in Verbindungen der Formel I umgewandelt werden, in denen Ac den Acylrest eines Monoesters der Kohlensäure darstellt. Solche Verbindungen können ebenfalls erhalten werden, wenn als Ausgangsstoffe der Formel IX Salze, insbesondere Alkalimetall- oder Erdalkalimetallsalze, der freien Carbonsäure verwendet und diese mit reaktionsfähigen Estern von Alkoholen, wie unsubstituierten oder substituierten Niederalkanolen, wie entsprechenden Halogeniden, z.B. Chloriden, Bromiden oder Iodiden, oder organischen Sulfonsäureestern, z.B. Niederalkansulfonsäure- oder Arensulfonsäureestern, wie Methansulfonsäure- bzw. p-Toluolsulfonsäureestern, behandelt werden, oder wenn man entsprechende hydrolysierbare Iminoester, wie entsprechende Imino-niederalkylester, zu den Estern hydrolysiert.

Die Umsetzung von freien Carbonsäuren mit Alkoholen, wie unsubstituierten oder substituierten Niederalkanolen, erfolgt vorteilhaft in Gegenwart eines sauren, wasserabspaltenden Katalysators, wie einer Protonensäure, z.B. Chlorwasserstoff-, Bromwasserstoff-, Schwefel-, Phosphor- oder Borsäure, Benzolsulfon- oder Toluolsulfonsäure, oder einer Lewissäure, z.B. Bortrifluorid-Aetherat, in einem Ueberschuss des eingesetzten Alkohols und/oder in einem inerten Lösungsmittel, erforderlichenfalls unter destillativer, z.B. azeotroper, Entfernung des bei der Reaktion freigesetzten Wassers. Weiter kann man die Umsetzungen auch in Gegenwart von wasserbindenden Kondensationsmitteln, wie geeignet substituierten Carbodiimiden, z.B. N,N'-Diäthyl-, N,N'-Dicyclohexyl- oder N-Aethyl-N'-(3-dimethylaminopropyl)-carbodiimid, gegebenenfalls in inerten organischen Lösungsmitteln, durchführen. Gemischte Anhydride, insbesondere Säurehalogenide, werden beispielsweise in Gegenwart säurebindender Mittel, z.B. organischer, insbesondere tertiärer Stickstoffbasen, wie Triäthylamin, Aethyldiisopropylamin oder Pyridin, oder auch anorganischer Basen, z.B. von Alkalimetall- oder Erdalkalimetallhydroxiden oder -carbonaten, wie Natrium-, Kalium- oder Calciumhydroxid bzw. -carbonat, mit Alkoholen oder mit Alkoholaten, z.B. Alkalimetallniederalkoxiden, umgesetzt.

Die Umsetzungen von reaktionsfähigen Estern, z.B. Cyanmethyl- oder Pentachlorphenylestern, mit Alkoholen werden beispielsweise in einem gegenüber den Reaktionsteilnehmern inerten Lösungsmittel und im Temperaturbereich von etwa 0° bis etwa 120°C, vorzugsweise bei Raumtemperatur bis etwa 60°C, durchgeführt.

Die Hydrolyse von Iminoester-, insbesondere Iminoniederalkylester-Ausgangsstoffen, erfolgt beispielsweise mittels wasserhaltiger Mineralsäuren, wie Salzsäure oder Schwefelsäure, wobei man z.B. die bei der Addition von Chlorwasserstoff an Nitrile und Umsetzung mit wasserfreien Alkoholen, insbesondere unsubstituierten oder substituierten Niederalkanolen, erhaltenen Iminoestersalze, z.B. -hydrochloride, nach Zusatz von Wasser direkt zu den entsprechenden Estern hydrolysieren kann. Man kann z.B. auch aus einem Gemisch des Nitril-Ausgangsmaterials, eines Alkohols und Schwefelsäure mit geeignetem Wassergehalt, ohne Isolierung des in situ entstandenen Iminoesters, die gewünschte Esterverbindung der Formel I erhalten.

Verbindungen der Formel I, in denen der Rest Ac den Acylrest eines Kohlensäuremonoamids darstellt, kann man aus Verbindungen der Formel IX erhalten, in denen $Ac_o$ für eine Carboxygruppe, eine Säureanhydridgruppe, wie Halogencarbonyl, z.B. Chlorcarbonyl, oder eine aktivierte Estergruppe, wie Cyanmethoxycarbonyl oder Pentachlorphenyloxycarbonyl, steht, indem man solche Ausgangsstoffe, gegebenenfalls in Gegenwart eines geeigneten Kondensationsmittels, mit Ammoniak oder einem Ammoniak abgebenden Mittel oder einem N-mono- oder N,N-disubstituierten Amin umsetzt.

Diese Umwandlungen von Carboxy- und geeignet funktionell abgewandelten reaktionsfähigen Carboxygruppen in gegebenenfalls N-mono- oder N,N-disubstituierte Carbamoylgruppen können in an sich bekannter Weise, z.B. nach den für die Bildung der Estergruppen beschriebenen Verfahren, durchgeführt werden.

Verbindungen der Formel I, in welchen die Gruppe Ac für Carbamoyl steht, kann man, ausgehend von Verbindungen der Formel IX, in welchen

der Rest $Ac_o$ Cyan darstellt, ebenfalls erhalten. Solche Ausgangsstoffe können hydrolytisch, vorzugsweise unter sauren oder basischen Bedingungen, z.B. in Gegenwart eines Alkalimetall-, wie Natriumhydroxids, und, wenn erwünscht, von Wasserstoffperoxid, in einem wässrig-alkoholischen Lösungsmittel, wie wässrigem Aethanol, in die gewünschten Verbindungen der Formel I mit einer Carbamoylgruppe Ac übergeführt werden.

Die oben oder weiter unten angegebenen Reaktionen können unter an sich bekannten Reaktionsbedingungen durchgeführt werden, in Ab- oder üblicherweise Anwesenheit von Lösungs- oder Verdünnungsmitteln, je nach Art der Reaktion und/oder Reaktionsteilnehmer bei erniedrigter oder erhöhter Temperatur, z.B. im Temperaturbereich von etwa -10°C bis etwa 150°C, unter atmosphärischem Druck oder in einem geschlossenen Gefäss, gegebenenfalls unter Druck, und/oder in einer inerten Atmosphäre, z.B. unter einer Stickstoffatmosphäre.

Ausgangsstoffe der Formel IX mit freier Carboxygruppe $Ac_o$ können z.B. erhalten werden, indem man den entsprechenden 2-Cyanoäthylester herstellt und diesen, unter milden Bedingungen, z.B. mittels wässrigem oder wässrig-niederalkanolischem 1-n. Natriumhydroxid bei Raumtemperatur, zur freien Carbonsäure spaltet. Den 2-Cyanäthylester selber kann man z.B. durch Umsetzen einer Verbindung der Formel IV mit einer Verbindung der Formel VI, worin Ac für eine 2-Cyanäthyloxycarbonylgruppe steht, und einer Verbindung der Formel V erhalten. Die im Ausgangsmaterial der Formel IX vorhandene freie Carboxygruppe kann, falls notwendig, in an sich bekannter Weise in die gewünschte reaktionsfähige, funktionell abgewandelte Form überführt werden.

Die als Ausgangsstoffe für die Verfahrensvariante e) ebenfalls in Betracht kommenden Nitrilverbindungen der Form IX können z.B. analog zu den obgenannten 2-Cyanäthylester-Verbindungen hergestellt werden, indem man Zwischenprodukte verwendet, die anstelle des Restes Ac eine Cyangruppe enthalten.

Verbindungen der Formel I, worin $R_3$ funktionell abgewandeltes Formyl ist, lassen sich z.B. nach Verfahren b) aus einer Verbindung der Formel V, worin $R_3$ funktionell abgewandeltes Formyl, z.B. ein Diniederalkylacetal, bedeutet, und je einer Verbindung der Formeln IV und VI herstellen (vgl. auch Europäische Patentanmeldung 107 203).

Verbindungen der Formel I, worin $R_3$ funktionell abgewandeltes Formyl ist, lassen sich in an sich bekannter Weise in Verbindungen der Formel I, worin $R_3$ Formyl bedeutet, umwandeln, die ihrerseits in an sich bekannter Weise in andere Verbindungen der Formel I, worin $R_3$ z.B. Hydroxymethyl, Hydroxyimino oder Cyan bedeutet, überführt werden können (vgl. Belgisches Patent 879,263). Ebenso ist es z.B. möglich, analog zu Verfahren b) aus einer Verbindung der Formel V, worin n = 0 ist, $R_3$ funktionell abgewandeltes Formyl und Z vorzugsweise eine Gruppe $-OR_7$ bedeutet, worin $R_7$ z.B. für eine Säureschutzgruppe, etwa 2-Cyanäthyl oder Carbamoylmethyl, steht, und je einer Verbindung der Formeln IV und VI zunächst eine Verbindung der Formel II herzustellen, worin n = 0 ist und $R_3$ funktionell abgewandeltes Formyl bedeutet. In der letzteren kann der Rest $R_3$ wiederum wie oben angegeben z.B. in Formyl, Hydroxymethyl, Hydroxyimino oder Cyan überführt werden. Die erwähnten Verbindungen der Formel II lassen sich gemäss Verfahren a) in Verbindungen der Formel I überführen.

In analoger Weise sind auch Verbindungen der Formel I, worin $R_2$ funktionelles Formyl ist, erhältlich, nämlich z.B. nach Verfahren cb) durch Umsetzung von je einer Verbindung der Formel IV und Vb mit einer Verbindung der Formel VIb, worin $R_2$ funktionell abgewandeltes Formyl bedeutet. Solche Verbindungen der Formel I lassen sich wiederum in an sich bekannter Weise in andere Verbindungen der Formel I überführen, worin $R_2$ z.B. Formyl, Hydroxymethyl, Hydroxyimino oder Cyan bedeutet.

Verbindungen der Formel II- analog auch der Formel I -, worin $R_3$ Amino bedeutet, können z.B. gemäss Verfahren c), insbesondere nach der Variante cb), aus einer Verbindung der Formel Vb',die völlig analog zu einer Verbindung der Formel Vb ist, worin $R_3$ Amino bedeutet und n z.B. für 0 steht,

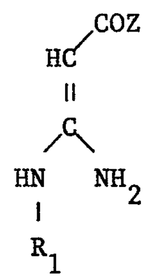

(Vb')

und je einer Verbindung der Formeln IV und VIb hergestellt werden (vgl. Liebigs Ann. Chem. 1977, 1895-1908). Ausgangsverbindungen der Formel Vb' sind gemäss den üblichen Amidinsynthesen erhältlich, z.B. durch Ueberführung einer Verbindung $NC-CH_2-COZ$ in den entsprechenden Iminoäther, etwa mittels Methanol und HCl, und Behandlung des letzteren z.B. mit Ammoniak.

In gleicher Weise lassen sich auch Verbindungen der Formel I, worin $R_2$ Amino bedeutet, herstellen, nämlich z.B. nach Verfahren b) aus je einer Verbindung der Formel IV und V mit einer Verbindung der Formel VI, worin $R_2$ Amino bedeutet.

Verbindungen der Formel I, worin Ac einen Acylrest von cyclischen Kohlensäurederivaten und/oder $R_2$ einen carbocyclischen oder heterocyclischen Arylrest bedeutet, können z.B. nach Verfahren b) hergestellt werden, wobei die Ausgangsverbindungen der Formel VI z.B. analog den in US Patent 4 414 213 beschriebenen Verfahren erhältlich sind.

Verbindungen der Formel I, worin $R_1$ unsubstituiertes oder substituiertes Niederalkyl bedeutet, können z.B. durch N-Alkylierung

aus entsprechenden Verbindungen der Formel I, worin $R_1$ Wasserstoff ist, erhalten werden (vgl. US Patent 4 258 042).

Verbindungen der Formel I, worin $R_1$ z.B. Hydroxy bedeutet, lassen sich z.B. gemäss Verfahren c), insbesondere Variante ce), aus einer Verbindung der Formel XI und Hydroxylamin herstellen (vgl. Europäische Patentanmeldung 93 945). Aus solchen Verbindungen der Formel I, worin $R_1$ Hydroxy ist, erhält man nach an sich bekannten Methoden der O-Alkylierung und O-Acylierung Verbindungen der Formel I, worin $R_1$ veräthertes oder verestertes Hydroxy ist.

Verbindungen der Formel I, worin $R_1$ und $R_2$ zusammen unsubstituiertes oder substituiertes Niederalkylen, worin ein Kohlenstoffatom gegebenenfalls durch ein Heteroatom ersetzt ist, bedeuten, lassen sich z.B. gemäss Verfahren b') herstellen (vgl. Liebigs Ann. Chem. 1977, 1888-1894). Völlig analog sind Verbindungen der Formel I, worin $R_1$ und $R_3$ zusammen unsubstituiertes oder substituiertes Niederalkylen, worin ein Kohlenstoffatom gegebenenfalls durch ein Heteroatom ersetzt ist, bedeuten, z.B. in einer Umsetzung einer Verbindung der Formel VIa mit einer Verbindung der Formel Vb gemäss Verfahren cg) erhältlich.

Verbindungen der Formel I, worin $R_3$ Wasserstoff ist, sind z.B. durch Reaktion einer Verbindung der Formel VIa mit einer Verbindung der Formel Vb, in der $R_3$ Diniederalkylamino oder Niederalkylenamino bedeutet, erhältlich. Es entstehen zunächst 3,4-Dihydropyridine, die durch Hydrierung, beispielsweise in Eisessig über $PtO_2$, in einer Additions-Eliminations-Reaktion zu den gewünschten Verbindungen führen [vgl. Angew. Chem. 93, 755-763 (1981)]. Verbindungen der Formel I, worin $R_2$ Wasserstoff ist, sind völlig analog aus einer Verbindung der Va und einer Verbindung der Formel VI, worin $R_2$ Diniederalkylamino oder Niederalkylenamino bedeutet, erhältlich.

Es finden sich in der Literatur zahlreiche Hinweise auf die Herstellung von 4-Aryl-1,4-dihydropyridinen, z.B. in Angew. Chem. 93, 755-763 (1981), Liebigs Ann. Chem. 1977, 1888-1894 und 1895-1908, BE 879 263, DE-A-3 207 982, in den Europäischen Patentanmeldungen 26 317, 60 897, 93 945 und 107 203, sowie in den US-Patenten 4 258 042 und 4 414 213.

Erfindungsgemäss erhältliche Verbindungen der Formel I können in an sich bekannter Weise in andere Verbindungen der Formel I umgewandelt werden.

So kann man z.B. in Verbindungen der Formel I, worin $R_1$ für Wasserstoff steht, durch Behandeln mit einem reaktionsfähigen Ester eines Alkohols der Formel $R_1$-OH (XII) einen organischen Rest $R_1$ einführen und so zu Verbindungen der Formel I gelangen, worin $R_1$ von Wasserstoff verschieden ist.

Reaktionsfähige Ester von Verbindungen der Formel XII, z.B. von unsubstituierten oder substituierten Niederalkanolen, sind solche mit starken, anorganischen oder organischen Säuren; dabei kommen beispielsweise die entsprechenden Halogenide, insbesondere Chloride, Bromide oder Jodide, ferner Sulfate, weiter Niederalkansulfonsäure- oder Arensulfonsäureester, z.B. Methansulfonsäure-, Benzolsulfonsäure- oder p-Toluolsulfonsäureester, in Betracht. Die Umsetzung wird, falls notwendig, unter Kühlen oder Erwärmen, z.B. in einem Temperaturbereich von etwa 0°C bis etwa 100°C, in Gegenwart eines geeigneten basischen Kondensationsmittels, z.B. eines Alkalimetalls, Alkalimetallamids oder -hydrids, oder eines Alkalimetallniederalkoxids, wie Natrium- oder Kalium-methoxid, -äthoxid oder -tert-butoxid, in An- oder Abwesenheit eines Lösungs- oder Verdünnungsmittels, bei erniedrigter oder erhöhter Temperatur, z.B. in einem Temperaturbereich von etwa 0°C bis etwa 100°C, und/oder unter atmosphärischem Druck oder in einem geschlossenen Gefäss durchgeführt.

Vorzugsweise verwendet man in solchen N-Substituierungsreaktionen in erster Linie Verbindungen der Formel I, die keine anderen primären oder sekundären Aminogruppen als Substituenten aufweisen, da diese unter Umständen ebenfalls mit dem reaktionsfähigen Ester eines Alkohols der Formel XII in Reaktion treten können.

Verbindungen der Formel I, worin Z für den Rest $NR_8R_9$ oder für $OR_7$, wobei $R_7$ gegebenenfalls substituiertes Niederalkyl ist, steht, können in gleicher Weise wie oben für Verbindungen der Formel IIa, worin $R_c$ eine funktionell abgewandelte Carboxygruppe bedeutet, beschrieben, z.B. durch Hydrolyse in alkalischem oder saurem Medium, in eine Verbindung der Formel I, worin Z für Hydroxy steht, umgewandelt werden. Umgekehrt lassen sich Verbindungen der Formel I, in denen Z Hydroxy bedeutet, in an sich bekannter Weise nach den üblichen Methoden der Veresterung, z.B. mit einem Ueberschuss eines Niederalkanols in Gegenwart einer Säure, z.B. Chlorwasserstoff, als Katalysator, in eine Verbindung der Formel I, worin Z $OR_7$ bedeutet und $R_7$ gegebenenfalls substituiertes Niederalkyl darstellt,überführen. Analog können Verbindungen der Formel I, worin Z Hydroxy bedeutet, auch nach den üblichen Methoden der Amidbildung, z.B. durch Umsetzung mit Ammoniak oder einem primären oder sekundären Amin, gegebenenfalls unter Entzug des bei der Reaktion entstehenden Wassers, in Verbindungen der Formel I, worin Z die Gruppe $NR_8R_9$ bedeutet, überführt werden.

Verbindungen der Formel I, in denen n für 1 oder 2 steht und Z Hydroxy ist, sind identisch mit Verbindungen der Formel II, worin n' für 1 oder 2 steht und $R_b$ Carboxy bedeutet. Sie können gemäss Verfahren a) durch Umsetzung mit einer Verbindung der Formel III in andere Verbindungen der Formel I überführt werden, worin n für 2 oder 3 steht.

Verbindungen der Formel I, worin Z $OR_7$ bedeutet und $R_7$ Benzyl ist, können z.B. durch Hydrogenolyse in andere Verbindungen der Formel I, worin Z für Hydroxy steht, umgewandelt werden.

Ferner können in verfahrensgemäss erhältlichen Verbindungen der Formel I vorhandene Substituenten in andere Substituenten umgewandelt werden.

So kann man z.B. veresterte Carboxygruppen, wie entsprechende Gruppen Ac und/oder $C(=O)-OR_7$, durch Umesterung in andere Ester überführen. Dabei verwendet man vorzugsweise entsprechende Alkoholverbindungen, die einen Siedepunkt aufweisen, der deutlich über demjenigen des Alkohols der veresterten Gruppe in der umzuwandelnden Verbindung der Formel I liegt, und führt die Reaktion z.B. in einem Ueberschuss der Hydroxyverbindung und/oder einem inerten organischen, vorzugsweise ebenfalls deutlich über dem Alkohol der veresterten Gruppe siedenden Lösungsmittel, vorzugsweise in Gegenwart eines Katalysators, z.B. eines Alkalimetall-niederalkoxids, wie Natrium- oder Kaliummethoxid oder -äthoxid, oder eines Orthotitansäure-niederalkylesters, wie z.B. Orthotitansäure-isopropylester, in der Wärme und üblicherweise unter Abdestillieren des freigesetzten Alkohols durch.

Veresterte Carboxygruppen, z.B. entsprechende Reste Ac oder vor allem ein Rest $Z = C(=O)-OR_7$, können z.B. auch dadurch in andere Ester überführt werden, dass man sie zunächst z.B. sauer oder bevorzugt alkalisch verseift und die erhaltene Säure in an sich bekannte Weise in einen anderen Ester überführt.

Verbindungen der Formel I mit veresterten Carboxygruppen, wie Niederalkoxycarbonylgruppen, insbesondere entsprechenden Gruppen Ac oder dem Rest $C(=O)-OR_7$, können in solche mit entsprechenden Carbamoylgruppen umgewandelt werden, z.B. durch Behandeln mit Ammoniak, ferner mono- oder di-substituierten Aminen, wenn notwenig unter erhöhter Temperatur und/oder in einem geschlossenen Gefäss.

Geeignete Substituenten eines aromatischen Restes, z.B. Halogen-, insbesondere Fluoratome, können gegen andere Substituenten, z.B. eine gegebenenfalls substituierte Aminogruppe (z.B. durch Behandeln mit einem primären oder sekundären Amin, z.B. einer Verbindung der Formel X) ausgetauscht werden.

Alle in den beschriebenen Verfahren und Nachoperationen erwähnten Ausgangsstoffe, die ein oder mehrere optisch aktive Kohlenstoffatome enthalten, können als Racematgemische, Racemate, optische Isomere oder Mischungen von optischen Isomeren in die entsprechenden Reaktionen eingesetzt werden und ergeben in der Regel Verbindungen der Formel I, worin an den entsprechenden Kohlenstoffatomen die gleiche Konfiguration oder die gleichen Konfigurationen wie im Ausgangsprodukt vorliegen.

Je nach Reaktionsbedingungen können die Verbindungen der Formel I in freier Form oder in Form von Salzen erhalten werden.

Erhaltene Säureadditionssalze können in an sich bekannter Weise in die freien Verbindungen, z.B. durch Behandeln mit einer Base, wie einem Alkalimetallhydroxid, oder in andere Salze, z.B. durch Behandeln mit geeigneten Säuren oder Derivaten davon, umgewandelt werden. Erhaltene freie Verbindungen mit salzbildenden Eigenschaften, z.B. mit entsprechenden basischen Gruppen, können z.B. durch Behandeln mit Säuren oder entsprechenden Anionenaustauschern in ihre Salze umgewandelt werden.

Infolge der engen Beziehung zwischen den Verbindungen der Formel I in freier Form und in Form von Salzen, sind im Vorausgegangenen und nachfolgend unter den freien Verbindungen oder ihren Salzen sinn- und zweckmässig gegebenenfalls auch die entsprechenden Salze bzw. freien Verbindungen zu verstehen.

Die Verbindungen, einschliesslich ihre Salze, können auch in Form ihrer Hydrate erhalten werden, oder ihre Kristalle können z.B. das zur Kristallisation verwendete Lösungsmittel einschliessen.

Die Verbindungen der Formel I können, je nach Verfahrensreaktion und/ oder Art der Ausgangsstoffe in Form von Racematen, Racematgemischen oder optischen Antipoden erhalten werden.

Erhaltene Racematgemische können auf Grund der physikalisch-chemischen Unterschiede der Racemate in bekannter Weise in die reinen Racemate bzw. Diastereomeren aufgetrennt werden, beispielsweise durch Chromatographie und/oder fraktionierte Kristallisation.

Racemate lassen sich nach an sich bekannten Methoden in die optischen Antipoden zerlegen, beispielsweise durch Umkristallisation aus einem optisch aktiven Lösungsmittel, mit Hilfe von geeigneten Mikroorganismen oder durch Umsetzen einer Verbindung der Formel I mit salz-bildenden, z.B. basischen, Eigenschaften mit einem optisch aktiven, salzbildenden Mittel, wie einer optisch aktiven Säure, und Trennen der auf diese Weise erhaltenen Gemische von Salzen, z.B. auf Grund ihrer verschiedenen Löslichkeiten, in die diastereomeren Salze, aus denen die Antipoden, z.B. durch Behandeln mit einer Base, freigesetzt werden können.

Optische Antipoden von neutralen Verbindungen der Formel I kann man z.B. auch gemäss Verfahren e) unter Verwendung einer optisch aktiven Säure der Formel IX erhalten, wobei man diese z.B. aus der entsprechenden racemischen Säure in üblicher Weise, z.B. durch Salzbildung mit einer optisch aktiven Base, Trennung der diastereomeren Salze und Freisetzung der optisch aktiven Säure, oder unter Verwenden eines reaktionsfähigen funktionellen Derivats einer optisch aktiven Säure bilden kann.

Ferner kann man z.B. Verbindungen der Formel I, die eine veresterte Carboxygruppe, z.B. eine entsprechende Gruppe Ac, aufweisen, unter Verwendung eines optisch aktiven Alkohols nach dem oben beschriebenen Verfahren umestern und das so erhältliche Diastereomerengemisch, z.B. mittels fraktionierter Kristallisation, in die Antipoden auftrennen.

Vorteilhafterweise isoliert man aus einem Diastereomerengemisch bzw. Racemat das pharmakologisch aktivere Isomere bzw. den aktiveren Antipoden. Die pharmakologische Aktivität ist in der Regel dann besonders hoch, wenn am C4-Kohlenstoff des 1,4-Dihydropyridingerüsts die folgende Konfiguration vorliegt: 4R, wenn nach der Regel von

Cahn, Ingold und Prelog der Substituent in 5-Stellung, d.h. die Aminosäure-Seitenkette, geringere Priorität als der Rest Ac in 3-Stellung des Dihydropyridingerüsts und höhere Priorität als der Rest Ar hat. Umgekehrt 4S, wenn der Rest Ac eine geringere Priorität als die Aminosäure-Seitenkette und eine höhere Priorität als der Rest Ar besitzt. Es ist also die folgende Konfiguration am C4-Kohlenstoff bevorzugt, worin die Kohlenstoffatome C3, C4 und C5 in der Papierebene, der Rest Ar vor und das Wasserstoffatom hinter der Papierebene liegt:

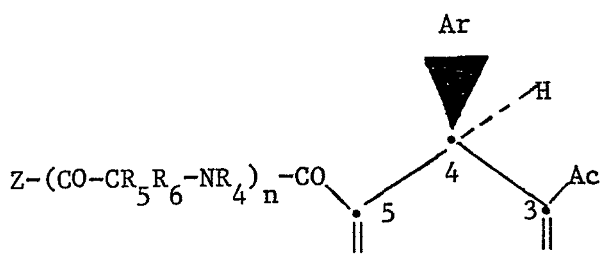

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates, z.B. Salzes, und/oder seiner Racemate bzw. Antipoden verwendet oder unter den Reaktionsbedingungen bildet.

Bei den Verfahren der vorliegenden Erfindung werden vorzugsweise solche Ausgangsstoffe verwendet, welche zu den eingangs als besonders wertvoll geschilderten Verbindungen führen. Neue Ausgangsstoffe und Verfahren zu deren Herstellung bilden ebenfalls einen Gegenstand der vorliegenden Erfindung. Die Erfindung betrifft auch verfahrensgemäss erhältliche neue Zwischenprodukte, sowie Verfahren zu ihrer Herstellung.

Die Erfindung betrifft ebenfalls die Verwendung der Verbindungen der Formel I oder von pharmazeutisch verwendbaren Salzen von solchen Verbindungen mit salzbildenden Eigenschaften, insbesondere als pharmakologisch, in erster Linie als coronardilatatorisch und/oder antihypertensiv verwendbare Verbindungen. Dabei kann man sie, vorzugsweise in Form von pharmazeutischen Präparaten, in einem Verfahren zur prophylaktischen und/oder therapeutischen Behandlung des tierischen oder menschlichen Körpers, insbesondere zur Behandlung von Angina pectoris, Hypertonie und von anderen cardiovasculären Krankheiten verwenden.

Die Dosierung des Wirkstoffs, der allein oder zusammen mit dem üblichen Träger- und Hilfsmaterial verabreicht wird, hängt von der zu behandelnden Spezies, deren Alter und individuellem Zustand, sowie der Verabreichungsweise ab. Die täglichen Dosen liegen für Mammalien mit einem Körpergewicht von etwa 70 kg, je nach Art der Erkrankung, individuellem Zustand und Alter, vorzugsweise zwischen 1 und 100 mg und insbesondere zwischen 2 und 20 mg.

Die Erfindung betrifft im weiteren pharmazeutische Präparate, die Verbindungen der Formel I oder pharmazeutisch verwendbare Salze von solchen Verbindungen mit salzbildenden Eigenschaften als Wirkstoffe enthalten, sowie Verfahren zu ihrer Herstellung.

Bei den erfindungsgemässen pharmazeutischen Präparaten handelt es sich um solche zur enteralen, wie peroralen oder rektalen, weiter zur sublingualen, sowie zur parenteralen Verabreichung an Warmblüter. Entsprechende Dosiseinheitsformen, insbesondere zur peroralen und/oder sublingualen Verabreichung, z.B. Dragées, Tabletten oder Kapseln, enthalten vorzugsweise von etwa 0,5 bis etwa 100 mg, insbesondere von etwa 5 bis etwa 50 mg einer Verbindung der Formel I oder eines pharmazeutisch annehmbaren Salzes einer zur Salzbildung befähigten entsprechenden Verbindung zusammen mit pharmazeutisch verwendbaren Trägerstoffen.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose und/oder Polyvinylpyrrolidon, und/oder, wenn erwünscht, Sprengmittel, wie die obgenannten Stärken, ferner Carboxymethylstärke, quervernetztes Polyvinylpyrroli-

don, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyäthylenglykol und/oder Titandioxid enthalten, oder Lacklösungen in geeigneten organischen Lösungsmitteln oder Lösungsmittelgemischen, oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Lactose, Bindemitteln, wie Stärken und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können. Bevorzugt sind u.a. Kapseln, die sowohl leicht zerbissen werden können, um z.B. bei Anzeichen eines Anfalls von Angina pectoris durch sublinguale Aufnahme des Wirkstoffes eine möglichst rasche Wirkung zu erzielen, als auch unzerkaut geschluckt werden können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner

können auch Gelatine-Rektalkapseln verwendet werden, die eine Kombination des Wirkstoffes mit einer Grundmasse enthalten; als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässerige Lösungen eines Wirkstoffes in wasserlöslicher Form, z.B. eines wasserlöslichen Salzes, ferner Suspensionen des Wirkstoffes, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat, oder Triglyceride verwendet, oder wässrige Injektionssuspensionen, welche viskositätserhöhende Stoffe, z.B. Natriumcarboxymethylcellulose, Sorbit und/oder Dextran und gegebenenfalls Stabilisatoren enthalten.

Die pharmazeutischen Präparate der vorliegenden Erfindung können in an sich bekannter Weise, z.B. mittels konventioneller Misch-, Granulier-, Dragier-, Lösungs- oder Lyophilisierungsverfahren hergestellt werden. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Die nachfolgenden Beispiele a) bis c) sollen die Herstellung einiger typischer Applikationsformen erläutern, jedoch keineswegs die einzigen Ausführungsformen von solchen darstellen.

a) Tabletten enthaltend 25 mg Wirkstoff können wie folgt hergestellt werden:

Zusammensetzung: (für 1000 Tabletten)

| | |
|---|---|
| Wirkstoff | 25.0 g |
| Maisstärke | 70.0 g |
| Lactose (fein) | 78.5 g |
| Cellulose | 75.0 g |

| | |
|---|---|
| Magnesiumstearat | 1.5 g |
| Wasser | q.s. |

Der Wirkstoff wird mit 60 g Maisstärke und der Lactose vermischt und mit einem aus 10 g Maisstärke und Wasser hergestellten Kleister geknetet. Die feuchte Masse wird granuliert, getrocknet und mit der kristallinen Cellulose und dem Magnesiumstearat gemischt. Die homogene Mischung wird zu Tabletten von 250 mg (mit Bruchrille) verpresst; diese haben einen Durchmesser von 9 mm.

b) Kapseln enthaltend 10 mg Wirkstoff können wie folgt hergestellt werden:

Zusammensetzung:

| | |
|---|---|
| Wirkstoff | 2500 mg |
| Talkum | 200 mg |
| Kolloidale Kieselsäure | 50 mg |

Der Wirkstoff wird mit Talkum und kolloidaler Kieselsäure innig gemischt, das Gemisch durch ein Sieb mit 0,5 mm Maschenweite getrieben und dieses in Portionen von jeweils 11 mg in Hartgelatinekapseln geeigneter Grösse abgefüllt.

c) Eine sterile Lösung von 5.0 g Wirkstoff in 5000 ml destilliertem Wasser wird in Ampullen zu 5 ml gefüllt, die in 5 ml Lösung 5 mg Wirkstoff enthalten.

Die nachfolgenden Beispiele illustrieren die Herstellung der neuen Verbindungen der Formel I, sollen jedoch den Umfang der Erfindung in keiner Weise beschränken. Temperaturen werden in Celsiusgraden angegeben.

Beispiel 1: Ein Gemisch von 9.6 g 1,4-Dihydro-2,6-dimethyl-4-(3-nitrophenyl)-pyridin-3,5-dicarbonsäure-3-(1-benzotriazolyl)-ester-5-methylester, 3.6 g L-Valin-äthylester-hydrochlorid und 2.5 ml N-Aethylmorpholin in 90 ml wasserfreiem Dimethylformamid wird während 16 Stunden bei 80° unter Stickstoffatmosphäre gerührt. Das gelbe Reaktionsgemisch versetzt man unter Eiswasserkühlung mit 300 ml Eiswasser. Anschliessend wird noch während 1 Stunde im Eisbad bei 0-5° gerührt, wobei sich das Rohprodukt als hochviskose Masse abscheidet. Das Lösungsmittel wird abdekantiert und der Rückstand unter vermindertem Druck getrocknet. Das so erhältliche harzige Rohprodukt ist ein 1:1 Diastereomerengemisch von (4R)- und (4S)-1,4-Dihydro-2,6-dimethyl-3-methoxycarbonyl-4-(3-nitrophenyl)-pyridin-5-carbonsäure-N-[(1S)-1-äthoxycarbonyl-2-methyl-1-propyl]-amid.

Zur Trennung der Diastereomeren wird das Rohprodukt mit 30 ml Diisopropyläther versetzt und während 1 Stunde bei 0-5° gerührt, wobei (+)-(4S)-1,4-Dihydro-2,6-dimethyl-3-methoxycarbonyl-4-(3-nitrophenyl)-pyridin-5-carbonsäure-N-[(1S)-1-äthoxycarbonyl-2-methyl-1-propyl]-amid in Form schwach gelber Kristalle kristallisiert. Dieses erste Diastereomere ist konfigurativ einheitlich; es schmilzt bei 198-200° und seine spezifische Drehung beträgt $[\alpha]_D^{20} = +75°$ (c = 0.6, Aethanol).

Zur Isolierung des zweiten Diastereomeren wird die Mutterlauge unter vermindertem Druck eingedampft. Man reinigt den Rückstand durch Chromatographie an der etwa 100-fachen Menge Silikagel (Elution mit einem 7:3 Gemisch von Hexan und Essigsäureäthylester) und kristallisiert das rohe zweite Diastereomere durch Rühren in 20 ml Diäthyläther bei 0-5°. Das so erhältliche (-)-(4R)-1,4-Dihydro-2,6-dimethyl-3-methoxycarbonyl-4-(3-nitrophenyl)-pyridin-5-carbonsäure-N-[(1S)-1-äthoxycarbonyl-2-methyl-1-propyl]-amid schmilzt bei 147-148°. Das Rohprodukt wird durch Umkristallisation aus Aceton weiter gereinigt. Die so erhaltenen hellgelben, feinblättrigen Nadeln schmelzen bei 156-157°, wobei gleichzeitig als neue Kristallmodifikation tiefgelbe, rhombische Kristalle aus der Schmelze zu wachsen beginnen; erneutes Schmelzen findet statt bei 171-172°. Dieses kristalline zweite Diastereomere ist konfigurativ einheitlich und zeigt eine spezifische Drehung von $[\alpha]_D^{20} = -34.9°$ (c = 1.0, Aethanol).

Das Ausgangsmaterial kann wie folgt hergestellt werden:
Eine Lösung von 6.7 g 1,4-Dihydro-2,6-dimethyl-4-(3-nitrophenyl)-pyridin-3,5-dicarbonsäure-monomethylester [s. Europäische Patentanmeldung 11.706], 3.4 g 1-Hydroxybenzotriazol und 4.6 g N,N'-Dicyclohexylcarbodiimid in 100 ml wasserfreiem Dimethylformamid wird während 16 Stunden bei 0-5° unter Stickstoffatmosphäre stehen gelassen. Der auskristallisierte N,N'-Dicyclohexylharnstoff wird abfiltriert. Man versetzt das gelbe Filtrat unter Eisbadkühlung mit 300 ml Wasser und rührt noch während 1 Stunde bei 0-5°, wobei das amorphe Rohprodukt abgeschieden wird. Es wird abfiltriert, der Filterrückstand mit 400 ml Wasser gewaschen und im Vakuum getrocknet. Zur weiteren Reinigung wird das Rohprodukt in 40 ml Essigsäureäthylester gelöst und während 1 Stunde bei 0-5° gerührt. Anschliessend entfernt man den auskristallisierten N,N'-Dicyclohexylharnstoff durch Filtration und dampft das Filtrat unter vermindertem Druck ein, wobei 1,4-Dihydro-2,6-dimethyl-4-(3-nitrophenyl)-pyridin-3,5-dicarbonsäure-3-(1-benzotriazolyl)-ester-5-methylester als hochviskoser Schaum in quantitativer Ausbeute erhalten wird:
250 MHz FT-$^1$H-NMR ($CDCl_3$): 2.46 (2s, 6H, Dihydropyridyl-$CH_3$); 3.70 (s, 3H, -$COOCH_3$); 5.44 (s, 1H, 4-Dihydropyridyl-H); 6.78 (s, 1H, N-H); 6.92, 7.39, 8.04 (3m, 4H, Benzotriazolyl-H); 6.48, 7.79, 8.14, 8.24 (4m, 4H, Phenyl-H).

Beispiel 2: Ein Gemisch von 12.5 g (S)-N-Acetoacetyl-α-amino-isovaleriansäure-äthylester [hergestellt analog Pharm. Acta Helv. 38, 616 (1963)], 6.2 g 3-Amino-crotonsäure-methylester und 8.2 g 3-Nitrobenzaldehyd in 100 ml Aethanol wird während 16 Stunden bei 80° unter Stickstoffatmosphäre gerührt. Das gelbe Reaktionsgemisch wird unter vermindertem Druck eingedampft. Der ölige Rückstand wird in 300 ml Dichlormethan gelöst und zweimal mit je 100 ml Wasser gewaschen. Man trocknet die organische Phase mit Natriumsulfat, filtriert und dampft das Filtrat unter vermindertem Druck ein. Den Rückstand reinigt man durch Chromatographie an der etwa 100-fachen Menge Silikagel (Elution mit einem 7:3 Gemisch von Hexan und Essigsäureäthylester). Das so erhältliche harzige Rohprodukt ist ein 1:1 Diastereomerengemisch von (4R)- und (4S)-1,4-Dihydro-2,6-dimethyl-3-methoxycarbonyl-4-(3-nitrophenyl)-pyridin-5-carbonsäure-N-[(1S)-1-äthoxycarbonyl-2-methyl-1-propyl]-amid. Die Trennung der Diastereomeren erfolgt wie im Beispiel 1 beschrieben.

Beispiel 3: Zu einer Lösung von 3.5 g 1,4-Dihydro-2,6-dimethyl-3-methoxycarbonyl-4-(3-nitrophenyl)-pyridin-5-carbonsäurechlorid [s. Chem. Pharm. Bull. 28, 2809 (1980)] und 1.3 ml N-Aethylmorpholin in 30 ml wasserfreiem Tetrahydrofuran tropft man bei 0-10° eine Lösung von 1.8 g L-Valin-äthylester-hydrochlorid in 30 ml wasserfreiem Tetrahydrofuran. Nach Ende der Zugabe lässt man auf Raumtemperatur erwärmen. Das Reaktionsgemisch wird unter vermindertem Druck eingedampft. Der Rückstand wird in 100 ml Dichlormethan gelöst

und mehrmals mit Wasser gewaschen. Man trocknet die organische Phase mit Natriumsulfat, filtriert und dampft das Filtrat unter vermindertem Druck ein. Der Rückstand wird durch Chromatographie an der etwa 100-fachen Menge Silikagel gereinigt (Elution mit einem 3:7 Gemisch von Essigsäureäthylester und Hexan). Das so erhältliche Rohprodukt ist ein 1:1 Diastereomerengemisch von (4R)- und (4S)-1,4-Dihydro-2,6-dimethyl-3-methoxycarbonyl-4-(3-nitrophenyl)-pyridin-5-carbonsäure-N-[(1S)-1-äthoxycarbonyl-2-methyl-1-propyl]-amid. Die Trennung der Diastereomeren erfolgt wie im Beispiel 1 beschrieben.

Beispiel 4: Analog dem im Beispiel 1 beschriebenen Verfahren erhält man aus einem Gemisch von 42 g 1,4-Dihydro-2,6-dimethyl-4-(3-nitrophenyl)-pyridin-3,5-dicarbonsäure-3-(1-benzotriazolyl)-ester-5-methylester, 16 g D-Valin-äthylester-hydrochlorid und 11 ml N-Aethylmorpholin in 350 ml wasserfreiem Dimethylformamid (-)-(4R)-1,4-Dihydro-2,6-dimethyl-3-methoxycarbonyl-4-(3-nitrophenyl)-pyridin-5-carbonsäure-N-[(1R)-1-äthoxycarbonyl-2-methyl-1-propyl]-amid, das nach Umkristallisation aus einem Gemisch von Aceton und Diisopropyläther bei 199-200° schmilzt. Dieses Diastereomere ist konfigurativ einheitlich und zeigt eine spezifische Drehung von $[\alpha]_D^{20}$ = -75° (c = 0.7, Aethanol).

Aus der Mutterlauge wird, analog dem im Beispiel 1 beschriebenen Verfahren, (+)-(4S)-1,4-Dihydro-2,6-dimethyl-3-methoxycarbonyl-4-(3-nitrophenyl)-pyridin-5-carbonsäure-N-[(1R)-1-äthoxycarbonyl-2-methyl-1-propyl]-amid isoliert. Durch Verreiben des amorphen Rohproduktes mit Diäthyläther kristallisiert dieses Diastereomere in konfigurativ einheitlicher Form; es schmilzt bei 144-145° und zeigt eine spezifische Drehung von $[\alpha]_D^{20}$ = +30° (c= 0.4, Aethanol).

Beispiel 5: Analog dem im Beispiel 1 beschriebenen Verfahren erhält man aus einem Gemisch von 10.2 g 1,4-Dihydro-2,6-dimethyl-4-(3-nitrophenyl)-pyridin-3,5-dicarbonsäure-3-(1-benzotriazolyl)-5-isopropylester, 3.5 g L-Valin-äthylester-hydrochlorid und 2.5 ml

N-Aethylmorpholin in 90 ml wasserfreiem Dimethylformamid (-)-(4R)-1,4-Dihydro-2,6-dimethyl-3-isopropoxycarbonyl-4-(3-nitrophenyl)-pyridin-5-carbonsäure-N-[(1S)-1-äthoxycarbonyl-2-methyl-1-propyl)-amid, das nach Umkristallisation aus Essigsäureäthylester bei 178-179° schmilzt. Die spezifische Drehung dieses konfigurativ einheitlichen Diastereomeren beträgt $[\alpha]_D^{20} = -28°$ (c = 0.9, Aethanol).

Aus der Mutterlauge wird, analog dem im Beispiel 1 beschriebenen Verfahren,(+)-(4S)-1,4-Dihydro-2,6-dimethyl-3-isopropoxycarbonyl-4-(3-nitrophenyl)-pyridin-5-carbonsäure-N-[(1S)-1-äthoxycarbonyl-2-methyl-1-propyl]-amid in amorpher Form isoliert. Die spezifische Drehung dieses amorphen Diastereomeren beträgt $[\alpha]_D^{20} = +46°$ (c = 0.45, Aethanol); es enthält laut $^1$H-NMR-Spektrum noch ca. 20% an (-)-(4R)-1,4-Dihydro-2,6-dimethyl-3-isopropoxycarbonyl-4-(3-nitrophenyl)-pyridin-5-carbonsäure-N-[(1S)-1-äthoxycarbonyl-2-methyl-1-propyl]-amid.

Das Ausgangsmaterial kann wie folgt erhalten werden. Analog dem im Beispiel 1 beschriebenen Verfahren erhält man aus einem Gemisch von 16.2 g 1,4-Dihydro-2,6-dimethyl-4-(3-nitrophenyl)-pyridin-3,5-dicarbonsäure-monoisopropylester [s. Europäische Patentanmeldung 11.706], 7.6 g 1-Hydroxybenzotriazol und 10.2 g N,N'-Dicyclohexylcarbodiimid in 225 ml wasserfreiem Dimethylformamid 1,4-Dihydro-2,6-dimethyl-4-(3-nitrophenyl)-pyridin-3,5-dicarbonsäure-3-(1-benzotriazolyl)-ester-5-isopropylester als amorphes Produkt.

250 mHz FT-$^1$H-NMR ($CDCl_3$): 1.13, 1.30 (2d, 6H, Isopropyl-$CH_3$); 2.44, 2.46 (2s, 6H, Dihydropyridyl-$CH_3$); 5.00 (m, 1H, -O-CH-); 5.40 (s, 1H, 4-Dihydropyridyl-H); 6.28 (s, 1H, -NH-); 6.89, 7.40, 8.05 (3m, 4H, Benzotriazolyl-H); 7.49, 7.79, 8.15, 8.25 (4m, 4H, Phenyl-H).

Beispiel 6: Analog dem im Beispiel 1 beschriebenen Verfahren erhält man aus einem Gemisch von 30 g 1,4-Dihydro-2,6-dimethyl-4-(3-nitrophenyl)-pyridin-3,5-dicarbonsäure-3-(1-benzotriazolyl)-ester-5-isopropylester, 9.8 g D-Valin-äthylester-hydrochlorid und 6.8 ml N-Aethylmorpholin in 120 ml wasserfreiem Dimethylformamid (+)-(4S)-1,4-Dihydro-2,6-dimethyl-3-isopropoxycarbonyl-4-(3-nitrophenyl)-pyridin-5-carbonsäure-N-[(1R)-1-äthoxycarbonyl-2-methyl-1-propyl]-amid. Dieses konfigurativ einheitliche Diastereomere schmilzt bei 176-177° und zeigt eine spezifische Drehung von $[\alpha]_D^{20}$ = +24 (c = 0.53, Aethanol).

Aus der Mutterlauge wird, analog dem im Beispiel 1 beschriebenen Verfahren, (-)-(4R)-1,4-Dihydro-2,6-dimethyl-3-isopropoxycarbonyl-4-(3-nitrophenyl)-pyridin-5-carbonsäure-N-[(1R)-1-äthoxycarbonyl-2-methyl-1-propyl]-amid in amorpher Form isoliert. Die spezifische Drehung dieses amorphen Diastereomeren beträgt $[\alpha]_D^{20}$ = -47° (c = 0.65, Aethanol); es enthält laut $^1$H-NMR Spektrum noch ca. 5% an (+)-(4S)-1,4-Dihydro-2,6-dimethyl-3-isopropoxycarbonyl-4-(3-nitrophenyl)-pyridin-5-carbonsäure-N-[(1R)-1-äthoxycarbonyl-2-methyl-1-propyl]-amid.

Beispiel 7: Analog dem im Beispiel 1 beschriebenen Verfahren erhält man aus einem Gemisch von 42 g 1,4-Dihydro-2,6-dimethyl-4-(3-nitrophenyl)-pyridin-3,5-dicarbonsäure-3-(1-benzotriazolyl)-ester-5-isopropylester, 14 g L-Leucin-methylester-hydrochlorid und 9.6 ml N-Aethylmorpholin in 250 ml wasserfreiem Dimethylformamid (+)-(4S)-1,4-Dihydro-2,6-dimethyl-3-isopropoxycarbonyl-4-(3-nitrophenyl)-pyridin-5-carbonsäure-N-[(1S)-1-methoxycarbonyl-3-methyl-1-butyl]-amid, das nach Umkristallisation aus Essigsäureäthylester bei 176-177° schmilzt. Dieses kristalline Diastereomere ist konfigurativ einheitlich und zeigt eine spezifische Drehung von $[\alpha]_D^{20}$= + 106° (c = 0.48, Aethanol).

Aus der Mutterlauge wird analog dem im Beispiel 1 beschriebenen Verfahren (-)-(4R)-1,4-Dihydro-2,6-dimethyl-3-isopropoxycarbonyl-4-(3-nitrophenyl)-pyridin-5-carbonsäure-N-[(1S)-1-methoxycarbonyl-3-methyl-1-butyl]-amid isoliert. Durch Verreiben des amorphen Rohproduktes mit Diäthyläther kristallisiert dieses Diastereomere in konfigurativ einheitlicher Form; es schmilzt bei 154-155° und zeigt eine spezifische Drehung von $[\alpha]_D^{20}$ = -55° (c = 0.78, Aethanol).

Beispiel 8: Analog dem im Beispiel 1 beschriebenen Verfahren erhält man aus einem Gemisch von 21 g 1,4-Dihydro-2,6-dimethyl-4-(3-nitrophenyl)-pyridin-3,5-dicarbonsäure-3-(1-benzotriazolyl)-ester-5-isopropylester, 6.9 g D-Leucin-methylester-hydrochlorid und 4.8 ml N-Aethylmorpholin in 120 ml wasserfreiem Dimethylformamid (-)-(4R)-1,4-Dihydro-2,6-dimethyl-3-isopropoxycarbonyl-4-(3-nitrophenyl)-pyridin-5-carbonsäure-N-[(1R)-1-methoxycarbonyl-3-methyl-1-butyl]-amid, das nach Umkristallisation aus Essigsäureäthylester bei 175-176° schmilzt. Dieses konfigurativ einheitliche Diastereomere zeigt eine spezifische Drehung von $[\alpha]_D^{20}$= -109° (c = 0.28, Aethanol).

Aus der Mutterlauge wird, analog dem im Beispiel 1 beschriebenen Verfahren, (+)-(4S)-1,4-Dihydro-2,6-dimethyl-3-isopropoxycarbonyl-4-(3-nitrophenyl)-pyridin-5-carbonsäure-N-[(1R)-1-methoxycarbonyl-3-methyl-1-butyl]-amid in amorpher, jedoch konfigurativ einheitlicher Form isoliert. Die spezifische Drehung dieses Diastereomeren beträgt $[\alpha]_D^{20}$ = + 48° (c = 0.79, Aethanol).

Beispiel 9: Analog dem im Beispiel 1 beschriebenen Verfahren erhält man aus einem Gemisch von 23 g 1,4-Dihydro-2,6-dimethyl-4-(3-nitrophenyl)-pyridin-3,5-dicarbonsäure-3-(1-benzotriazolyl)-ester-5-methylester, 9.3 g D-Leucin-äthylester-hydrochlorid und 3.5 ml N-Aethylmorpholin in 150 ml wasserfreiem Dimethylformamid (-)-(4R)-1,4-Dihydro-2,6-dimethyl-3-methoxycarbonyl-4-(3-nitrophenyl)-pyridin-5-carbonsäure-N-[(1R)-1-äthoxycarbonyl-3-methyl-1-butyl]-amid,

das nach Umkristallisation aus einem Gemisch von Essigsäureäthylester und Diisopropyläther bei 207-209° schmilzt. Dieses Diastereomere ist konfigurativ einheitlich, die spezifische Drehung beträgt $[\alpha]_D^{20}$ = -116° (c = 0.76, Aethanol).

Aus der Mutterlauge wird, analog dem im Beispiel 1 beschriebenen Verfahren, (+)-(4S)-1,4-Dihydro-2,6-dimethyl-3-methoxycarbonyl-4-(3-nitrophenyl)-pyridin-5-carbonsäure-N-[(1R)-1-äthoxycarbonyl-3-methyl-1-butyl]-amid isoliert. Nach Umkristallisation des Rohproduktes aus einem Gemisch von Diäthyläther und Hexan erhält man ein kristallines Produkt, das bei 112-114° schmilzt und dessen spezifische Drehung $[\alpha]_D^{20}$ = +21° (c = 0.5, Aethanol) beträgt. Diese kristalline Form enthält auf Grund des $^1$H-NMR Spektrums noch 5-10% (-)-(4R)-1,4-Dihydro-2,6-dimethyl-3-methoxycarbonyl-4-(3-nitrophenyl)-pyridin-5-carbonsäure-N-[(1R)-1-äthoxycarbonyl-3-methyl-1-butyl]-amid.

Beispiel 10: Analog dem im Beispiel 1 beschriebenen Verfahren erhält man aus einem Gemisch von 19.2 g 1,4-Dihydro-2,6-dimethyl-4-(3-nitrophenyl)-pyridin-3,5-dicarbonsäure-3-(1-benzotriazolyl)-ester-3-methylester, 7.8 g L-Leucin-äthylester-hydrochlorid und 2.9 ml N-Aethylmorpholin in 120 ml wasserfreiem Dimethylformamid (+)-(4S)-1,4-Dihydro-2,6-dimethyl-3-methoxycarbonyl-4-(3-nitrophenyl)-pyridin-5-carbonsäure-N-[(1S)-1-äthoxycarbonyl-3-methyl-1-butyl]-amid, das nach Umkristallisation aus einem Gemisch von Essigsäureäthylester und Diisopropyläther bei 206-207° schmilzt. Diese kristalline Form ist konfigurativ einheitlich, die spezifische Drehung beträgt $[\alpha]_D^{20}$ = + 105° (c = 0.26, Aethanol).

Aus der Mutterlauge wird, analog dem im Beispiel 1 beschriebenen Verfahren, (-)-(4R)-1,4-Dihydro-2,6-dimethyl-3-methoxycarbonyl-4-(3-nitrophenyl)-pyridin-5-carbonsäure-N-[(1S)-1-äthoxycarbonyl-3-methyl-1-butyl]-amid als Rohprodukt isoliert. Durch Umkristallisation aus einem Gemisch von Diäthyläther und Hexan erhält man ein kristallines

Produkt, das bei 118-120° schmilzt und dessen spezifische Drehung $[\alpha]_D^{20}$ = -23° (c = 0.7, Aethanol) beträgt. Diese kristalline Form enthält auf Grund des $^1$H-NMR Spektrums noch ca. 20% (+)-(4S)-1,4-Dihydro-2,6-dimethyl-3-methoxycarbonyl-4-(3-nitrophenyl)-pyridin-5-carbonsäure-N-[(1S)-1-äthoxycarbonyl-3-methyl-1-butyl]-amid.

Beispiel 11: Analog dem im Beispiel 1 beschriebenen Verfahren erhält man eine einem Gemisch von 25 g 1,4-Dihydro-2,6-dimethyl-4-(3-nitrophenyl)-pyridin-3,5-dicarbonsäure-3-(1-benzotriazolyl)-ester-5-methylester, 8.6 g L-Prolin-methylester-hydrochlorid und 6.6 ml N-Aethylmorpholin in 120 ml wasserfreiem Dimethylformamid (+)-(4S)-5-[(2S)-2-Methoxycarbonyl-pyrrolidin-1-yl-carbonyl]-1,4-dihydro-2,6-dimethyl-3-methoxycarbonyl-4-(3-nitrophenyl)-pyridin, das nach Umkristallisation aus einem Gemisch von Essigsäureäthylester und Hexan bei 180-181° schmilzt. Dieses kristalline Diastereomere ist konfigurativ einheitlich, die spezifische Drehung beträgt $[\alpha]_D^{20}$= + 104° (c = 0.7, Aethanol).

Aus der Mutterlauge wird, analog dem im Beispiel 1 beschriebenen Verfahren, (-)-(4R)-5-[(2S)-2-Methoxycarbonyl-pyrrolidin-1-yl-carbonyl]-1,4-dihydro-2,6-dimethyl-3-methoxycarbonyl-4-(3-nitrophenyl)-pyridin isoliert, das nach Umkristallisation aus einem Gemisch von Essigsäureäthylester und Diisopropyläther bei 118-119° schmilzt. Dieses konfigurativ einheitliche Diastereomere zeigt eine spezifische Drehung von $[\alpha]_D^{20}$ = -59° (c = 0.5, Aethanol).

Beispiel 12: Analog dem im Beispiel 1 beschriebenen Verfahren erhält man aus einem Gemisch von 23 g 1,4-Dihydro-2,6-dimethyl-4-(3-nitrophenyl)-pyridin-3,5-dicarbonsäure-3-(1-benzotriazolyl)-ester-5-methylester, 7.9 g D-Prolin-methylester-hydrochlorid und 6 ml N-Aethylmorpholin in 150 ml wasserfreiem Dimethylformamid (-)-(4R)-5-[(2R)-2-Methoxycarbonyl-pyrrolidin-1-yl-carbonyl]-1,4-dihydro-2,6-dimethyl-3-methoxycarbonyl-4-(3-nitrophenyl)-pyridin, das nach Umkristallisation aus einem Gemisch von Aceton und Diisopropyläther bei 180-181° schmilzt. Dieses kristalline Diastereomere ist konfigurativ einheitlich und zeigt eine spezifische Drehung von $[\alpha]_D^{20}$ = -97° (c = 0.97, Aethanol).

Aus der Mutterlauge wird, analog dem im Beispiel 1 beschriebenen Verfahren, (+)-(4S)-5-[(2R)-2-Methoxycarbonyl-pyrrolidin-1-yl-carbonyl]-1,4-dihydro-2,6-dimethyl-3-methoxycarbonyl-4-(3-nitrophenyl)-pyridin isoliert, das nach Umkristallisation aus einem Gemisch von Aceton und Diäthyläther bei 120-122° schmilzt. Dieses konfigurativ einheitliche Diastereomere zeigt eine spezifische Drehung von $[\alpha]_D^{20}$ = +56° (c = 0.55, Aethanol).

Beispiel 13: Analog dem im Beispiel 1 beschriebenen Verfahren erhält man aus einem Gemisch von 18 g 1,4 Dihydro-2,6-dimethyl-4-(3-nitrophenyl)-pyridin-3,5-dicarbonsäure-3-(1-benzotriazolyl)-ester-5-methylester, 4.7 g Glycin-methylester-hydrochlorid und 4.7 ml N-Aethylmorpholin in 150 ml wasserfreiem Dimethylformamid racemisches 1,4-Dihydro-2,6-dimethyl-3-methoxycarbonyl-4-(3-nitrophenyl)-pyridin-5-carbonsäure-N-(methoxycarbonyl-methyl)-amid in amorpher Form.
250 MHz FT-$^1$H-NMR ($CDCl_3$): 2.32, 2.35 (2s, 6H, Dihydropyridyl-$CH_3$); 3.67, 3.73 (2s, 6H, -$COOCH_3$); 4.00 (d, 2H, -N-$CH_2$); 4.96 (s, 1H, 4-Dihydropyridyl-H); 5.75 (s, 1H, 1-Dihydropyridyl-H); 5.90 (t, 1H, -CONH); 7.4-8.18 (m, 4H, Phenyl-H).

Beispiel 14: Analog dem im Beispiel 1 beschriebenen Verfahren erhält man aus einem Gemisch von 15 g 1,4-Dihydro-2,6-dimethyl-4-(3-nitrophenyl)-pyridin-3,5-dicarbonsäure-3-(1-benzotriazolyl)-ester-5-methylester, 5.2 g α-Aminoisobuttersäure-äthylester-hydrochlorid und 3.6 ml N-Aethylmorpholin in 100 ml wasserfreiem Dimethylformamid racemisches 1,4-Dihydro-2,6-dimethyl-3-methoxycarbonyl-4-(3-nitrophenyl)-pyridin-5-carbonsäure-N-(1-äthoxycarbonyl-1-methyl-1-äthyl)-amid, das nach Umkristallisation aus einem Gemisch von Essigsäure-

äthylester und Diäthyläther bei 160-161° schmilzt.

Beispiel 15: Analog dem im Beispiel 1 beschriebenen Verfahren erhält man aus einem Gemisch von 18.5 g 1,4-Dihydro-2,6-dimethyl-4-(3-nitrophenyl)-pyridin-3,5-dicarbonsäure-3-(1-benzotriazolyl)-ester-5-methylester, 6 g L-Alanin-äthylester-hydrochlorid und 4.6 ml N-Aethylmorpholin in 100 ml wasserfreiem Dimethylformamid ein 1:1 Diastereomerengemisch von (4R)- und (4S)-1,4-Dihydro-2,6-dimethyl-3-methoxycarbonyl-4-(3-nitrophenyl)-pyridin-5-carbonsäure-N-[(1S)-1-äthoxycarbonyl-1-äthyl]-amid, das nach Umkristallisation aus einem Gemisch von Essigsäureäthylester und Diisopropyläther bei 134-135° schmilzt.

Beispiel 16: Analog dem im Beispiel 1 beschriebenen Verfahren erhält man aus einem Gemisch von 21.7 g 1,4-Dihydro-2,6-dimethyl-4-(3-nitrophenyl)-pyridin-3,5-dicarbonsäure-3-(1-benzotriazolyl)-ester-5-methylester und 8.2 g L-Tyrosin-methylester in 100 ml wasserfreiem Dimethylformamid ein 1:1 Diastereomerengemisch von (4R)- und (4S)-1,4-Dihydro-2,6-dimethyl-3-methoxycarbonyl-4-(3-nitrophenyl)-pyridin-5-carbonsäure-N-[(1S)-1-methoxycarbonyl-2-(4-hydroxyphenyl)-1-äthyl]-amid in amorpher Form. 250 MHz FT $^1$H-NMR ($CDCl_3$): 2.18, 2.21, 2.30, 2.32 (4s, 6H, Dihydropyridyl-$CH_3$); 2.80-3.10 (m, 2H, Phenyl-$CH_2$); 3.65, 3.75 (2s, 6H, -$COOCH_3$); 4.74-4.94 (m, 1H, -CON-CH-); 4.83 (s, 1H, 4-Dihydropyridyl-H), 5.5-5.8 (m, 3H, -NH und -OH); 6.52-6.78 (m, 4H, Hydroxyphenyl-H); 7.30-8.06 (m, 4H, Nitrophenyl-H).

Beispiel 17: Analog dem im Beispiel 1 beschriebenen Verfahren erhält man aus einem Gemisch von 20 g 1,4-Dihydro-2,6-dimethyl-4-(3-nitrophenyl)-pyridin-3,5-dicarbonsäure-3-(1-benzotriazolyl)-ester-5-methylester, 11.5 g D,L-α-Phenylglycin-benzylester-hydrochlorid und 5.2 ml N-Aethylmorpholin in 90 ml wasserfreiem Dimethylformamid ein racemisches 1:1 Diastereomerengemisch von 1,4-Dihydro-2,6-dimethyl-3-methoxycarbonyl-4-(3-nitrophenyl)-pyridin-5-carbonsäure-N-(1-benzyloxycarbonyl-1-benzyl)-amid in amorpher Form. 250 MHz FT-$^1$H-NMR

($CDCl_3$): 2.23, 2.26, 2.32, 2.33 (4s, 6H, Dihydropyridyl-H); 3.63, 2.67 (2s, 3H, $-COOCH_3$); 4.96, 5.09 (2s, 2H, $-COOCH_2$); 5.15, 5.16 (2s, 1H, 4-Dihydropyridyl-H); 5.55 (m, 1H, -N-CH-); 5.6, 5.65 (2s, 1H, 1-Dihydropyridyl-H); 6.32, 6.40, (2d, 1H, -CONH-); 7.04-8.12 (m, 14H, Phenyl-H).

Beispiel 18: Analog dem im Beispiel 1 beschriebenen Verfahren erhält man aus einem Gemisch von 20 g 4-(2-Difluormethoxyphenyl)-1,4-dihydro-2,6-dimethylpyridin-3,5-dicarbonsäure-3-(1-benzotriazolyl)-ester-5-methylester, 7 g L-Valin-äthylester-hydrochlorid und 4.9 ml N-Aethylmorpholin in 150 ml wasserfreiem Dimethylformamid ein 1:1 Diastereomerengemisch von (4R)- und (4S)-4-(2-Difluormethoxyphenyl)-1,4-dihydro-2,6-dimethyl-3-methoxycarbonyl-pyridin-5-carbonsäure-N-[(1S)-1-äthoxycarbonyl-2-methyl-1-propyl]-amid, das nach Umkristallisation aus einem Gemisch von Diäthyläther und Diisopropyläther bei 98-99° schmilzt.

Das Ausgangsmaterial kann wie folgt erhalten werden:
Analog dem im Beispiel 1 beschriebenen Verfahren erhält man aus einem Gemisch von 17 g 4-(2-Difluormethoxyphenyl)-1,4-dihydro-2,6-dimethylpyridin-3,5-dicarbonsäure-monomethylester [Herstellung analog EP 11.706; Smp. 150-152°], 8 g 1-Hydroxybenzotriazol und 11 g N,N'-Dicyclohexylcarbodiimid in 150 ml wasserfreiem Dimethylformamid 4-(2-Difluormethoxyphenyl)-1,4-dihydro-2,6-dimethyl-pyridin-3,5-dicarbonsäure-3-(1-benzotriazolyl)-ester-5-methylester als Rohprodukt in amorpher Form, das ohne weitere Reinigung in der folgenden Umsetzung eingesetzt wird.

Beispiel 19: Analog dem im Beispiel 1 beschriebenen Verfahren erhält man aus einem Gemisch von 20 g 1,4-Dihydro-2,6-dimethyl-4-(3-nitrophenyl)-pyridin-3,5-dicarbonsäure-3-(1-benzotriazolyl)-ester-5-methylester, 8.9 g L-α-Phenylglycin-äthylester-hydrochlorid und 5.2 ml

N-Aethylmorpholin in 150 ml wasserfreiem Dimethylformamid ein 1:1 Diastereomerengemisch von (4R)- und (4S)-1,4-Dihydro-2,6-dimethyl-3-methoxycarbonyl-4-(3-nitrophenyl)-pyridin-5-carbonsäure-N-[(1S)-1-äthoxycarbonyl-1-benzyl]-amid als harziges Rohprodukt.

Zur Trennung der Diastereomeren wird das Rohprodukt mit einem Gemisch von Essigsäureäthylester und Diisopropyläther versetzt und während 1 Stunde bei 0-5° gerührt, wobei ein erstes Diastereomeres kristallisiert. Dieses konfigurativ einheitliche Diastereomere schmilzt bei 193-194° und zeigt eine spezifische Drehung von $[\alpha]_{436}^{20} = -66°$ (c = 0.7, Aethanol).

Zur Isolierung des zweiten Diastereomeren wird die Mutterlauge unter vermindertem Druck eingedampft und der Rückstand durch Chromatographie an der etwa 100-fachen Menge Silikagel (Elution mit einem 1:1 Gemisch von Hexan und Essigsäureäthylester) gereinigt. Das Eluat wird unter vermindertem Druck eingedampft und der Rückstand aus einem Gemisch von Essigsäureäthylester und Hexan umkristallisiert. Das so erhältliche zweite Diastereomere schmilzt bei 139-140° und zeigt eine spezifische Drehung von $[\alpha]_D^{20} = +46°$ (c = 0.8, Aethanol); diese kristalline Form ist laut $^1$H-NMR-Spektrum noch mit ca. 20% des ersten Diastereomeren verunreinigt.

Beispiel 20: Analog dem im Beispiel 1 beschriebenen Verfahren erhält man aus einem Gemisch von 20 g 1,4-Dihydro-2,6-dimethyl-4-(3-nitrophenyl)-pyridin-3,5-dicarbonsäure-3-(1-benzotriazolyl)-ester-5-methylester, 8.9 g D-α-Phenylglycin-äthylester-hydrochlorid und 5.2 ml N-Aethylmorpholin in 150 ml wasserfreiem Dimethylformamid ein 1:1 Diastereomerengemisch von (4R)- und (4S)-1,4-Dihydro-2,6-dimethyl-3-methoxycarbonyl-4-(3-nitrophenyl)-pyridin-5-carbonsäure-N-[(1R)-1-äthoxycarbonyl-1-benzyl]-amid als hochviskoses Rohprodukt.

Zur Trennung der Diastereomeren wird das Rohprodukt mit einem Gemisch von Essigsäureäthylester und Diisopropyläther versetzt und während 16 Stunden bei 0-5° stehen gelassen, wobei ein erstes Diastereomeres kristallisiert. Die Kristalle werden abgenutscht und aus Aethylalkohol umkristallisiert. Das so erhältliche erste Diastereomere ist konfigurativ einheitlich; es schmilzt bei 196-197° und zeigt eine spezifische Drehung von $[\alpha]_{436}^{20}$ = +61° (c = 0.34, Aethanol).

Zur Isolierung des zweiten Diastereomeren wird die Mutterlauge unter vermindertem Druck eingedampft und der Rückstand durch Chromatographie an der etwa 100-fachen Menge Silikagel (Elution mit einem 1:1 Gemisch von Hexan und Essigsäureäthylester) gereinigt. Das Eluat wird unter vermindertem Druck eingedampft und der Rückstand zweimal aus einem Gemisch von Essigsäureäthylester und Diäthyläther umkristallisiert.

Das so erhältliche zweite Diastereomere schmilzt bei 134-135° und zeigt eine spezifische Drehung von $[\alpha]_D^{20}$ = -45° (c = 0.7, Aethanol); diese kristalline Form ist laut $^1$H-NMR Spektrum mit ca. 15% des ersten Diastereomeren verunreinigt.

<u>Beispiel 21:</u> Ein Gemisch von 5.9 g 1,4-Dihydro-2,6-dimethyl-4-(3-nitrophenyl)-pyridin-3,5-dicarbonsäure-3-(1-benzotriazolyl)-ester-5-isopropylester, 5.34 g L-Phenylalanyl-L-phenylalanin-O-(2-trimethylsilyl-äthyl)-ester-hydrochlorid und 1.5 ml N-Aethylmorpholin in 90 ml wasserfreiem Dimethylformamid wird während 15 Stunden bei 80° in einer Stickstoffatmosphäre gerührt. Das Reaktionsgemisch versetzt man unter Eiskühlung mit 200 ml Wasser. Die Kristalle werden abfiltriert, mit Wasser gewaschen und unter vermindertem Druck getrocknet. Dieses Rohprodukt reinigt man durch Chromatographie an der etwa 100-fachen Menge Silikagel (Elution mit einem 1:1 Gemisch von Chloroform und Methanol). Das so erhältliche amorphe Produkt ist ein 1:1 Diastereomerengemisch von N-[1,4-Dihydro-2,6-dimethyl-3-isopropoxycarbonyl-4-(3-nitrophenyl)-5-pyridoyl]-L-phenylalanyl-

L-phenylalanin-O-(2-trimethylsilyl-äthyl)-ester; es zeigt einen dünnschichtchromatographischen Rf-Wert von 0.65 im Elutions-System Toluol-Aceton 1:1.

Zur Spaltung des 2-Trimethylsilyl-äthyl-esters wird ein Gemisch von 3.6 g des oben erhaltenen Esters in 34 ml einer 0.7 M Lösung von Tetraäthylammoniumfluorid in Dimethylsulfoxid während 20 Minuten bei 20° gerührt. Man kühlt die klare Reaktionslösung im Eisbad und gibt 24 ml 1N Salzsäure und 150 ml Wasser zu. Der abgeschiedene Niederschlag wird abfiltriert, der Filterrückstand mit Wasser gewaschen und über Kaliumhydroxid unter vermindertem Druck getrocknet. Das Rohprodukt wird in Essigsäureäthylester aufgenommen, filtriert und das Filtrat unter vermindertem Druck eingedampft. Durch Verreiben des viskosen Rückstandes mit Diisopropyläther erhält man amorphes N-[1,4-Dihydro-2,6-dimethyl-3-isopropoxycarbonyl-4-(3-nitrophenyl)-5-pyridoyl]-L-phenylalanyl-L-phenylalanin als Diastereomerengemisch; es zeigt einen dünnschichtchromatographischen Rf-Wert von 0.70 im Elutions-System Acetonitril-Wasser 3:1.

Das Ausgangsmaterial kann wie folgt erhalten werden:
Ein Gemisch von 8.2 g N-Benzyloxycarbonyl-L-phenylalanin und 8.3 g L-Phenylalanin-(2-trimethylsilyl-äthyl)-ester-hydrochlorid in 125 ml wasserfreiem Dichlormethan wird bei 0° mit 3.50 ml N-Aethylmorpholin und 6.7 g N,N-Dicyclohexylcarbodiimid versetzt. Es wird 1 Stunde bei 0° und anschliessend noch 15 Stunden bei Raumtemperatur gerührt. Das Reaktionsgemisch wird filtriert und das Filtrat mit 80 ml Dichlormethan verdünnt. Man wäscht die Dichlormethanlösung mit 1 N Citronensäure, 1 N Natriumhydrogencarbonat und Wasser. Die organische Phase wird über Natriumsulfat getrocknet, filtriert und das Filtrat unter vermindertem Druck eingedampft. Der Rückstand kristallisiert aus einem Gemisch von Essigsäureäthylester und Petroläther. Der so erhältliche N-Benzyloxycarbonyl-L-phenylalanyl-L-phenylalanin-O-(2-trimethylsilyl-äthyl)-ester schmilzt bei 89-90°.

1.80 g dieses Zwischenproduktes werden in 20 ml Methanol in Gegenwart von 180 mg 10%-iger Palladium-Kohle der Hydrogenolyse unterworfen, wobei der pH-Wert des Reaktionsgemisches mit 1 N Salzsäure konstant bei 4.0 gehalten wird. Nach beendeter Reaktion wird vom Katalysator abfiltriert und das Filtrat unter vermindertem Druck eingedampft, wobei L-Phenylalanyl-L-phenylalanin-O-(2-trimethylsilyl-äthyl)-ester-hydrochlorid als weisser Schaum erhalten wird. Das Produkt zeigt einen dünnschichtchromatographischen Rf-Wert von 0.75 im Elutions-System Chloroform-Methanol 8:2.

Beispiel 22: 10 g (-)-(4R)-1,4-Dihydro-2,6-dimethyl-3-methoxycarbonyl-4-(3-nitrophenyl)-pyridin-5-carbonsäure-N-[(1S)-1-äthoxycarbonyl-2-methyl-1-propyl]-amid werden in einem Gemisch von 300 ml Methanol und 300 ml 0,1 N Natronlauge 15 Stunden bei Raumtemperatur gerührt. Die klare, gelbe Lösung wird anschliessend unter vermindertem Druck zur Trockene eingedampft. Der feste, gelbliche Rückstand wird in 160 ml 1N Natronlauge bei Raumtemperatur gerührt, mit Aktivkohle behandelt, filtriert und das Filtrat mit 200 ml 1N Salzsäure angesäuert. Die ausgefallene Säure wird abfiltriert, mehrmals mit Wasser gewaschen und 12 Stunden bei 50°C im Hochvakuum getrocknet. Das (-)-(4R)-1,4-Dihydro-2,6-dimethyl-3-methoxycarbonyl-4-(3-nitrophenyl)-pyridin-5-carbonsäure-N-[(1S)-1-carboxy-2-methyl-1-propyl]-amid schmilzt bei 217-219°; $[\alpha]_D^{20}$ = -8.1° (c = 0.87, Aethanol).

Beispiel 23: In völlig analoger Weise zu Beispiel 22, jedoch ausgehend von (+)-(4S)-1,4-Dihydro-2,6-dimethyl-3-methoxycarbonyl-4-(3-nitrophenyl)-pyridin-5-carbonsäure-N-[(1S)-1-äthoxycarbonyl-2-methyl-1-propyl]-amid, erhält man (+)-(4S)-1,4-Dihydro-2,6-dimethyl-3-methoxycarbonyl-4-(3-nitrophenyl)-pyridin-5-carbonsäure-N-[(1S)-1-carboxy-2-methyl-1-propyl]-amid, das bei 198-202° unter Zersetzung schmilzt und dessen spezifische Drehung $[\alpha]_D^{20}$ = + 84.1° (c = 1.0, Aethanol) beträgt.

Beispiel 24: 47 g (-)-(4R)-1,4-Dihydro-2,6-dimethyl-3-methoxycarbonyl-4-(3-nitrophenyl)-pyridin-5-carbonsäure-N-[(1S)-1-carboxy-2-methyl-1-propyl]-amid werden mit 30.5 g O-n-Butyl-N,N'-dicyclohexyl-isoharnstoff [Herstellung nach Chem. Ber. 99, 1479 (1966)] in 400 ml Essigsäureäthylester 15 Stunden unter Stickstoff auf 80° erhitzt. Die anfängliche Suspension wird klar und nach einer Stunde beginnt N,N'-Dicyclohexyl-harnstoff auszufallen. Der Harnstoff wird abfiltriert, mit Essigsäureäthylester gewaschen und das Filtrat eingedampft. Der Rückstand wird an einer 1050 g Flashsäule in einem Gemisch von Essigsäureäthylester/Hexan 3:7 als Laufmittel chromatographiert. Das so gereinigte Produkt (51 g) wird aus 60 ml abs. Aether umkristallisiert und mit einem Gemisch von Aether/Hexan 95 : 5 gewaschen. Das so erhaltene (-)-(4R)-1,4-Dihydro-2,6-dimethyl-3-methoxycarbonyl-4-(3-nitrophenyl)-pyridin-5-carbonsäure-N-[(1S)-1-(n-butoxycarbonyl)-2-methyl-1-propyl]-amid hat einen Smp. von 69-70°; $[\alpha]_D^{20}$ = -22° (c = 0.6, Aethanol).

Beispiel 25: In völlig analoger Weise zu Beispiel 24, jedoch ausgehend von (+)-(4S)-1,4-Dihydro-2,6-dimethyl-3-methoxycarbonyl-4-(3-nitrophenyl)-pyridin-5-carbonsäure-N-[(1S)-1-carboxy-2-methyl-1-propyl]-amid, erhält man (+)-(4S)-1,4-Dihydro-2,6-dimethyl-3-methoxycarbonyl-4-(3-nitrophenyl)-pyridin-5-carbonsäure-N-[(1S)-1-(n-butoxycarbonyl)-2-methyl-1-propyl]-amid vom Smp. 144-145°; $[\alpha]_D^{20}$ = + 66° (c = 1.0, Aethanol).

Beispiel 26: In völlig analoger Weise zu Beispiel 24, jedoch ausgehend von den entsprechenden O-substituierten N,N'-Dicyclohexyl-isoharnstoffen [hergestellt nach Chem. Ber. 99, 1479 (1966) sowie Liebigs Ann. Chemie 597, 235 (1956)], erhält man die nachfolgend ausgeführten Verbindungen in konfigurativ einheitlicher Form:

a) (-)-(4R)-1,4-Dihydro-2,6-dimethyl-3-methoxycarbonyl-4-(3-nitrophenyl)-pyridin-5-carbonsäure-N-[(1S)-1-(n-hexyloxycarbonyl)-2-methyl-1-propyl]-amid; $[\alpha]_D^{20} = -15.7 \pm 3.2°$ (c = 0.31, Aethanol).

b) (-)-(4R)-1,4-Dihydro-2,6-dimethyl-3-methoxycarbonyl-4-(3-nitrophenyl)-pyridin-5-carbonsäure-N-[(1S)-1-(benzyloxycarbonyl)-2-methyl-1-propyl]-amid; $[\alpha]_D^{20} = -15.5°$ (c = 0.9, Aethanol).

c) (-)-(4R)-1,4-Dihydro-2,6-dimethyl-3-methoxycarbonyl-4-(3-nitrophenyl)-pyridin-5-carbonsäure-N-{(1S)-1-[3-(4-methyl-1-piperazinyl)-1-propoxy]-carbonyl-2-methyl-1-propyl}-amid; $[\alpha]_D^{20} = -10.0°$ (c = 0.93, Aethanol).

d) (-)-(4R)-1,4-Dihydro-2,6-dimethyl-3-methoxycarbonyl-4-(3-nitrophenyl)-pyridin-5-carbonsäure-N-{(1S)—1-[3-(4-benzyl-1-piperazinyl)-1-propoxy]-carbonyl-2-methyl-1-propyl}-amid; $[\alpha]_D^{20} = -12.7°$ (c = 0.6, Aethanol).

Beispiel 27: In völlig analoger Weise zu Beispiel 24, jedoch ausgehend von einem 1:1-Diastereomerengemisch von (4R)- und (4S)-1,4-Dihydro-2,6-dimethyl-3-methoxycarbonyl-4-(3-nitrophenyl)-pyridin-5-carbonsäure-N-[(1S)-1-carboxy-2-methyl-1-propyl]-amid sowie den entsprechend O-substituierten N,N'-Dicyclohexyl-isoharnstoffen erhält man nachfolgend aufgeführte Verbindungen als amorphe 1:1-Diasteromerengemische:

a) (4R)- und (4S)-1,4-Dihydro-2,6-dimethyl-3-methoxycarbonyl-4-(3-nitrophenyl)-pyridin-5-carbonsäure-N-[(1S)-1-(2-dimethylamino-1-aethoxy)-carbonyl-2-methyl-1-propyl]-amid.
250 MHz FT-$^1$H-NMR ($CDCl_3$): 0.75 (m, 6H, $-CH_3$); 2.1 (m, 1H, -CH); 2.3 (m, 12H, Dihydropyridyl $-CH_3$; $-N-CH_3$); 2.55, 4.20 (2m, 4H, $-O-CH_2-CH_2-N$); 3.65 (2s, 3H, $COOCH_3$); 4.55 (m, 1H, -CON-CH); 5.0 (2s, 1H, 4-Dihydropyridyl-H); 5.65 (2s, 1H, 1-Dihydropyridyl-H); 5.85 (2d, 1H, -CONH); 7.4-8.2 (m, 4H, Phenyl-H).

b) (4R)- und (4S)-1,4-Dihydro-2,6-dimethyl-3-methoxycarbonyl-4-(3-nitrophenyl)-pyridin-5-carbonsäure-N-[(1S)-1-(3-dimethylamino-1-propoxy)-carbonyl-2-methyl-1-propyl]-amid.
250 MHz FT-$^1$H-NMR ($CDCl_3$): 0.75 (m, 6H, $-CH_3$); 1.8 (m, 2H, $-CH_2-$); 2.1 (m, 1H, -CH-); 2.2-2.4 (m, 15H, Dihydropyridyl-$CH_3$; $-CH_2-N-CH_3$); 3.65 (2s, 6H, $COOCH_3$); 4.15 (m, 2H, $COOCH_2$ ); 4.5 (m, 1H, -CON-CH); 5.0 (2s, 1H, 4-Dihydropyridyl-H); 5.6 (2s, 1H, 1-Dihydropyridyl-H); 5.8 (2d, 1H, -CONH); 7.4-8.2 (m, 4H, Phenyl-H).

c) (4R)- und (4S)-1,4-Dihydro-2,6-dimethyl-3-methoxycarbonyl-4-(3-nitrophenyl)-pyridin-5-carbonsäure-N-{(1S)-1-[2-(4-morpholino)-1-äthoxy]-carbonyl-2-methyl-1-propyl}-amid.
250 MHz FT-$^1$H-NMR ($CDCl_3$): 0.75 (m, 6H, $-CH_3$); 2.1 (m, 1H, -CH-); 2.3 (m, 6H, Dihydropyridyl-$CH_3$); 2.4-2.65 (m, 6H, $-N-CH_2$); 3.7 (m, 7H, $-COOCH_3$; $-O-CH_2-$); 4.05-4.4 (m, 2H, $COOCH_2$); 4.55 (m, 1H, CON-CH-); 5.0 (2s, 1H, 4-Dihydropyridyl-H), 5.62 (2s, 1H, 1-Dihydropyridyl-H); 5.8 (2d, 1H, -CONH); 7.4-8.2 (m, 4H, Phenyl-H).

d) (4R)- und (4S)-1,4-Dihydro-2,6-dimethyl-3-methoxycarbonyl-4-(3-nitrophenyl)-pyridin-5-carbonsäure-N-[(1S)-1-(2-methoxy-1-äthoxy)-carbonyl-2-methyl-1-propyl]-amid.
250 MHz FT-$^1$H-NMR ($CDCl_3$): 0.75 (m, 6H, $-CH_3$); 2.1 (m, 1H, -CH-); 2.2-2.36 (4s, 6H, Dihydropyridyl-$CH_3$); 3.36 (2s, 3H, $-OCH_3$); 3.55 (m, 2H, $-OCH_2-$); 3.62 (2s, 3H, $-COOCH_3$); 4.25 (m, 2H, $-COOCH_2-$); 4.60 (m, 1H, -CON-CH-); 4.98 (2s, 1H, 4-Dihydropyridyl-H); 5.6 (2s, 1H, 1-Dihydropyridyl-H); 5.8 (2d, 1H, -CONH-); 7.4-8.2 (m, 4H, Phenyl-H).

Beispiel 28: Analog dem im Beispiel 1 beschriebenen Verfahren erhält man aus einem Gemisch von 54.5 g 1,4-Dihydro-2,6-dimethyl-4-(3-nitrophenyl)-pyridin-3,5-dicarbonsäure-3-(1-benzotriazolyl)-ester-5-methylester, 31 g D,L-Phenylglycin-carbamoylmethylester-hydrochlorid [hergestellt analog Tetrahedron Lett. 24, 5219 (1983)] und 16.2 ml N-Aethylmorpholin in 300 ml wasserfreiem

Dimethylformamid ein racemisches 1:1-Diastereomerengemisch von 1,4-Dihydro-2,6-dimethyl-3-methoxycarbonyl-4-(3-nitrophenyl)-pyridin-5-carbonsäure-N-[1-(carbamoyl-methoxycarbonyl)-1-phenyl-methyl]-amid als harziges Zwischenprodukt.

16 g des obigen Zwischenproduktes werden in einem Gemisch von 67 ml 0.5 N Natronlauge und 50 ml N,N-Dimethylformamid während 2 Stunden bei Raumtemperatur gerührt. Anschliessend dampft man die dunkle Reaktionslösung unter vermindertem Druck zur Trockene ein. Der dunkle Rückstand wird in 100 ml 0.02 N Natronlauge gelöst, mit Essigsäureäthylester mehrmals gewaschen, zusätzlich mit Aktivkohle behandelt, filtriert und das Filtrat mit 2 N Salzsäure angesäuert. Die ausgefallene Säure wird abfiltriert, der Filterrückstand mehrmals mit Wasser gewaschen und 12 Stunden bei 50° im Hochvakuum getrocknet. Das so erhältliche racemische 1:1-Diastereomerengemisch von 1,4-Dihydro-2,6-dimethyl-3-methoxycarbonyl-4-(3-nitrophenyl)-pyridin-5-carbonsäure-N-(1-carboxy-1-phenyl-methyl)-amid schmilzt bei 126-128°.

Beispiel 29:

a) 4 g 1,4-Dihydro-2,6-dimethyl-3-methoxycarbonyl-4-(3-nitrophenyl)-pyridin-5-carbonsäure-N-(1-carboxy-1-phenyl-methyl)-amid (racemisches 1:1-Diastereomerengemisch) werden mit 2.6 g O-(2-Dimethylamino-aethyl)-N,N'-dicyclohexyl-isoharnstoff [Herstellung analog Liebigs Ann. Chem. 597, 235 (1956)] in 60 ml Essigsäureäthylester während 15 Stunden unter einer Stickstoffatmosphäre auf 80° erhitzt. Vom ausgefallenen Harnstoff wird abfiltriert und das Filtrat unter vermindertem Druck eingedampft. Der Rückstand wird durch Chromatographie an der etwa 100-fachen Menge Kieselgel (Elution mit einem 9:1 Gemisch von Methylenchlorid und Methanol) gereinigt. Man gibt zu der so erhaltenen rohen Base 1 Aequivalent 1.7 N alkoholische Salzsäure und dampft die Lösung unter vermindertem Druck ein. Der harzige Rückstand wird in einem Gemisch von

20 ml Essigsäureäthylester und 50 ml Diäthyläther im Eisbad gerührt, wobei das 1,4-Dihydro-2,6-dimethyl-3-methoxycarbonyl-4-(3-nitrophenyl)-pyridin-5-carbonsäure-N-[1-(2-dimethylamino-1-äthoxy)-carbonyl-1-phenyl-methyl]-amid-Hydrochlorid kristallisiert. Dieses 1:1-Diastereomerengemisch schmilzt bei 100-112° unter Zersetzung.

b) In völlig analoger Weise zu a), jedoch ausgehend von O-(2-Morpholino-äthyl)-N,N'-dicyclohexyl-isoharnstoff [Herstellung analog Liebigs Ann. Chem. 597, 235 (1956)], erhält man 1,4-Dihydro-2,6-dimethyl-3-methoxycarbonyl-4-(3-nitrophenyl)-pyridin-5-carbonsäure-N-{1-[2-(4-morpholino)-1-äthoxy]-carbonyl-1-phenyl-methyl}-amid-Hydrochlorid als 1:1-Diastereomerengemisch, das bei 127-129° schmilzt.

Beispiel 30:

a) Analog dem im Beispiel 1 beschriebenen Verfahren erhält man aus einem Gemisch von 50 g 1,4-Dihydro-2,6-dimethyl-4-(3-nitro-phenyl)-pyridin-3,5-dicarbonsäure-3-(1-benzotriazolyl)-ester-5-methylester, 20.4 g L-Isoleucin-äthylester-hydrochlorid und 12.2 ml N-Aethylmorpholin in 300 ml wasserfreiem Dimethylformamid (+)-(4S)-1,4-Dihydro-2,6-dimethyl-3-methoxycarbonyl-4-(3-nitrophenyl)-pyridin-5-carbonsäure-N-[(1S,2S)-1-äthoxy-carbonyl-2-methyl-1-butyl]-amid, das nach Umkristallisation aus einem Gemisch von Essigsäuremethylester und Diisopropyläther bei 170-171° schmilzt. Dieses Diastereomere α ist konfigurativ einheitlich und zeigt eine spezifische Drehung von $[\alpha]_D^{20}$ = + 93° (c = 0.44, Aethanol).

Aus der Mutterlauge wird, analog dem im Beispiel 1 beschriebenen Verfahren, (4R)-1,4-Dihydro-2,6-dimethyl-3-methoxycarbonyl-4-(3-nitrophenyl)-pyridin-5-carbonsäure-N-[(1S,2S)-1-äthoxy-carbonyl-2-methyl-1-butyl]-amid isoliert. Durch Verreiben des amorphen Rohproduktes mit Diäthyläther kristallisiert

dieses Diastereomer β, das nach weiterer Umrkistallisation aus einem Gemisch von Essigsäureäthylester und n-Hexan bei 117-118° schmilzt. Die spezifische Drehung beträgt $[\alpha]_D^{20}$ = +5° (c = 0.91, Aethanol); dieses β-Diastereomer enthält laut $^1$H-NMR Spektrum noch ca. 25 % des obigen α-Diastereomeren.

b) In völlig analoger Weise zu a), jedoch ausgehend von L-Isoleucin-methylester-hydrochlorid, erhält man ein 1:1-Diastereomerengemisch von (4R)- und (4S)-1,4-Dihydro-2,6-dimethyl-3-methoxycarbonyl-4-(3-nitrophenyl)-pyridin-5-carbonsäure-N-[(1S,2S)-1-methoxycarbonyl-2-methyl-1-butyl]-amid vom Smp. 139-140°.

Beispiel 31: Analog dem im Beispiel 2 beschriebenen Verfahren erhält man aus einem Gemisch von 9.4 g N-Acetoacetyl-α-amino-cyclohexylcarbonsäure-äthylester [hergestellt analog Pharm. Acta Helv. 38, 616 (1963)], 4.1 g 3-Aminocrotonsäure-methylester und 5.5 g 3-Nitrobenzaldehyd in 70 ml Aethanol amorphes 1,4-Dihydro-2,6-dimethyl-3-methoxycarbonyl-4-(3-nitrophenyl)-pyridin-5-carbonsäure-N-(1-äthoxycarbonyl-1-cyclohexyl)-amid, das beim Verrühren mit Diäthyläther kristallisiert, Smp. 166-168°.

Beispiel 32: Analog dem im Beispiel 2 beschriebenen Verfahren erhält man aus einem Gemisch von 8.1 g N-Acetoacetyl-α,α-diphenylglycin-äthylester [hergestellt analog Pharm. Acta Helv. 38, 616 (1963)], 2.8 g 3-Aminocrotonsäure-methylester und 3.6 g 3-Nitrobenzaldehyd in 45 ml Aethanol amorphes 1,4-Dihydro-2,6-dimethyl-3-methoxycarbonyl-4-(3-nitrophenyl)-pyridin-5-carbonsäure-N-(1-äthoxycarbonyl-1,1-diphenyl-methyl)-amid.

250 MHz FT-$^1$H-NMR ($CDCl_3$): 1.15 (t, 3H, $-COO-C-CH_3$); 2.18, 2.30 (2s, 6H, Dihydropyridyl-H); 3.61 (s, 3H, $-COOCH_3$); 4.19 (q, 2H, $-COO-CH_2-$); 5.05 (s, 1H, 4-Dihydropyridyl-H);

5.72 (s, 1H, 1-Dihydropyridyl-H); 6.96 (s,1H, -CONH-); 7.1-8.18 (m, 14H, Phenyl-H).

Beispiel 33: Analog dem im Beispiel 1 beschriebenen Verfahren erhält man aus 7.9 g 1,4-Dihydro-2,6-dimethyl-4-(3-nitrophenyl)-pyridin-3,5-dicarbonsäure-3-(1-benzotriazolyl)-ester-5-methylester und 5 g D,L-α-[2-(N-tert.-butoxycarbonyl-amino)-4-thiazolyl]-glycinäthylester in 30 ml wasserfreiem Dimethylformamid racemisches 1,4-Dihydro-2,6-dimethyl-3-methoxycarbonyl-4-(3-nitrophenyl)-pyridin-5-carbonsäure-N-{1-[2-(N-tert.-butoxycarbonyl-amino)-4-thiazolyl]-1-äthoxycarbonyl-methyl}-amid als harziges Zwischenprodukt.

Dieses wird in 40 ml mit Salzsäure gesättigtem, eiskaltem Eisessig aufgelöst und während 1 1/2 Stunden bei 0° gerührt. Anschliessend wird die kalte Reaktionslösung mit 2N Natronlauge neutralisiert und das Rohprodukt mehrmals mit Essigsäureäthylester extrahiert. Die vereinigten organischen Extrakte werden unter vermindertem Druck eingedampft, wobei 1,4-Dihydro-2,6-dimethyl-3-methoxycarbonyl-4-(3-nitrophenyl)-pyridin-5-carbonsäure-N-[1-(2-amino-4-thiazolyl)-1-äthoxycarbonyl-methyl]-amid als amorphes 1:1-Diasteromerengemisch zurück bleibt.

250 MHz FT-$^1$H-NMR ($CDCl_3$): 1.2 (2t, 3H, $-COO-C-CH_3$); 2.32 (2d, 6H, Dihydropyridyl-$CH_3$); 2.65 (2s, 3H, $-COOCH_3$); 4.15 (2m, 2H, $-COO-CH_2-$); 5.0 (2s, 1H, 4-Dihydropyridyl-H); 5.06 (s, 1H, $-NH_2$); 5.40 (2s, 1H, -N-CH-); 5.60 (2s, 1H, 1-Dihydropyridyl-H); 6.42 (2s, 1H, Thiazolyl-H); 6.50, 6.65 (2d, 1H, -CONH); 7.35-8.20 (m, 4H, Phenyl-H).

Beispiel 34:

a) Eine Lösung von 7 g 1,4-Dihydro-2,6-dimethyl-3-methylsulfonyl-4-(3-nitrophenyl)-pyridin-5-carbonsäure [hergestellt analog EP 11.706; Smp. 228-229°], 3.4 g 1-Hydroxybenzotriazol und 4.6 g

N,N'-Dicyclohexylcarbodiimid in 100 ml wasserfreiem Dimethylformamid wird während 16 Stunden bei 0-5° unter Stickstoffatmosphäre stehen gelassen. Der auskristallisierte N,N'-Dicyclohexylharnstoff wird abfiltriert. Man versetzt das gelbe Filtrat mit 4 g L-Valin-äthylester-hydrochlorid und 2.8 ml N-Aethylmorpholin und rührt während 16 Stunden bei 80° unter Stickstoffatmosphäre. Zum gelben Reaktionsgemisch gibt man unter Eiswasserkühlung 250 ml Eiswasser und rührt noch während 1 Stunde bei 0-5°, wobei das Rohprodukt kristallisiert. Es wird abfiltriert, der Filterrückstand mit 1 l Wasser gewaschen und im Vakuum getrocknet. Das so erhältliche 1:1-Diastereomerengemisch von (4R)- und (4S)-1,4-Dihydro-2,6-dimethyl-3-methylsulfonyl-4-(3-nitrophenyl)-pyridin-5-carbonsäure-N-[(1S)-1-äthoxycarbonyl-2-methyl-1-propyl]-amid schmilzt nach dem Umkristallisieren aus Essigsäureäthylester bei 191-192°.

b) In völlig analoger Weise zu a), jedoch ausgehend von D-Valinäthylester-hydrochlorid, erhält man ein 1:1-Diastereomerengemisch von (4R)- und (4S)-1,4-Dihydro-2,6-dimethyl-3-methylsulfonyl-4-(3-nitrophenyl)-pyridin-5-carbonsäure-N-[(1R)-1-äthoxycarbonyl-2-methyl-1-propyl]-amid, das bei 188-191° schmilzt.

Beispiel 35: Ein Gemisch von 44.6 g (S)-N-(3-Nitrobenzylidenacetoacetyl)-α-amino-isovaleriansäure-äthylester und 14.1 g 3-Amino-crotonsäure-methylester in 350 ml Aethanol wird während 16 Stunden bei 80° unter Stickstoffatmosphäre gerührt. Das gelbe Reaktionsgemisch wird unter vermindertem Druck eingedampft und der Rückstand durch Chromatographie an der etwa 100-fachen Menge Kieselgel (Elution mit einem 7:3 Gemisch von n-Hexan und Essigsäureäthylester) gereinigt. Das so erhältliche harzige Rohprodukt ist ein 1:1-Diastereomerengemisch von (4R)- und (4S)-1,4-Dihydro-2,6-dimethyl-3-methoxycarbonyl-4-(3-nitrophenyl)-pyridin-5-carbonsäure-N-[(1S)-1-äthoxycarbonyl-2-methyl-1-propyl]-amid.

Die Trennung der Diastereomeren erfolgt wie in Beispiel 1 beschrieben.

Das Ausgangsmaterial kann wie folgt hergestellt werden:

In eine Lösung von 21.6 g 3-Nitrobenzaldehyd und 32.8 g (S)-N-Acetoacetyl-α-amino-isovaleriansäure-äthylester [hergestellt analog Pharm. Acta Helv. 38, 616 (1963)] in 140 ml wasserfreiem Toluol wird während 2 Stunden bei 5-15° HCl-Gas eingeleitet. Anschliessend rührt man noch während 2 Stunden bei Raumtemperatur und dampft das Reaktionsgemisch unter vermindertem Druck ein. Der Rückstand wird während 4 Stunden bei 80° unter Wasserstrahlvakuum gerührt, wobei Salzsäure abgespalten wird. Der so erhältliche rohe (S)-N-(3-Nitrobenzyliden-acetoacetyl)-α-amino-isovaleriansäure-äthylester schmilzt nach dem Umkristallisieren aus einem Gemisch von 24 ml Tetrahydrofuran und 180 ml Diäthyläther bei 114-116°.

Beispiel 36: In völlig analoger Weise zu Beispiel 35, jedoch ausgehend von 2-Nitrobenzaldehyd, erhält man zuerst (S)-N-(2-Nitrobenzyliden-acetoacetyl)-α-amino-isovaleriansäure-äthylester [Smp. 106-107°] als Zwischenprodukt, und daraus durch Kondensation mit 3-Amino-crotonsäure-methylester ein harziges Diastereomerengemisch von (4R)- und (4S)-1,4-Dihydro-2,6-dimethyl-3-methoxycarbonyl-4-(2-nitrophenyl)-pyridin-5-carbonsäure-N-[(1S)-1-äthoxycarbonyl-2-methyl-1-propyl]-amid.

Die Trennung der Diastereomeren erfolgt wie im Beispiel 1 beschrieben, wobei man nach Umkristallisation aus einem Gemisch von Essigsäureäthylester und Hexan ein konfigurativ einheitliches Diastereomeres α erhält, dessen spezifische Drehung $[\alpha]_D^{20} = -363.5 \pm 6.4°$ (c = 0.15, Aethanol) beträgt und das bei 179-180° schmilzt. Aus der Mutterlauge wird das amorphe Diastereomer β isoliert, das laut $^1$H-NMR-Spektrum noch ca. 15% des obigen α-Diastereomeren enthält.

250 MHz FT-$^1$H-NMR ($CDCl_3$)-Spektrum der Diastereomeren β: 0.52, 0.6 (2d, 6H, $-CH_3$); 1.3 (t, 3H, $-O-CH_2-$); 1.98 (m, 1H, -CH-); 2.28, 2.48 (2s, 6H, Dihydropyridyl-$CH_3$); 3.55 (s, 3H, $-COOCH_3$); 4.12 (m, 2H, $-COOCH_2-$); 4.6 (m, 1H, -CON-CH-); 5.7, 5.83 (2s, 2H, 1- bzw. 4-Dihydropyridyl-H); 7.25-7.8 (m, 4H, Phenyl-H).

Beispiel 37: Analog dem im Beispiel 1 beschriebenen Verfahren erhält man aus 18.7 g 1.4-Dihydro-2,6-dimethyl-4-(3-nitrophenyl)-pyridin-3,5-dicarbonsäure-3-(1-benzotriazolyl)-ester-5-methylester und 6.7 g L-Valinamid in 100 ml wasserfreiem Dimethylformamid ein 1:1-Diastereomerengemisch von (4R)- und (4S)-1,4-Dihydro-2,6-dimethyl-3-methoxycarbonyl-4-(3-nitrophenyl)-pyridin-5-carbonsäure-N-[(1S)-1-carbamoyl-2-methyl-1-propyl]-amid, das nach Umkristallisation aus einem Gemisch von Aceton und Diäthyläther bei 190-191° schmilzt.

Beispiel 38:

a) Zu einem Gemisch von 8.6 g 1,4-Dihydro-2,6-dimethyl-3-methoxycarbonyl-4-(3-nitrophenyl)-pyridin-5-carbonsäure-N-(1-carboxy-2-methyl-1-propyl)-amid (racemisches 1:1-Diastereomerengemisch) und 2.5 ml N-Aethylmorpholin in 50 ml wasserfreiem Tetrahydrofuran tropft man innerhalb von 10 Minuten 1.9 ml Chlorameisensäure-isobutylester unter Kühlen so zu, dass die Temperatur -15° nicht übersteigt. Anschliessend rührt man noch während 10 Minuten bei -20° bis -15° und tropft dann unter Kühlen 2.8 ml 2-(2-Aminoäthyl)-pyridin so zu, dass die Temperatur -10° nicht übersteigt. Nach Ende der Zugabe lässt man das Reaktionsgemisch auf Raumtemperatur erwärmen und rührt noch während 3 Stunden, wonach man es unter reduziertem Druck eindampft. Den harzigen Rückstand reinigt man durch

Chromatographie an der etwa 100-fachen Menge Kieselgel (Elution mit einem 95:5 Gemisch von Methylenchlorid und Methanol). Das so erhältliche harzige 1,4-Dihydro-2,6-dimethyl-3-methoxycarbonyl-4-(3-nitrophenyl)-pyridin-5-carbonsäure-N-{1-[2-(2-pyridyl)-1-äthylamino]-carbonyl-2-methyl-1-propyl}-amid kristallisiert aus einem Gemisch von Methylenchlorid und Diäthylaether als 1:4-Diastereomerengemisch vom Smp. 158-160°.

b) In völlig analoger Weise zu a), jedoch ausgehend von N-Methyl-benzylamin, erhält man ein 1:4-Diastereomerengemisch von 1,4-Dihydro-2,6-dimethyl-3-methoxycarbonyl-4-(3-nitrophenyl)-pyridin-5-carbonsäure-N-[1-(N-benzylmethylamino)-carbonyl-2-methyl-1-propyl]-amid vom Smp. 94-95°.

c) In völlig analoger Weise zu a), jedoch ausgehend von N-Benzyl-piperazin, erhält man ein amorphes 3:7-Diastereomerengemisch von 1,4-Dihydro-2,6-dimethyl-3-methoxycarbonyl-4-(3-nitrophenyl)-pyridin-5-carbonsäure-N-[1-(4-benzyl-1-piperazinyl)-carbonyl-2-methyl-1-propyl]-amid.

250 MHz FT-$^1$H-NMR($CDCl_3$): 0.6-0.85 (m, 6H, $-CH_3$); 1.85 (m, 1H, -CH-), 2.2-2.35 (4s, 6H, Dihydropyridyl-$CH_3$); 2.4 (m, 4H, $-N-CH_2$); 3.44-3.66 (m, 9H, $N-CH_2$, $-COOCH_3$); 4.85 (m, 1H, -CON-CH-); 5.0 (2s, 1H, 4-Dihydropyridyl-H); 5.55, 5.6 (2s, 1H, 1-Dihydropyridyl-H): 6.15, 6.30 (2d, 1H, -CONH); 7.25-8.18 (m, 9H, Phenyl-H).

d) In völlig analoger Weise zu a), jedoch ausgehend von N-Diphenylmethyl-piperazin, erhält man ein amorphes 3:7 Diastereomerengemisch von 1,4-Dihydro-2,6-dimethyl-3-methoxycarbonyl-4-(3-nitrophenyl)-pyridin-5-carbonsäure-N-[1-(4-diphenylmethyl-1-piperazinyl)-carbonyl-2-methyl-1-propyl]-amid.

250 MHz FT-$^1$H-NMR ($CDCl_3$): 0.55-0.9 (m, 6H, $-CH_3$); 1.8 (m, 1H, -CH-); 2.15-2.45 (4s, 10H, Dihydropyridyl-$CH_3$, $-N-CH_2-$); 3.4-3.6 (m, 4H, $-\overset{\overset{O}{\|}}{C}-N-CH_2$; 3.55, 3.65 (2s, 3H, $-COOCH_3$); 4.2 (2s, 1H, $-CHPh_2$; 4.8 (m, 1H, -CON-CH); 4.98 (2s, 1H, 4-Dihydropyridyl-H); 5.45, 5.55 (2S, 1H, 1-Dihydropyridyl-H); 6.1, 6.28 (2d, 1H, -CONH-); 7.15-8.15 (m, 14H, Phenyl-H).

e) In völlig analoger Weise zu a), jedoch ausgehend von n-Butylamin, erhält man ein amorphes 1:2-Diastereomerengemisch von 1,4-Dihydro-2,6-dimethyl-3-methoxycarbonyl-4-(3-nitrophenyl)-pyridin-5-carbonsäure-N-[1-(n-butylaminocarbonyl)-2-methyl-1-propyl]-amid.

250 MHz FT-$^1$H-NMR ($CDCl_3$): 0.65-0.98 (m, 9H, $-CH_3$); 1.25-1.55 (m, 4H, $-CH_2-CH_2-$); 1.95 (m, 1H, -CH-); 2.18-2.38 (4s, 6H, Dihydropyridyl-$CH_3$); 3.22 (m, 2H, $-CON-CH_2-$); 3.65 (2s, 3H, $-COOCH_3$); 4.15 (m, 1H, -CON-CH-); 4.98 (2s, 1H, 4-Dihydropyridyl-H); 5.55, 5.65 (2s, 1H, 1-Dihydropyridyl-H); 5.8, 6.05 (2m, 2H, -CONH); 7.4-8.15 (m, 4H, Phenyl-H).

f) In völlig analoger Weise zu a), jedoch ausgehend von N-n-Heptyl-methylamin, erhält man ein amorphes 1:1-Diastereomerengemisch von 1,4-Dihydro-2,6-dimethyl-3-methoxycarbonyl-4-(3-nitrophenyl)-pyridin-5-carbonsäure-N-[1-(N-methyl-n-heptylaminocarbonyl)-2-methyl-1-propyl]-amid.

250 MHz FT-$^1$H-NMR ($CDCl_3$): 0.63-0.98 (m, 9H, $-CH_3$); 1.2-1.6 (m, 10H, $-CH_2-$); 1.9 (m, 1H, -CH-); 2.15, 2.35 (2d, 6H, Dihydropyridyl-$CH_3$); 2.89, 3.02 (2d, 3H, $N-CH_3$); 3.1-3.55 (m, 2H, $N-CH_2$); 3.6, 3.65 (2s, 3H, $COOCH_3$); 4.8 (m, 1H, -CON-CH-); 5.0 (2s, 1H, 4-Dihydropyridyl-H); 5.52, 5.62 (2s, 1H, 1-Dihydropyridyl-H); 6.08-6.30 (m, 1H, -CONH); 7.38-8.18 (m, 4H, Phenyl-H).

<u>Beispiel 39:</u> In völlig analoger Weise zu Beispiel 22, jedoch ausgehend von (+)-(4S)-1,4-Dihydro-2,6-dimethyl-3-methoxycarbonyl-4-(3-nitrophenyl)-pyridin-5-carbonsäure-N-[(1R)-1-äthoxycarbonyl-2-methyl-1-propyl]-amid, erhält man (+)-(4S)-1,4-Dihydro-2,6-dimethyl-3-methoxycarbonyl-4-(3-nitrophenyl)-pyridin-5-carbonsäure-N-[(1R)-1-carboxy-2-methyl-1-propyl]-amid, das bei 216-218° schmilzt und dessen spezifische Drehung $[\alpha]_D^{20}$ = + 8.0°(c = 1.0, Aethanol) beträgt.

<u>Beispiel 40:</u> In völlig analoger Weise zu Beispiel 22, jedoch ausgehend von (-)-(4R)-1,4-Dihydro-2,6-dimethyl-3-methoxycarbonyl-4-(3-nitrophenyl)-pyridin-5-carbonsäure-N-[(1R)-1-äthoxycarbonyl-2-methyl-1-propyl]-amid, erhält man (-)-(4R)-1,4-Dihydro-2,6-dimethyl-3-methoxycarbonyl-4-(3-nitrophenyl)-pyridin-5-carbonsäure-N-[(1R)-1-carboxy-2-methyl-1-propyl]-amid, das bei 121-122° unter Zersetzung schmilzt und dessen spezifische Drehung $[\alpha]_D^{20}$ = -77.0° (c = 1.0, Aethanol) beträgt.

<u>Beispiel 41:</u> In völlig analoger Weise zu Beispiel 24, jedoch ausgehend von (+)-(4S)-1,4-Dihydro-2,6-dimethyl-3-methoxycarbonyl-4-(3-nitrophenyl)-pyridin-5-carbonsäure-N-[(1R)-1-carboxy-2-methyl-1-propyl]-amid, erhält man (+)-(4S)-1,4-Dihydro-2,6-dimethyl-3-methoxycarbonyl-4-(3-nitrophenyl)-pyridin-5-carbonsäure-N-[(1R)-1-(n-butoxycarbonyl)-2-methyl-1-propyl]-amid, das bei 67-68° schmilzt und eine spezifische Drehung von $[\alpha]_D^{20}$ = -21° (c = 1.0, Aethanol) besitzt.

<u>Beispiel 42:</u> In völlig analoger Weise zu Beispiel 24, jedoch ausgehend von (-)-(4R)-1,4-Dihydro-2,6-dimethyl-3-methoxycarbonyl-4-(3-nitrophenyl)-pyridin-5-carbonsäure-N-[(1R)-1-carboxy-2-methyl-1-propyl]-amid, erhält man (-)-(4R)-1,4-Dihydro-2,6-dimethyl-3-methoxycarbonyl-4-(3-nitrophenyl)-pyridin-5-carbonsäure-N-[(1R)-1-(n-butoxycarbonyl)-2-methyl-1-propyl]-amid, das bei 143-144° schmilzt und dessen spezifische Drehung $[\alpha]_D^{20}$ = -65° (c = 1.0, Aethanol) beträgt.

Beispiel 43: In völlig analoger Weise zu Beispiel 35, jedoch ausgehend von 3-Aminocrotonsäure-äthylester, erhält man ein ca. 1:1-Diastereomerengemisch als harziges Rohprodukt. Durch Kristallisation aus einem 2:1-Gemisch von Essigsäureäthylester und Diisopropyläther erhält man kristallines (-)-(4R)-1,4-Dihydro-2,6-dimethyl-3-äthoxycarbonyl-4-(3-nitrophenyl)-pyridin-5-carbonsäure-N-[(1S)-1-äthoxycarbonyl-2-methyl-1-propyl]-amid in konfigurativ einheitlicher Form. Es schmilzt bei 169-170°. Seine spezifische Drehung beträgt $[\alpha]_D^{20}$ = -46.6° (c = 0.476, Aethanol). Aus der Mutterlauge wird kristallines (+)-(4S)-1,4-Dihydro-2,6-dimethyl-3-äthoxycarbonyl-4-(3-nitrophenyl)-pyridin-5-carbonsäure-N-[(1S)-1-äthoxycarbonyl-2-methyl-1-propyl]-amid isoliert; Smp. 135-136°; $[\alpha]_D^{20}$ = + 40.6° (c = 0.94, Aethanol).

Beispiel 44: In völlig analoger Weise zu Beispiel 35, jedoch ausgehend von 3-Aminocrotonsäure-(n-propyl)-ester, erhält man ein ca. 1:1-Diastereomerengemisch als harziges Rohprodukt. Durch Kristallisation aus einem 2:1-Gemisch von Essigsäureäthylester und Diisopropyläther erhält man kristallines (-)-(4R)-1,4-Dihydro-2,6-dimethyl-3-(n-propyloxycarbonyl)-4-(3-nitrophenyl)-pyridin-5-carbonsäure-N-[(1S)-1-äthoxycarbonyl-2-methyl-1-propyl]-amid in konfigurativ einheitlicher Form. Es schmilzt bei 148-149°. Seine spezifische Drehung beträgt $[\alpha]_D^{20}$ = -19.9° (c = 0.55, Aethanol). Aus der Mutterlauge wird kristallines (+)-(4S)-1,4-Dihydro-2,6-dimethyl-3-(n-propyloxycarbonyl)-4-(3-nitrophenyl)-pyridin-5-carbonsäure-N-[(1S)-1-äthoxycarbonyl-2-methyl-1-propyl]-amid isoliert; Smp. 119-121°; $[\alpha]_D^{20}$ = + 70.9° (c = 0.67, Aethanol).

Beispiel 45: 2,4 g (S)-N-(3-Nitrobenzyliden-acetoacetyl)-α-amino-isovaleriansäure-butylester und 0,92 g Amidinoessigsäure-methylester-hydrochlorid [s. Ann. Chem. 1977, 1895] werden in 6 ml abs. Methanol gelöst und unter Rückfluss eine aus 140 mg Natrium und 6 ml abs. Methanol hergestellte Natriummethylatlösung zugetropft und noch eine Stunde am Rückfluss erhitzt. Das ausgefallene Natriumchlorid wird abfiltriert, das Filtrat unter vermindertem Druck eingedampft und der Rückstand an

einer Mitteldrucksäule in einem 1:1 Gemisch von Hexan und Essigsäureäthylester chromatographiert. Das so erhaltene, amorphe 2-Amino-1,4-dihydro-6-methyl-3-methoxycarbonyl-4-(3-nitrophenyl)-pyridin-5-carbonsäure-N-[(1S)-1-(n-butoxycarbonyl)-2-methyl-1-propyl]-amid ist ein S,4R : S,4S Diastereomerengemisch und zeigt folgende NMR-Daten:
250 MHz FT-$^1$H-NMR ($CDCl_3$) : 0.80 (2m, 6H, Isopropyl-$CH_3$); 0.95 (2t, 3H, -COO-C-C-C-$CH_3$); 1.34, 1.60 (2m, 4H, -COO-C-$CH_2$-$CH_2$-C); 2.06 (m, 1H, -$CHC_2$); 2.10, 2.18 (2s, 6H, Dihydropyridyl-$CH_3$); 2.62 (2s, 6H, -$COOCH_3$); 4.1 (m, 2H, -$COOCH_2$-C-); 4.46 (m, 1H, -OOC-CH-N-CO-); 4.80, 4.86 (2s, 1H, 4-Dihydropyridyl-H); 5.84, 5.98 (2d, 1H, -NH-); 6.35 (1s, 2H, -$NH_2$); 6.76, 6.90 (2d, 1H, -CO-NH-); 7.40, 7.64, 8.20, 8.14 (m, t, d, m, 4H, Phenyl-H).

Das Ausgangsmaterial kann wie folgt hergestellt werden:

Analog dem im Beispiel 35 beschriebenen Verfahren erhält man aus einem Gemisch von 14,0 g (S)-N-Acetoacetyl-α-amino-isovaleriansäure--butylester, hergestellt analog Pharm. Acta Helv. 38, 616 (1963), und 8,2 g 3-Nitrobenzaldehyd in 50 ml Toluol den (S)-N-(3-Nitrobenzyliden--acetoacetyl)-α-amino-isovaleriansäure-butylester.

Beispiel 46: 26,5 g 3-Aethoxycarbonyl-2-diäthoxymethyl-1,4-dihydro-6-methyl-4-(3-nitrophenyl)-pyridin-5-carbonsäure-N-[(1S)-1-äthoxycarbonyl-2-methyl-1-propyl]-amid werden in 160 ml Aceton gelöst und 26,5 ml 6N Salzsäure zugegeben. Es wird während einer Stunde bei Raumtemperatur gerührt, die Reaktionslösung unter vermindertem Druck eingedampft und der Rückstand an einer Flashsäule in einem 8:2 Gemisch von Hexan und Essigsäureäthylester chromatographiert. Das so erhältliche amorphe 3-Aethoxycarbonyl-1,4-dihydro-2-formyl-6-methyl-4-(3-nitrophenyl)-pyridin-5-carbonsäure-N-[(1S)-1-äthoxycarbonyl-2-methyl-1-propyl]-amid ist ein S,4R : S,4S Diastereomerengemisch und zeigt folgende NMR-Daten:
250 MHz FT-$^1$H-NMR ($CDCl_3$) : 0.80 (2m, 6H, -C$(CH_3)_2$); 1.26 (2m, 6H, -COO-C-$CH_3$); 2.06 (m, 1H, -$CHC_2$); 2.28, 2.36 (2s, 3H, Dihydropyridyl--$CH_3$); 4.18 (m, 4H, -COO-$CH_2$-C); 4.53 (m, 1H, -OOC-CH-N-CO-); 5.00, 5.16 (2s, 1H, 4-Dihydropyridyl-H); 5.72, 5.88 (2d, 1H, -NH-); 6.83, 6.90 (2s, 1H, -CO-NH-); 7.47, 7.70, 8.10, 8.18 (t, m, m, m, 4H,

Phenyl-H).

Das Ausgangsmaterial kann wie folgt synthetisiert werden:

Ein Gemisch von 29,0 g (S)-N-(3-Nitrobenzyliden-acetoacetyl)-α-amino--isovaleriansäure-äthylester (s. Beispiel 35), 17,4 g 3-Amino-4,4-diäthoxy-crotonsäure-äthylester, 50 g Molekularsieb (Union Carbide 3Å) und 160 ml abs. Alkohol wird während 40 Stunden unter Rückfluss erhitzt. Das Molekularsieb wird abfiltriert, das Filtrat unter vermindertem Druck eingedampft und der Rückstand an einer Flashsäule in einem 1:1 Gemisch von Hexan und Essigsäureäthylester chromatographiert. Das so erhaltene 3-Aethoxycarbonyl-2-diäthoxymethyl-1,4-dihydro-6-methyl-4-(3-nitrophenyl)-pyridin-5-carbonsäure-N-[(1S)-1-äthoxycarbonyl-2-methyl-1-propyl]-amid wird sofort weiter umgesetzt.

Beispiel 47: 6,5 g 3-Aethoxycarbonyl-1,4-dihydro-2-formyl-6-methyl-4--(3-nitrophenyl)-pyridin-5-carbonsäure-N-[(1S)-1-äthoxycarbonyl-2-methyl-1-propyl]-amid werden in 100 ml Alkohol gelöst und unter Rühren bei 0° werden innerhalb 5 Minuten 0,65 g Natriumborhydrid zugegeben und 30 Minuten bei Raumtemperatur ausgerührt. Dann wird mit 2N Salzsäure angesäuert und unter vermindertem Druck zur Trockne eingedampft. Der Rückstand wird zwischen Wasser und Essigsäureäthylester verteilt, die organische Phase getrocknet, eingedampft und der Rückstand an einer Mitteldrucksäule in einem 1:1 Gemisch von Hexan und Essigsäureäthylester chromatographiert. Das so erhaltene amorphe 3-Aethoxycarbonyl-1,4-dihydro-2-hydroxymethyl-6-methyl-4-(3-nitrophenyl)-pyridin-5-carbonsäure-N-[(1S)-1-äthoxycarbonyl-2-methyl-1--propyl]-amid ist ein S,4R : S,4S Diastereomerengemisch und zeigt folgende NMR-Daten:

250 MHz FT-$^{1}$H-NMR ($CDCl_3$) : 0.74 (2m, 6H, $-C(CH_3)_2$); 1.28 (2m, 6H, $-COO-C-CH_3$); 2.06 (m, 1H, $-CHC_2$); 2.26, 2.32 (2s, 3H, Dihydropyridyl-$-CH_3$); 2.76, 2.96 (2m, 1H, -OH); 4.1 (m, 4H, $-COO-CH_2-C-$); 4.50 (m, 1H, -OOC-CH-N-CO-); 4.68, 4.84 (2m, 2H, $-CH_2-O$); 4.98, 5.02, (2s, 1H, 4-Dihydropyridyl-H); 5.72, 5.98 (2d, 1H, -NH-); 6.96, 7.12 (2s, 1H, -CO-NH-); 7.46, 7.67, 8.05, 8.16 (t, t, m, m, 4H, Phenyl-H).

Beispiel 48: Ein Gemisch von 12,0 g 3-Aethoxycarbonyl-1,4-dihydro-2-formyl-6-methyl-4-(3-nitrophenyl)-pyridin-5-carbonsäure-N-[(1S)-1--äthoxycarbonyl-2-methyl-1-propyl]-amid, 2,0 g Hydroxylaminhydrochlorid, 1,6 g Natriumcarbonat und 150 ml abs. Alkohol werden während $2\frac{1}{2}$ Stunden bei Raumtemperatur gerührt. Dann wird unter vermindertem Druck eingedampft und der Rückstand zwischen Wasser und Essigsäureäthylester verteilt. Die organische Phase wird getrocknet, eingedampft und das erhaltene Oxim sofort weiter umgesetzt.

Ein Gemisch von 12,5 g 3-Aethoxycarbonyl-1,4-dihydro-2-hydroxyiminomethyl-6-methyl-4-(3-nitrophenyl)-pyridin-5-carbonsäure-N-[(1S)-1--äthoxycarbonyl-2-methyl-1-propyl]-amid, 12,9 g N,N-Dicyclohexylcarbodiimid und 50 ml abs. Pyridin werden während 5 Stunden unter Rückfluss erhitzt. Hierauf wird die Lösung unter vermindertem Druck eingedampft, der Rückstand zwischen 1N Salzsäure und Essigsäureäthylester verteilt und vom ausgefallenen Harnstoff abfiltriert. Die organische Phase wird mit Wasser und Sole gewaschen, getrocknet und eingedampft. Der Rückstand wird an einer Mitteldrucksäule in einem Gemisch von Hexan und Essigsäureäthylester chromatographiert. Das so erhaltene amorphe 3-Aethoxycarbonyl-2-cyan-1,4-dihydro-6-methyl-4-(3-nitrophenyl)-pyridin-5-carbonsäure-N-[(1S)-1-äthoxycarbonyl-2-methyl-1-propyl]-amid ist ein S,4R : S,4S Diastereomerengemisch und zeigt folgende NMR-Daten:

250 MHz FT-$^{1}$H-NMR ($CDCl_3$):

0.74 (2m, 6H, $-C(CH_3)_2$); 1.26 (m, 6H, $-COO-C-CH_3$); 2.05 (m, 1H, $-CHC_2$); 2.24, 2.32 (2s, 3H, Dihydropyridyl-$CH_3$); 4.2 (m, 4H, $-COO-CH_2-C-$); 4.50 (m, 1H, -OOC-CH-N-CO-); 5.06, 5.10 (2s, 1H, 4-Dihydropyridyl-H); 5.74, 5.86 (2d, 1H, -NH-); 6.26, 6.38 (2s, 1H, -CO-NH-); 7.50, 7.70, 8.14 (t, m, m, 4H, Phenyl-H).

Beispiel 49: In völlig analoger Weise zu Beispiel 26 erhält man auch die folgenden Verbindungen in konfigurativ einheitlicher Form:

a) (-)-(4R)-1,4-Dihydro-2,6-dimethyl-3-methoxycarbonyl-4-(3-nitrophenyl)-pyridin-5-carbonsäure-N-[(1S)-1-(2-furylmethoxycarbonyl)-2-methyl-1-propyl]-amid; $[\alpha]_D^{20}$ = -38.4° (c = 0.91, Aethanol); Smp. 76-78°.

b) (-)-(4R)-1,4-Dihydro-2,6-dimethyl-3-methoxycarbonyl-4-(3-nitrophenyl)-pyridin-5-carbonsäure-N-[(1S)-1-isopropyloxycarbonyl-2-methyl-1-propyl]-amid; $[\alpha]_D^{20}$ = -18.5° (c = 0.56, Aethanol); Smp. 166-167°.

c) (4R)-1,4-Dihydro-2,6-dimethyl-3-methoxycarbonyl-4-(3-nitrophenyl)-pyridin-5-carbonsäure-N-[(1S)-1-(tert-butyloxycarbonyl)-2-methyl-1-propyl]-amid; Smp. 163-164°.

Patentansprüche für die Vertragsstaaten: BE, CH, LI, DE, FR, GB, IT, LU, NL, SE

1. Verbindungen der Formel I

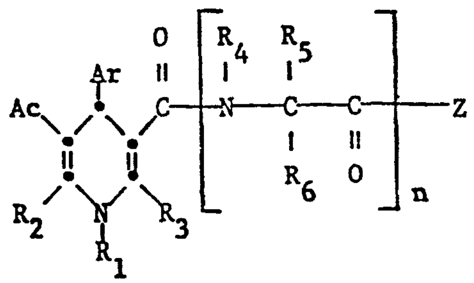

(I),

worin n für 1, 2 oder 3 steht, Ar einen carbocyclischen oder heterocyclischen Arylrest bedeutet, Ac den Acylrest einer Säure darstellt, Z für einen Rest $-OR_7$ oder $-NR_8R_9$ steht, $R_1$ Wasserstoff, unsubstituiertes oder substituiertes Niederalkyl, einen carbocyclischen oder heterocyclischen Arylrest oder freies, veräthertes oder verestertes Hydroxy bedeutet, $R_2$ und $R_3$ unabhängig voneinander für Wasserstoff, unsubstituiertes oder substituiertes Niederalkyl, Formyl oder funktionell abgewandeltes Formyl, Carboxy oder funktionell abgewandeltes Carboxy, einen carbocyclischen oder heterocyclischen Arylrest oder unsubstituiertes, mono- oder disubstituiertes Amino stehen, $R_4$ Wasserstoff oder Niederalkyl bedeutet, $R_5$ und $R_6$ unabhängig voneinander Wasserstoff, unsubstituiertes oder substituiertes Niederalkyl oder einen carbocyclischen oder heterocyclischen Arylrest darstellen, $R_7$, $R_8$ und $R_9$ unabhängig voneinander Wasserstoff, unsubstituiertes oder substituiertes Alkyl oder einen carbocyclischen oder heterocyclischen Arylrest bedeuten, worin $R_1$ und $R_2$ zusammen oder $R_1$ und $R_3$ zusammen unsubstituiertes oder substituiertes Niederalkylen, worin ein Kohlenstoffatom gegebenenfalls durch ein Heteroatom ersetzt ist, bedeuten können, worin $R_4$ und $R_5$ zusammen, ebenso wie $R_5$ und $R_6$ zusammen und/oder $R_8$ und $R_9$ zusammen, unabhängig voneinander unsubstituiertes oder substituiertes

Niederalkylen, worin ein Kohlenstoffatom durch ein Heteroatom ersetzt sein kann, bedeuten können, optische Isomere von Verbindungen der Formel I, Mischungen dieser optischen Isomere und Salze von solchen Verbindungen, die eine salzbildende Gruppierung aufweisen.

2. Verbindungen der Formel I gemäss Anspruch 1, worin n für 1, 2 oder 3 steht, Ar einen carbocyclischen oder heterocyclischen Arylrest bedeutet, Ac den Acylrest einer Säure darstellt, Z für einen Rest $-OR_7$ steht, $R_1$ und $R_7$ Wasserstoff oder unsubstituiertes oder substituiertes Niederalkyl bedeuten, $R_2$ und $R_3$ Niederalkyl bedeuten, $R_4$ Wasserstoff oder Niederalkyl bedeutet, $R_5$ Wasserstoff, unsubstituiertes oder substituiertes Niederalkyl, unsubstituiertes oder substituiertes Phenyl oder einen heterocyclischen Arylrest bedeutet, und $R_6$ Wasserstoff, Niederalkyl, gegebenenfalls im Phenylring substituiertes Phenylniederalkyl, unsubstituiertes oder substituiertes Phenyl oder einen heterocyclischen Arylrest bedeutet, worin $R_4$ und $R_5$ zusammen unsubstituiertes oder substituiertes Niederalkylen, Oxaniederalkylen oder Thianiederalkylen sein können und worin $R_5$ und $R_6$ zusammen unsubstituiertes oder substituiertes Niederalkylen, worin ein Kohlenstoffatom durch ein Heteroatom ersetzt sein kann, sein können, und Salze von solchen Verbindungen, die eine salzbildende Gruppierung aufweisen.

3. Verbindungen der Formel I gemäss Anspruch 1, worin n für 1, 2 oder 3 steht, Ar für einen mono- oder bicyclischen, carbocyclischen Arylrest oder für einen fünf- oder sechsgliedrigen, ein bis und mit vier Ringstickstoffatome, ein Ringsauerstoff- oder Ringschwefelatom, oder ein oder zwei Ringstickstoffatome zusammen mit einem Ringsauerstoff oder einem Ringschwefelatom als Ringglieder enthaltenden, monocyclischen Heteroarylrest, der gegebenenfalls einen ankondensierten Benzoring enthält, steht, und insbesondere Phenyl, Naphthyl, Pyrryl, Pyrazolyl, Imidazolyl, Triazolyl, Tetrazolyl, Furyl, Thienyl, Isoxazolyl, Oxazolyl, Oxadiazolyl, Isothiazolyl, Thiazolyl, Thiadiazolyl,

Pyridyl, Pyridazinyl, Pyrimidinyl, Pyrazinyl, Triazinyl, Indolyl, Isoindolyl, Benzoxadiazolyl, Benzthiadiazolyl, Benzimidazolyl, Benzofuranyl, Benzothienyl, Chinolinyl oder Isochinolinyl bedeutet, wobei in diesen Resten Ringkohlenstoffatome gegebenenfalls durch Niederalkyl, Niederalkenyl, Niederalkinyl, Niederalkylen, Cycloalkyl, Phenyl, Phenylniederalkyl, Phenylniederalkoxy und/oder Phenylniederalkylthio, wobei Niederalkyl, Phenyl, Phenylniederalkyl, Phenylniederalkoxy und/oder Phenylniederalkylthio gegebenenfalls Hydroxy, Niederalkoxy, Halogenniederalkoxy, Niederalkenyloxy, Niederalkinyloxy, Niederalkylendioxy, Niederalkanoyloxy, Halogen, Carboxy, Niederalkoxycarbonyl, Carbamoyl, N-Niederalkylcarbamoyl, N,N-Diniederalkyl-carbamoyl und/oder Cyan, die cyclischen Reste auch Niederalkyl, das seinerseits wie angegeben substituiert sein kann, als Substituenten enthalten können, und/oder durch Hydroxy, Niederalkoxy, Halogenniederalkoxy, Niederalkenyloxy, Niederalkinyloxy, Niederalkylendioxy, Niederalkanoyloxy, Halogen, Nitro, Amino, Niederalkylamino, Diniederalkylamino, N-Niederalkyl-N-phenylniederalkylamino, Niederalkylenamino, Oxaniederalkylenamino, Thianiederalkylenamino und/oder Azaniederalkylenamino, worin das Azastickstoffatom durch Niederalkyl, Phenyl oder Phenylniederalkyl substituiert sein kann, wobei diese Substituenten Hydroxy, Niederalkoxy, Halogenniederalkoxy, Niederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkyl-carbamoyl und/oder Cyan als Substituenten enthalten können, und/oder durch Niederalkanoylamino, Azido, Carboxy, Niederalkoxycarbonyl, Carbamoyl, N-Niederalkylcarbamoyl, N,N-Diniederalkyl-carbamoyl, Cyan, Sulfo, Aminosulfonyl, Niederalkylthio, Niederalkylsulfinyl und/oder Niederalkylsulfonyl, und/oder Ringstickstoffatome gegebenenfalls durch Niederalkoxycarbonyl oder durch Niederalkyl, das als Substituenten gegebenenfalls Hydroxy, Niederalkoxy, Halogenniederalkoxy, Niederalkenyloxy, Niederalkinyloxy, Niederalkylendioxy, Niederalkanoyloxy, Halogen, Carboxy, Niederalkoxycarbonyl, Carbamoyl, N-Niederalkyl-

carbamoyl, N,N-Diniederalkyl-carbamoyl oder Cyan enthalten kann, oder durch Hydroxy oder Oxido substituiert sein können, der Rest Ac für Niederalkanoyl, unsubstituiertes oder durch Niederalkyl, Niederalkoxy, Nitro und/oder Halogen substituiertes Benzoyl, Niederalkylsulfonyl, unsubstituiertes oder durch Niederalkyl, Niederalkoxy, Nitro und/oder Halogen substituiertes Phenylsulfonyl, Niederalkoxycarbonyl, Hydroxyniederalkoxycarbonyl, Niederalkoxyniederalkoxycarbonyl, Aminoniederalkoxycarbonyl, Niederalkylaminoniederalkoxycarbonyl, N,N-Diniederalkylaminoniederalkoxycarbonyl, N-Niederalkyl-N-phenylniederalkyl-aminoniederalkoxycarbonyl, N,N-Niederalkylenaminoniederalkoxycarbonyl, Morpholinoniederalkoxycarbonyl, Thiomorpholinoniederalkoxycarbonyl, Piperazinoniederalkoxycarbonyl, 4-Niederalkyl-piperazino-niederalkoxycarbonyl, unsubstituiertes oder durch Niederalkyl, Niederalkoxy, Nitro und/oder Halogen substituiertes Phenyl-, Thienyl-, Furyl-, Pyrryl- oder Pyridyl-niederalkoxycarbonyl, Niederalkenyloxy- oder Niederalkinyloxycarbonyl, Carbamoyl, N-Niederalkyl-carbamoyl, N,N-Diniederalkylcarbamoyl, N-Hydroxycarbamoyl, N,N-Niederalkylencarbamoyl, Morpholinocarbonyl, Thiomorpholinocarbonyl, Piperazinocarbonyl, 4-Niederalkylpiperazinocarbonyl, 5-Tetrazolyl, unsubstituiertes oder durch Niederalkyl oder Phenyl substituiertes 4,5-Dihydro-2-oxazolyl oder 5,6-Dihydro-4H-1,3-oxazin-2-yl steht, Z einen Rest $-OR_7$ oder $-NR_8R_9$ bedeutet, $R_1$ Wasserstoff; Niederalkyl, das gegebenenfalls durch Niederalkylamino, Diniederalkylamino, Niederalkylenamino, Morpholino, Thiomorpholino, Piperazino, welches an einem Stickstoffatom gegebenenfalls Niederalkyl oder Niederalkanoyl als Substituenten trägt, Carboxy, funktionell abgewandeltes Carboxy, Niederalkoxy, Niederalkoxy-niederalkoxy oder Phenyl, das seinerseits gegebenenfalls Niederalkyl, Niederalkoxy, Halogen und/oder Nitro als Substituenten enthält, oder N-Pyrrolyl, N-Imidazolyl, N-Pyrazolyl, N-Indolyl oder N-Isoindolyl substituiert ist; Phenyl, das gegebenenfalls wie ein Phenyl-niederalkyl-Rest $R_1$ substituiert ist; Hydroxy, Niederalkoxy oder Phenylniederalkoxy darstellt, $R_2$ und $R_3$ unabhängig voneinander Wasserstoff, Niederalkyl, das gegebenenfalls durch Hydroxy, Niederalkoxy, das gegebenenfalls Amino ,

Niederalkylamino, Diniederalkylamino oder Acylamino als Substituenten enthält, Acyloxy oder Phenyl substituiert ist, Formyl, Diniederalkylacetal oder -dithioacetal, Niederalkylenacetal oder -dithioacetal, funktionell abgewandeltes Carboxy; Phenyl, das gegebenenfalls Niederalkyl, Hydroxy, Niederalkoxy, Niederalkylendioxy, Halogen, Nitro, Amino, Niederalkylamino, Diniederalkylamino, Niederalkanoylamino, Carboxy, funktionell abgewandeltes Carboxy und/oder Niederalkylthio als Substituenten enthält; Pyrryl, Furyl, Thienyl oder Pyridyl, wobei diese Reste gegebenenfalls wie ein Phenylrest $R_2$ oder $R_3$ substituiert sind; Amino, Niederalkylamino oder Diniederalkylamino bedeuten, $R_4$ Wasserstoff oder Niederalkyl bedeutet, $R_5$ Wasserstoff, unsubstituiertes oder durch freies oder veräthertes Hydroxy, freies oder veräthertes Mercapto, das gegebenenfalls oxidiert sein kann, durch Carboxy, funktionell abgewandeltes Carboxy, durch Amino, das gegebenenfalls seinerseits Niederalkyl, Carboxy, funktionell abgewandeltes Carboxy oder Acyl als Substituenten enthält, durch einen mono- oder bicyclischen carbocyclischen Arylrest oder einen fünf- oder sechsgliedrigen Heteroarylrest gemäss der obigen Definition für Ar substituiertes Niederalkyl, unsubstituiertes oder durch freies oder veräthertes Hydroxy, Niederalkyl, Nitro, Amino, Niederalkylamino, Diniederalkylamino, Niederalkanoylamino und/oder Halogen substituiertes Phenyl oder einen gegebenenfalls in gleicher Weise substituierten monocyclischen fünf- oder sechsgliedrigen Heteroarylrest gemäss der obigen Definition für Ar bedeutet, $R_6$ Wasserstoff, Niederalkyl, unsubstituiertes oder durch freies oder veräthertes Hydroxy, Niederalkyl, Nitro, Amino, Niederalkylamino, Diniederalkylamino, Niederalkanoylamino und/oder Halogen substituiertes Phenylniederalkyl, unsubstituiertes oder wie ein Phenylniederalkyl-Rest $R_6$ substituiertes Phenyl oder einen gegebenenfalls in gleicher Weise substituierten monocyclischen fünf- oder sechsgliedrigen Heteroarylrest bedeutet, und $R_7$, $R_8$ und $R_9$ unabhängig voneinander Wasserstoff; Alkyl, das gegebenenfalls durch Amino, Niederalkylamino, Diniederalkylamino, Niederalkylenamino, Oxa-, Thia- oder Azaniederalkylenamino, worin der Aza-Stickstoff gegebenenfalls Niederalkyl oder Niederalkanoyl als Substituenten trägt, Acylamino, freies oder veräthertes Hydroxy oder einen carbocyclischen Arylrest oder Heteroarylrest wie oben für die Gruppe Ar definiert substituiert ist; oder einen carbocyclischen Arylrest oder Heteroarylrest wie oben für die Gruppe Ar definiert bedeuten, worin $R_1$ und $R_2$ zusammen oder $R_1$ und $R_3$ zusammen $C_3$-$C_5$-Niederalkylen bedeuten können, in dem das mit dem C2- bzw. C6-Kohlenstoffatom des 1,4-Dihydropyridinrings direkt verbundene Kohlenstoffatom gegebenenfalls durch ein Sauerstoff-, Schwefel- oder ein Stickstoffatom, das durch Wasserstoff oder Niederalkyl substituiert ist, ersetzt ist, worin $R_4$ und $R_5$ zusammen unsubstituiertes oder durch freies oder veräthertes Hydroxy, Amino, Niederalkylamino, Diniederalkylamino und/oder Halogen, am Aza-Stickstoff auch durch Niederalkyl, substituiertes Niederalkylen, Oxa-, Thia- oder Azaniederalkylen bedeuten können, worin $R_5$ und $R_6$ zusammen Niederalkylen, Aza-, Oxa- oder Thianiederalkylen bedeuten können, wobei diese Reste gegebenenfalls an Kohlenstoffatomen durch freies oder veräthertes Hydroxy, Amino, Niederalkylamino, Diniederalkylamino, Halogen, Carboxy und/oder funktionell abgewandeltes Carboxy und am Aza-Stickstoff gegebenenfalls durch Niederalkyl substituiert sind, und worin $R_8$ und $R_9$ zusammen Niederalkylen, Aza-, Oxa- oder Thianiederalkylen bedeuten können, wobei diese Reste gegebenenfalls an Kohlenstoffatomen durch freies oder veräthertes Hydroxy, Amino, Niederalkylamino, Diniederalkylamino, Halogen, Carboxy, funktionell abgewandeltes Carboxy oder einen fünf- oder sechsgliedrigen Monoaza-, Diaza- oder Triaza-Heteroarylrest, der gegebenenfalls ganz oder partiell gesättigt und gegebenenfalls an Kohlenstoffatomen durch Oxo substituiert ist und an den Azastickstoffatomen gegebenenfalls durch Niederalkyl oder Phenyl, welches seinerseits Niederalkyl, Halogen, Niederalkoxy und/oder Nitro als Substituenten enthalten kann, substituiert ist; und am Aza-Stickstoff gegebenenfalls durch Niederalkyl, das seinerseits gegebenenfalls einen carbocyclischen Arylrest oder Heteroarylrest wie oben für die Gruppe Ar definiert und/oder freies oder veräthertes Hydroxy, Acyloxy, Amino, Niederalkylamino

und/oder Diniederalkylamino als Substituenten enthält; durch Acyl oder Phenyl, welches seinerseits Niederalkyl, Halogen, Niederalkoxy und/oder Nitro als Substituenten enthalten kann, substituiert sind, optische Isomere von Verbindungen der Formel I, Mischungen dieser optischen Isomere und Salze von solchen Verbindungen mit salzbildenden Gruppen.

4. Verbindungen der Formel I gemäss Anspruch 1, worin n für 1, 2 oder 3 steht, Ar Phenyl, das gegebenenfalls durch Niederalkyl, Phenyl, Phenylniederalkyl, Phenylniederalkoxy und/oder Phenylniederalkylthio, wobei solche Reste ihrerseits Hydroxy, Niederalkoxy, Niederalkylendioxy, Halogen, Carboxy, Niederalkoxycarbonyl und/oder Cyan, die cyclischen Reste auch Niederalkyl als Substituenten enthalten können, und/oder durch Hydroxy, Niederalkoxy, Halogen-niederalkoxy, Niederalkylendioxy, Halogen, Nitro, Amino, Niederalkylamino, Diniederalkylamino, Niederalkanoylamino, Carboxy, Niederalkoxycarbonyl, Carbamoyl, Cyan, Sulfo, Aminosulfonyl, Niederalkylthio, Niederalkylsulfinyl und/oder Niederalkylsulfonyl substituiert ist, oder Pyrryl, Furyl, Thienyl, Pyridyl, Benzoxadiazolyl oder Benzthiadiazolyl, wobei diese Reste gegebenenfalls wie ein Phenylrest Ar substituiert sind, und insbesondere Niederalkyl, Niederalkoxy, Halogen und/oder gegebenenfalls durch Niederalkyl, Niederalkoxy, Halogen und/oder Nitro substituiertes Phenyl als Substituenten enthalten, bedeutet, der Rest Ac für Niederalkanoyl, Niederalkylsulfonyl, Niederalkoxycarbonyl, Hydroxy-niederalkoxycarbonyl, Niederalkoxyniederalkoxycarbonyl, N,N-Diniederalkylamino-niederalkoxycarbonyl, N-Niederalkyl-N-phenylniederalkyl-amino-niederalkoxycarbonyl, N,N-Niederalkylenamino-niederalkoxycarbonyl, (4-Morpholino)-niederalkoxycarbonyl, Niederalkenyloxy- oder Niederalkinyloxycarbonyl, Carbamoyl, N-Niederalkyl-carbamoyl, N,N-Diniederalkyl-carbamoyl, N,N-Niederalkylencarbamoyl, 4-Morpholinocarbonyl oder 4-Niederalkyl-1-piperazino-carbonyl steht, Z einen Rest $-OR_7$ oder $-NR_8R_9$ bedeutet, $R_1$ für Wasserstoff, Niederalkyl, Diniederalkylaminoniederalkyl, Niederalkylenaminoniederalkyl, (4-Morpholino)-niederalkyl, Carboxy-niederalkyl, Niederalkoxycarbonyl-niederalkyl oder Niederalkoxyniederalkoxy-niederalkyl, wobei Diniederalkylamino, Niederalkylenamino bzw. 4-Morpholino durch mindestens zwei Kohlenstoffatome vom Ringstickstoffatom getrennt sind, oder für Phenylniederalkyl, Phenyl, Hydroxy oder Niederalkoxy steht, $R_2$ und $R_3$ unabhängig voneinander Niederalkyl, das gegebenenfalls durch Hydroxy, Niederalkoxy, das gegebenenfalls Amino, Niederalkylamino oder Diniederalkylamino als Substituenten enthält, Niederalkanoyloxy oder Phenyl substituiert ist, Formyl, Diniederalkylacetal oder -dithioacetal, Niederalkylenacetal oder -dithioacetal, Cyan, Phenyl, Thienyl oder Amino bedeuten, $R_4$ Wasserstoff oder Niederalkyl bedeutet, $R_5$ Wasserstoff, unsubstituiertes oder durch Hydroxy, Niederalkoxy, Mercapto, Niederalkylthio, Carboxy, Carbamoyl, Amino, Niederalkanoylamino, Carbamoylamino, Guanidino, durch Phenyl, welches seinerseits durch Hydroxy und/oder Halogen substituiert sein kann, Imidazolyl oder Indolyl substituiertes Niederalkyl, unsubstituiertes oder durch Hydroxy, Niederalkoxy, Niederalkyl, Nitro, Amino, Niederalkylamino, Diniederalkylamino und/oder Halogen substituiertes Phenyl oder unsubstituiertes oder in gleicher Weise wie für einen Phenylrest $R_5$ angegeben substituiertes Pyrryl, Pyrazolyl, Imidazolyl, Triazolyl, Tetrazolyl, Furyl, Thienyl, Isoxazolyl, Oxazolyl, Oxadiazolyl, Isothiazolyl, Thiazolyl, Thiadiazolyl, Pyridyl, Pyridazinyl, Pyrimidinyl, Pyrazinyl oder Triazinyl bedeutet, $R_6$ Wasserstoff, Niederalkyl, Phenylniederalkyl, unsubstituiertes oder durch Hydroxy, Niederalkoxy, Niederalkyl, Nitro, Amino, Niederalkylamino, Diniederalkylamino und/oder Halogen substituiertes Phenylniederalkyl oder einen unsubstituierten oder in gleicher Weise wie für einen Phenylniederalkylrest $R_6$ angegeben substituierten Rest aus der Gruppe Phenyl, Pyrryl, Pyrazolyl, Imidazolyl, Triazolyl, Tetrazolyl, Furyl, Thienyl, Isoxazolyl, Oxazolyl, Oxadiazolyl, Isothiazolyl, Thiazolyl, Thiadiazolyl, Pyridyl, Pyridazinyl, Pyrimidinyl, Pyrazinyl und Triazinyl bedeutet, und $R_7$, $R_8$ und $R_9$ unabhängig voneinander Wasserstoff, Niederalkyl oder durch Amino, Niederalkylamino, Diniederalkylamino, Niederalkylenamino, Oxa-, Thia- oder Azaniederalkylenamino, worin der Aza-Stickstoff gegebenenfalls durch Niederalkyl substituiert ist, Niederalkanoylamino, Hydroxy, Niederalkoxy oder durch Phenyl, welches unsubstituiert oder seinerseits durch Amino, Niederalkylamino, Diniederalkylamino, Niederalkylenamino, Hydroxy, Niederalkoxy, Halogen

und/oder Nitro substituiert sein kann, oder durch Pyrryl, Furyl, Thienyl oder Pyridyl, wobei diese Gruppen in gleicher Weise wie Phenyl substituiert sein können, substituiertes Niederalkyl oder Phenyl, Pyrryl, Furyl, Thienyl oder Pyridyl, wobei diese Gruppen in gleicher Weise wie ein Phenyl-niederalkyl-Rest $R_7$, $R_8$ oder $R_9$ substituiert sein können, bedeuten, worin $R_1$ und $R_2$ zusammen oder $R_1$ und $R_3$ zusammen $C_3$-$C_5$-Niederalkylen bedeuten können, in dem das mit dem C2 bzw. C6-Kohlenstoffatom des 1,4-Dihydropyridinrings direkt verbundene Kohlenstoffatom gegebenenfalls durch ein Sauerstoff-, Schwefel- oder ein Stickstoffatom, welches durch Wasserstoff oder Niederalkyl substituiert ist, ersetzt ist, worin $R_4$ und $R_5$ zusammen unsubstituiertes oder hydroxysubstituiertes $C_3$-$C_5$-Niederalkylen, $C_2$-$C_4$-Oxa- oder $C_2$-$C_4$-Thianiederalkylen bedeuten können, worin $R_5$ und $R_6$ zusammen Niederalkylen mit 2 bis 5 Kettenkohlenstoffatomen oder Azaniederalkylen mit 3 oder 4 Kettenkohlenstoffatomen bedeuten können, wobei diese Reste unsubstituiert oder durch Hydroxy, Niederalkoxy, Amino, Niederalkylamino oder Diniederalkylamino substituiert sein können, und

worin $R_8$ und $R_9$ zusammen $C_2$-$C_7$-Niederalkylen, $C_3$-$C_4$-Aza-, $C_3$-$C_4$-Oxa- oder $C_3$-$C_4$-Thianiederalkylen bedeuten können, wobei diese Reste gegebenenfalls an Kohlenstoffatomen durch Hydroxy, Niederalkoxy, Amino, Niederalkylamino, Diniederalkylamino oder fünf- oder sechsgliedriges Monoaza- oder Diazaheterocyclyl, das gegebenenfalls an Kohlenstoffatomen durch Oxo substituiert ist und\an den Azastickstoffatomen gegebenenfalls durch Niederalkyl oder Phenyl, welches seinerseits Niederalkyl, Halogen, Niederalkoxy und/oder Nitro als Substituenten enthalten kann, substituiert ist; und am Aza-Stickstoff gegebenenfalls durch Niederalkyl, das seinerseits durch Phenyl, Pyrryl, Furyl, Thienyl und/oder Pyridyl, wobei diese Reste selbst gegebenenfalls Niederalkyl, Hydroxy, Niederalkoxy, Halogen und/oder Nitro als Substituenten enthalten, und oder durch Hydroxy, Niederalkoxy, Niederalkanoyloxy, Amino, Niederalkylamino und/oder Diniederalkylamino substituiert sein kann; durch Benzoyl, Niederalkanoyl, Furanylcarbonyl, Thienylcarbonyl, Pyrrylcarbonyl,

Pyridinylcarbonyl oder Phenyl, welches seinerseits Niederalkyl, Halogen, Niederalkoxy und/oder Nitro als Substituenten enthalten kann, substituiert sind, optische Isomere von Verbindungen der Formel I, Mischungen dieser optischen Isomere und Salze von solchen Verbindungen mit salzbildenden Gruppen.

5. Verbindungen der Formel I gemäss Anspruch 2, worin n für 1, 2 oder 3 steht, Ar für Phenyl, das gegebenenfalls durch Niederalkyl, Phenyl und/oder Phenylniederalkyl, wobei solche Reste ihrerseits Hydroxy, Niederalkoxy, Niederalkylendioxy, Halogen, Carboxy, Niederalkoxycarbonyl und/oder Cyan, die cyclischen Reste auch Niederalkyl als Substituenten enthalten können, und/oder durch Hydroxy, Niederalkoxy, Halogen-niederalkoxy, Niederalkylendioxy, Halogen, Nitro, Amino, N-Niederalkylamino, N,N-Diniederalkylamino, Niederalkanoylamino, Carboxy, Niederalkoxycarbonyl, Carbamoyl, Cyan, Sulfo, Aminosulfonyl, Niederalkylthio, Niederalkylsulfinyl und/oder Niederalkylsulfonyl substituiert sein kann, oder für Pyrryl, Furyl, Thienyl oder Pyridyl, die gegebenenfalls wie ein Phenylrest Ar substituiert sein können, und insbesondere Niederalkyl, Niederalkoxy, Halogen und/oder gegebenenfalls durch Niederalkyl, Niederalkoxy, Halogen und/oder Nitro substituiertes Phenyl als Substituenten enthalten, steht, der Rest Ac für Niederalkanoyl, Niederalkylsulfonyl, Niederalkoxycarbonyl, Hydroxyniederalkoxycarbonyl, Niederalkoxyniederalkoxycarbonyl, N,N-Diniederalkylaminoniederalkoxycarbonyl, N-Niederalkyl-N-phenylniederalkyl-amino-niederalkoxycarbonyl, N,N-Niederalkylenaminoniederalkoxycarbonyl, (4-Morpholino)-niederalkoxycarbonyl, Carbamoyl, N-Niederalkyl-carbamoyl, N,N-Diniederalkyl-carbamoyl, N-N-Niederalkylencarbamoyl, 4-Niederalkyl-piperazino-carbonyl oder 4-Morpholinocarbonyl steht, Z für einen Rest $-OR_7$ steht, $R_1$ Wasserstoff, Niederalkyl, Diniederalkylaminoniederalkyl, Niederalkylenaminoniederalkyl, (4-Morpholino)-niederalkyl, Carboxy-niederalkyl oder Niederalkoxyniederalkoxy-niederalkyl darstellt, wobei Diniederalkylamino, Niederalkylenamino bzw. 4-Morpholino durch mindestens zwei Kohlenstoffatome vom Ringstickstoffatom getrennt

sind, $R_2$ und $R_3$ Niederalkyl bedeuten, $R_4$ Wasserstoff oder Niederalkyl bedeutet, $R_5$ Wasserstoff, unsubstituiertes oder durch Hydroxy, Niederalkoxy, Mercapto, Niederalkylthio, Carboxy, Carbamoyl, Amino, Niederalkanoylamino, Carbamoylamino, Guanidino, durch Phenyl, welches seinerseits durch Hydroxy und/oder Halogen substituiert sein kann, Imidazolyl oder Indolyl substituiertes Niederalkyl, unsubstituiertes oder durch Hydroxy, Niederalkoxy, Amino, N-Niederalkylamino oder N,N-Diniederalkylamino substituiertes Phenyl oder unsubstituiertes oder in gleicher Weise wie für Phenyl angegeben substituiertes Pyrryl, Pyrazolyl, Imidazolyl, Triazolyl, Tetrazolyl, Furyl, Thienyl, Isoxazolyl, Oxazolyl, Oxadiazolyl, Isothiazolyl, Thiazolyl, Thiadiazolyl, Pyridyl, Pyridazinyl, Pyrimidinyl, Pyrazinyl oder Triazinyl bedeutet, $R_6$ Wasserstoff, Niederalkyl, Phenylniederalkyl, unsubstituiertes oder durch Hydroxy, Niederalkoxy, Amino, N-Niederalkylamino oder N,N-Diniederalkylamino substituiertes Phenyl oder unsubstituiertes oder in gleicher Weise wie für Phenyl angegeben substituiertes Pyrryl, Pyrazolyl, Imidazolyl, Triazolyl, Tetrazolyl, Furyl, Thienyl, Isoxazolyl, Oxazolyl, Oxadiazolyl, Isothiazolyl, Thiazolyl, Thiadiazolyl, Pyridyl, Pyridazinyl, Pyrimidinyl, Pyrazinyl oder Triazinyl bedeutet, und $R_7$ Wasserstoff oder unsubstituiertes oder durch Amino, N-Niederalkylamino, N,N-Diniederalkylamino, Niederalkylenamino, Oxa-, Thia- oder gegebenenfalls azasubstituiertes Azaniederalkylenamino, Acylamino oder durch Phenyl, welches unsubstituiert oder seinerseits durch unsubstituiertes, mono- oder disubstituiertes Amino, Hydroxy, Niederalkoxy oder Halogen substituiert sein kann, substituiertes Niederalkyl bedeutet, worin $R_4$ und $R_5$ zusammen unsubstituiertes oder hydroxy-substituiertes Niederalkylen, Oxa- oder Thianiederalkylen bedeuten können und worin $R_5$ und $R_6$ zusammen Niederalkylen oder Azaniederalkylen sein können, wobei diese Reste unsubstituiert oder durch Hydroxy, Niederalkoxy, Amino, N-Niederalkylamino oder N,N-Diniederalkylamino substituiert sein können, wobei Niederalkyl, Niederalkoxy und Niederalkanoyl bis und mit 4 Kohlenstoffatome, Niederalkylen 3, 4 oder 5 Kettenkohlenstoffatome enthalten, und Salze von solchen Verbindungen mit salzbildenden Gruppen.

6. Verbindungen der Formel I gemäss Anspruch 2, worin n für 1 oder 2 steht, Ar für Phenyl, Thienyl oder Furyl steht, das gegebenenfalls durch Niederalkyl, Hydroxy, Niederalkoxy, Halogen-niederalkoxy, Niederalkylendioxy, Halogen, Trifluormethyl, Nitro, Niederalkanoylamino und/oder Cyan mono-, di- oder trisubstituiert sein kann, der Rest Ac Niederalkanoyl, Niederalkylsulfonyl, Niederalkoxycarbonyl, 2-Niederalkoxy-niederalkoxycarbonyl, Diniederalkylamino-niederalkoxycarbonyl, N-Niederalkyl-N-phenylniederalkyl-amino-niederalkoxycarbonyl, Carbamoyl, N-Niederalkyl-carbamoyl, N,N-Diniederalkylcarbamoyl, N,N-Niederalkylenamino-carbonyl oder 4-Morpholino-carbonyl bedeutet, Z für einen Rest $-OR_7$ steht, $R_1$ Wasserstoff, Niederalkyl, 2-(Diniederalkylamino)-niederalkyl, 2-(Niederalkylenamino)-niederalkyl, 2-(Morpholino)-niederalkyl, Carboxyniederalkyl oder Niederalkoxyniederalkoxyniederalkyl bedeutet, $R_2$ und $R_3$ Niederalkyl bedeuten, $R_4$ Wasserstoff darstellt, $R_5$ Wasserstoff, unsubstituiertes oder durch Hydroxy, Mercapto, Niederalkylthio, Carboxy, Carbamoyl, Amino, Carbamoylamino, Guanidino, durch Phenyl, welches seinerseits durch Hydroxy substituiert sein kann, Imidazol-4-yl oder Indol-3-yl substituiertes Niederalkyl oder unsubstituiertes oder durch Hydroxy, Niederalkoxy oder Amino substituiertes Phenyl, Thiazolyl, Imidazolyl, Furyl, Thienyl, Pyridyl oder Pyrimidinyl bedeutet, $R_6$ Wasserstoff, Niederalkyl, Phenylniederalkyl oder unsubstituiertes oder durch Hydroxy, Niederalkoxy oder Amino substituiertes Phenyl, Thiazolyl, Imidazolyl, Furyl, Thienyl, Pyridyl oder Pyrimidinyl bedeutet, und $R_7$ Wasserstoff oder unsubstituiertes oder durch Amino, N-Niederalkylamino, N,N-Diniederalkylamino oder Phenyl, welches unsubstituiert oder seinerseits durch Amino, Hydroxy, Niederalkoxy oder Halogen substituiert sein kann, substituiertes Niederalkyl bedeutet, worin $R_4$ und $R_5$ zusammen Niederalkylen bedeuten können und worin $R_5$ und $R_6$ zusammen Niederalkylen oder Azaniederalkylen bedeuten können, wobei Niederalkyl, Niederalkoxy und Niederalkanoyl bis und mit 4 Kohlenstoffatome, Niederalkylen 3, 4 oder 5 Kettenkohlenstoffatome enthalten, und Salze von solchen Verbindungen mit salzbildenden Gruppen.

7. Verbindungen der Formel I gemäss Anspruch 1, worin n für 1 oder 2 steht, Ar für Phenyl, Thienyl, Furyl oder Benzoxadiazolyl steht, wobei diese Gruppen gegebenenfalls durch Niederalkyl, Niederalkoxy, Halogenniederalkoxy, Phenylniederalkoxy, Phenylniederalkylthio, Niederalkylendioxy, Halogen, Trifluormethyl, Nitro, Niederalkanoylamino und/oder Cyan mono-, di- oder trisubstituiert sind,der Rest Ac Niederalkanoyl, Niederalkylsulfonyl, Niederalkoxycarbonyl, 2-Niederalkoxy-niederalkoxycarbonyl, N,N-Diniederalkylamino-niederalkoxycarbonyl, N-Niederalkyl-N-phenylniederalkylamino-niederalkoxycarbonyl, Carbamoyl, N-Niederalkyl--carbamoyl, N,N-Diniederalkyl-carbamoyl, N,N-Niederalkylen-carbamoyl oder 4-Morpholinocarbonyl bedeutet, Z für einen Rest $-OR_7$ oder $-NR_8R_9$ steht, $R_1$ Wasserstoff, Niederalkyl, 2-(Diniederalkylamino)-niederalkyl, 2-(Niederalkylenamino)-niederalkyl, 2-(4-Morpholino)-niederalkyl, Carboxyniederalkyl oder Niederalkoxyniederalkoxy-niederalkyl bedeutet, $R_2$ und $R_3$ unabhängig voneinander Niederalkyl, Hydroxyniederalkyl, Cyan oder Amino bedeuten, $R_4$ Wasserstoff oder Niederalkyl darstellt, $R_5$ Wasserstoff, unsubstituiertes oder durch Hydroxy, Niederalkoxy, Mercapto, Niederalkylthio, Carboxy, Carbamoyl, Amino, Carbamoylamino, Guanidino, Phenyl, welches seinerseits durch Hydroxy substituiert sein kann, oder Imidazol-4-yl oder Indol-3-yl substituiertes Niederalkyl oder Phenyl, Thiazolyl, Imidazolyl, Furyl, Thienyl, Pyridyl oder Pyrimidinyl, wobei diese Reste gegebenenfalls durch Hydroxy, Niederalkoxy und/oder Amino substituiert sind, bedeutet, $R_6$ Wasserstoff, Niederalkyl, Phenylniederalkyl, welches unsubstituiert oder durch Hydroxy, Niederalkoxy und/oder Amino substituiert ist, oder Phenyl, Thiazolyl, Imidazolyl, Furyl, Thienyl, Pyridyl oder Pyrimidinyl, wobei diese Reste gegebenenfalls wie ein Phenylniederalkyl-Rest $R_6$ substituiert sind, bedeutet, und $R_7$, $R_8$ und $R_9$ unabhängig voneinander Wasserstoff oder unsubstituiertes oder durch Amino, Niederalkylamino, Diniederalkylamino, Niederalkylenamino, Oxa- oder Azaniederalkylenamino, worin der Aza-Stickstoff gegebenenfalls durch Niederalkyl substituiertist, Hydroxy, Niederalkoxy, Phenyl, welches unsubstituiert oder seinerseits durch Amino, Hydroxy, Niederalkoxy und/oder Halogen substituiert sein kann, oder durch Thienyl oder Pyridyl, wobei diese Gruppen in gleicher Weise wie Phenyl substituiert sein können,

substituiertes Niederalkyl bedeuten, worin $R_4$ und $R_5$ zusammen $C_3$-$C_4$-Niederalkylen bedeuten können, worin $R_5$ und $R_6$ zusammen $C_2$-$C_5$-Niederalkylen oder $C_4$-Azaniederalkylen bedeuten können und worin $R_8$ und $R_9$ zusammen $C_4$-$C_5$-Niederalkylen, das gegebenenfalls durch 2-Imidazolidinon-1-yl, welches in 3-Stellung Phenyl oder Niederalkyl als Substituenten enthalten kann, substituiert ist, $C_4$-Oxa- oder $C_4$-Azaniederalkylen bedeuten können, wobei der Aza-Stickstoff durch Niederalkyl, das seinerseits unsubstituiert oder durch Phenyl, welches Niederalkyl, Niederalkoxy, Halogen und/oder Nitro als Substituenten enthalten kann, Thienyl und/oder Pyridyl mono- oder disubstituiert ist,oder durch Benzoyl, Furanylcarbonyl, Phenyl oder Niederalkoxyphenyl substituiert sein kann, optische Isomere von Verbindungen der Formel I, Mischungen dieser optischen Isomere und pharmazeutisch verwendbare Salze von solchen Verbindungen mit salzbildenden Gruppen.

8. Verbindungen der Formel I gemäss Anspruch 1, worin n für 1 oder 2 steht, Ar für unsubstituiertes oder durch Niederalkyl, Niederalkoxy, Halogenniederalkoxy, Benzyloxy, Benzylthio, Halogen, Trifluormethyl, Nitro und/oder Cyan mono- oder disubstituiertes Phenyl, 2- oder 3-Thienyl oder 2,1,3-Benzoxadiazol-4-yl steht,

der Rest Ac Niederalkylsulfonyl oder Niederalkoxycarbonyl darstellt, Z für einen Rest $-OR_7$ oder $-NR_8R_9$ steht, $R_1$ Wasserstoff, ferner 2-(4-Morpholino)-äthyl bedeutet, $R_2$ Niederalkyl, Hydroxymethyl, Cyan oder Amino bedeutet, $R_3$ für Niederalkyl steht, $R_4$ Wasserstoff oder Niederalkyl darstellt, $R_5$ Wasserstoff, unsubstituiertes oder durch Phenyl, welches seinerseits durch Hydroxy substituiert sein kann, substituiertes Niederalkyl, Phenyl oder unsubstituiertes oder Amino-substituiertes Thiazolyl bedeutet, $R_6$ Wasserstoff, Niederalkyl oder Phenyl bedeutet, $R_7$ für Wasserstoff, Niederalkyl oder Niederalkyl, das durch Phenyl, Amino, Niederalkylamino, Diniederalkylamino, 4-Morpholino, 1-Piperazino, welches seinerseits am 4-Stickstoff Niederalkyl als Substituenten enthalten kann,oder Niederalkoxy substituiert ist, steht, $R_8$ und $R_9$ unabhängig voneinander Wasserstoff, unsubstituiertes

oder durch Niederalkoxy, Phenyl oder Pyridyl substituiertes Niederalkyl bedeuten, worin $R_4$ und $R_5$ zusammen 1,3-Propylen bedeuten können, worin $R_5$ und $R_6$ zusammen für 1,5-Pentylen stehen können, und worin $R_8$ und $R_9$ zusammen 1,5-Pentylen, 3-(3-Phenyl-2-imidazolidinon-1-yl)-1,5-pentylen, 3-(2-Imidazolidinon-1-yl)-1,5-pentylen oder 3-Oxa- oder 3-Aza-1,5-pentylen bedeuten können, wobei der Aza-Stickstoff gegebenenfalls durch Niederalkyl, Benzyl, Diphenylmethyl, wobei die Reste Benzyl oder Diphenylmethyl gegebenenfalls Halogen, Niederalkyl und/oder Niederalkoxy als Substituenten enthalten, Benzoyl, Furanylcarbonyl, Phenyl oder Niederalkoxyphenyl, substituiert ist, optische Isomere von Verbindungen der Formel I, Mischungen dieser optischen Isomere und pharmazeutisch verwendbare Salze von solchen Verbindungen mit salzbildenden Gruppen.

9. Verbindungen der Formel I gemäss Anspruch 1, worin n für 1 steht, Ar 2- oder 3-Nitro-phenyl, 2- oder 3-Difluormethoxy-phenyl, 2- oder 3-Methyl-phenyl, 2- oder 3-Trifluormethyl-phenyl, 2,3-Dimethyl-phenyl, 2,3-Dichlor-phenyl, 2- oder 3-Benzyloxy-phenyl oder 2- oder 3-Benzylthiophenyl, bedeutet, Ac Niederalkoxycarbonyl darstellt, Z für einen Rest $-OR_7$ oder $-NR_8R_9$ steht, $R_1$ Wasserstoff ist, $R_2$ und $R_3$ unabhängig voneinander Niederalkyl bedeuten, $R_4$ Wasserstoff ist, $R_5$ Niederalkyl oder Phenyl bedeutet, $R_6$ Wasserstoff darstellt, $R_7$ unsubstituiertes oder durch 4-Morpholino, 1-Piperazino oder 4-Niederalkyl-1-Piperazino substituiertes Niederalkyl bedeutet, $R_8$ und $R_9$ unabhängig voneinander für Wasserstoff, unsubstituiertes oder durch Phenyl oder Pyridyl substituiertes Niederalkyl stehen, und worin $R_8$ und $R_9$ zusammen 3-Aza-1,5-Pentylen, in dem der Aza-Stickstoff gegebenenfalls durch Benzyl oder Diphenylmethyl substituiert ist, bedeuten können, optische Isomere von Verbindungen der Formel I, Mischungen dieser optischen Isomere und pharmazeutisch verwendbare Salze von solchen Verbindungen mit salzbildenden Gruppen.

10. Verbindungen der Formel I gemäss Anspruch 2, worin n für 1 oder 2 steht, Ar für 2- oder 3-Nitro-phenyl, 2- oder 3-Difluormethoxy-phenyl, 2,3-Dimethyl-phenyl oder 2- oder 3-Trifluormethyl-phenyl steht, der

Rest Ac Niederalkoxycarbonyl darstellt, $R_1$ Wasserstoff bedeutet, $R_2$ und $R_3$ Methyl bedeuten, $R_4$ Wasserstoff darstellt, $R_5$ Wasserstoff, unsubstituiertes oder durch Phenyl, welches seinerseits durch Hydroxy substituiert sein kann, substituiertes Niederalkyl oder Phenyl bedeutet, $R_6$ Wasserstoff oder Niederalkyl bedeutet, und $R_7$ für Wasserstoff, Niederalkyl oder Phenylniederalkyl steht, worin $R_4$ und $R_5$ zusammen 1,3-Propylen bedeuten können, und worin $R_5$ und $R_6$ zusammen für 1,5-Pentylen stehen können, wobei Niederalkyl, Niederalkoxy und Niederalkanoyl bis und mit 4 Kohlenstoffatome enthalten, und Salze von solchen Verbindungen mit salzbildenden Gruppen.

11. Verbindungen der Formel I gemäss Anspruch 1, worin am $C_4$-Kohlenstoff des 1,4-Dihydropyridingerüsts die folgende Konfiguration vorliegt, bei der die Kohlenstoffatome C3, C4 und C5 in der Papierebene, der Rest Ar vor und das Wasserstoffatom hinter der Papierebene liegt:

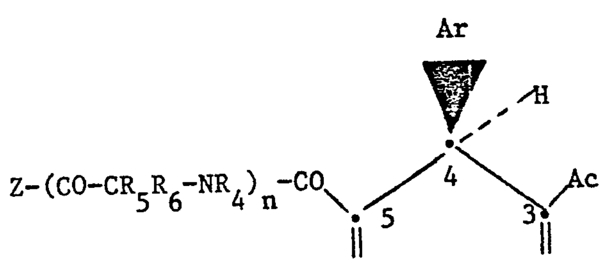

12. (-)-(4R)-1,4-Dihydro-2,6-dimethyl-3-methoxycarbonyl-4-(3-nitrophenyl)-pyridin-5-carbonsäure-N-[(1S)-1-äthoxycarbonyl-2-methyl-1-propyl]-amid und pharmazeutisch annehmbare Salze davon gemäss Anpruch 1.

13. (-)-(4R)-1,4-Dihydro-2,6-dimethyl-3-isopropoxycarbonyl-4-(3-nitro-phenyl)-pyridin-5-carbonsäure-N-[(1S)-1-äthoxycarbonyl-2-methyl-1-propyl)-amid und pharmazeutisch annehmbare Salze davon gemäss Anspruch 1.

14. (-)-(4R)-1,4-Dihydro-2,6-dimethyl-3-methoxycarbonyl-4-(3-nitrophenyl)-pyridin-5-carbonsäure-N-[(1S)-1-(n-butoxycarbonyl)-2-methyl-1-propyl]-amid und pharmazeutisch annehmbare Salze davon gemäss Anspruch 1.

15. (4R,4S)-1,4-Dihydro-2,6-dimethyl-3-methoxycarbonyl-4-(3-nitrophenyl)-pyridin-5-carbonsäure-N-[(1S,2S)-1-methoxycarbonyl-2-methyl-1-butyl]-amid, pharmazeutisch annehmbare Salze und die optischen Isomere davon gemäss Anspruch 1.

16. (4R)-1,4-Dihydro-2,6-dimethyl-3-methoxycarbonyl-4-(3-nitrophenyl)-pyridin-5-carbonsäure-N-[(1S,2S)-1-äthoxycarbonyl-2-methyl-1-butyl]-amid und pharmazeutisch annehmbare Salze davon gemäss Anspruch 1.

17. (4S,4R)-1,4-Dihydro-2,6-dimethyl-3-methoxycarbonyl-4-(3-nitrophenyl)-pyridin-5-carbonsäure-N-{(1S)-1-[2-(4-morpholino)-1-äthoxy]-carbonyl-2-methyl-1-propyl}-amid, pharmazeutisch annehmbare Salze und die optischen Isomere davon gemäss Anspruch 1.

18. 1,4-Dihydro-2,6-dimethyl-3-methoxycarbonyl-4-(3-nitrophenyl)-pyridin-5-carbonsäure-N-[1-(4-benzyl-1-piperazinyl)-carbonyl-2-methyl-1-propyl]-amid, pharmazeutisch annehmbare Salze und die optischen Isomere davon gemäss Anspruch 1.

19. (-)-(4R)-1,4-Dihydro-2,6-dimethyl-3-äthoxycarbonyl-4-(3-nitrophenyl)-pyridin-5-carbonsäure-N-[(1S)-1-äthoxycarbonyl-2-methyl-1-propyl]-amid und pharmazeutisch annehmbare Salze davon gemäss Anspruch 1.

20. (4S,4R)-1,4-Dihydro-2,6-dimethyl-3-methoxycarbonyl-4-(3-nitrophenyl)-pyridin-5-carbonsäure-N-[(1S)-1-(4-diphenylmethyl-1-piperazinyl)-carbonyl-2-methyl-1-propyl]-amid, pharmazeutisch annehmbare Salze und die optischen Isomere davon gemäss Anspruch 1.

21. Pharmazeutische Präparate, enthaltend eine Verbindung der Formel I gemäss Anspruch 1 oder ein pharmazeutisch annehmbares Salz davon.

22. Eine Verbindung der Formel I gemäss Anspruch 1 und pharmazeutisch annehmbare Salze davon zur Anwendung in einem Verfahren zur prophylaktischen und/oder therapeutischen Behandlung des tierischen oder menschlichen Körpers.

23. Eine Verbindung der Formel I gemäss Anspruch 1 und pharmazeutisch annehmbare Salze davon als blutdrucksenkende und coronardilatierende Verbindungen.

24. Verwendung von Verbindungen der Formel I gemäss Anspruch 1 oder von pharmazeutisch annehmbaren Salzen davon zur Herstellung von pharmazeutischen Präparaten.

25. Verfahren zur Herstellung von Verbindungen der Formel I gemäss Anspruch 1, oder Salzen von solchen Verbindungen mit salzbildenden Gruppen, dadurch gekennzeichnet, dass man

a) eine Verbindung der Formel II

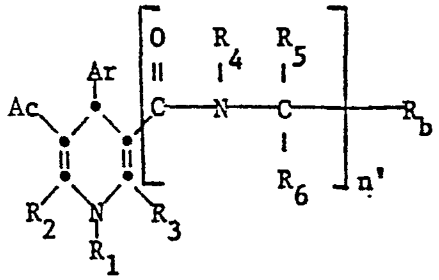

(II),

worin n' für 0, 1 oder 2 steht und $R_b$ eine gegebenenfalls aktivierte Carboxygruppe bedeutet, mit der Massgabe, dass $R_b$ von einem Rest C(=O)—Z verschieden ist, wenn n' für 1 oder 2 steht, mit einer Verbindung der Formel III

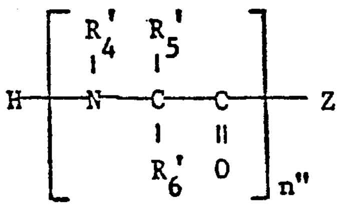

(III) ,

worin n" für 1, 2 oder 3 steht, und $R'_4$, $R'_5$ und $R'_6$ die unter Formel I für $R_4$, $R_5$ und $R_6$ angegebene Bedeutung haben, jedoch von den Resten $R_4$, $R_5$ bzw. $R_6$ in Formel II verschieden sein können, umsetzt, mit der Massgabe, dass die Summe aus n' und n" nicht grösser als 3 ist, oder

a') in einer Verbindung der Formel IIa

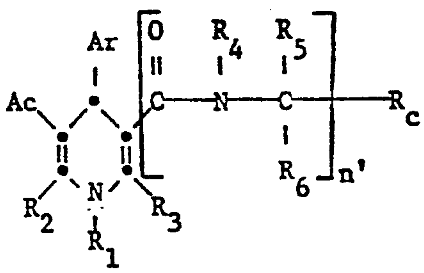

(IIa),

worin n' für 1, 2 oder 3 steht und $R_c$ eine in den Rest C(=O)-Z überführbare Gruppe ist, diese in den Rest C(=O)-Z überführt, oder

b) eine Verbindung der Formel IV, oder ein reaktionsfähiges Derivat davon, mit einer Verbindung der Formel V, oder einem Tautomeren davon, und mit einer Verbindung der Formel VI umsetzt,

(IV)

(VI)

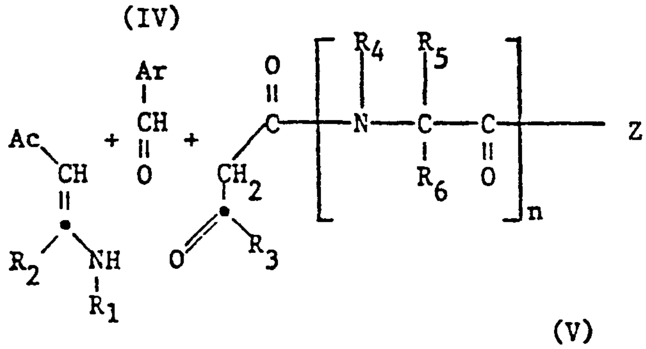

(V)

oder

b') völlig analog zu Verfahren b), eine Verbindung der Formel Va mit einer Verbindung der Formel VI umsetzt,

(VI) 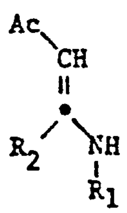 + 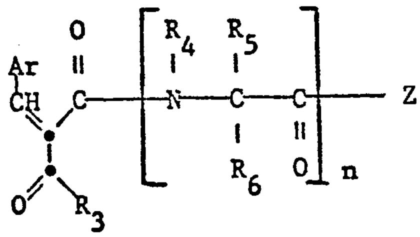 (Va)

c) eine Verbindung der Formel VII

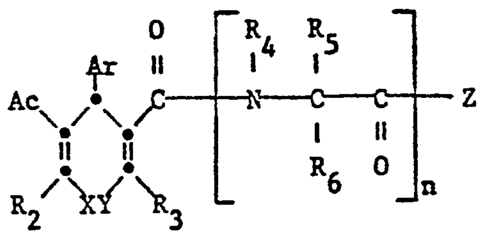 (VII) ,

worin einer der Reste X und Y für die Gruppe der Formel $-NH-R_1$ steht und der andere Hydroxy ist oder beide die Gruppe der Formel $-NH-R_1$ bedeuten, oder ein Tautomeres davon oder ein entsprechendes Tautomerengemisch ringschliesst, oder

d) eine Verbindung der Formel Ar-CHO (IV) oder ein reaktionsfähiges funktionelles Derivat davon mit einer Verbindung der Formel VIII

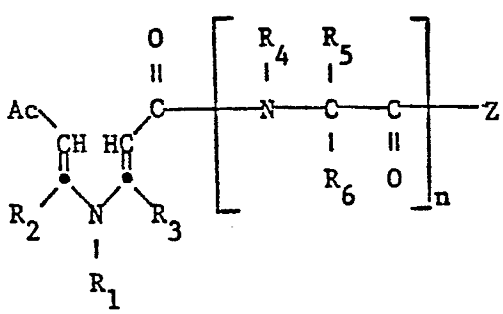 (VIII)

oder einem Tautomeren davon oder einem entsprechenden Tautomerengemisch umsetzt, oder

e) in einer Verbindung der Formel IX

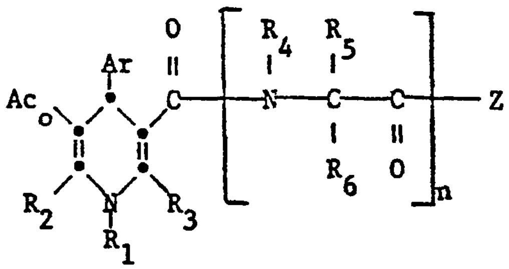

(IX) ,

worin $Ac_o$ einen in die Gruppe Ac überführbaren Rest darstellt, diesen in die Gruppe Ac überführt,

wobei in den obigen Ausgangsstoffen der Formeln II bis IX, IIa und Va, die, sofern sie salzbildende Eigenschaften aufweisen, auch in Form ihrer Salze verwendet werden können, die Symbole n, Ar, Ac, $R_1$, $R_2$, $R_3$, $R_4$, $R_5$, $R_6$ und Z die unter Formel I gegebenen Bedeutungen haben, und, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt, und/oder, wenn erwünscht, ein erhaltenes Gemisch von Isomeren oder Racematen in die einzelnen Isomere oder Racemate auftrennt, und/oder, wenn erwünscht, erhaltene Racemate in die optischen Antipoden aufspaltet.

26. Die nach dem Verfahren des Anspruchs 25 erhältlichen Verbindungen.

FO 7.4 BL/hc*

0145956

Patentansprüche für den Vertragsstaat AT

1. Verfahren zur Herstellung von Verbindungen der Formel I

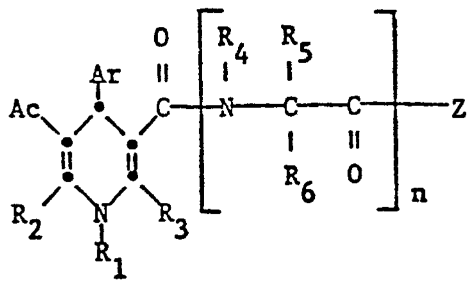

(I),

worin n für 1, 2 oder 3 steht, Ar einen carbocyclischen oder heterocyclischen Arylrest bedeutet, Ac den Acylrest einer Säure darstellt, Z für einen Rest $-OR_7$ oder $-NR_8R_9$ steht, $R_1$ Wasserstoff, unsubstituiertes oder substituiertes Niederalkyl, einen carbocyclischen oder heterocyclischen Arylrest oder freies, veräthertes oder verestertes Hydroxy bedeutet, $R_2$ und $R_3$ unabhängig voneinander für Wasserstoff, unsubstituiertes oder substituiertes Niederalkyl, Formyl oder funktionell abgewandeltes Formyl, Carboxy oder funktionell abgewandeltes Carboxy, einen carbocyclischen oder heterocyclischen Arylrest oder unsubstituiertes, mono- oder disubstituiertes Amino stehen, $R_4$ Wasserstoff oder Niederalkyl bedeutet, $R_5$ und $R_6$ unabhängig voneinander Wasserstoff, unsubstituiertes oder substituiertes Niederalkyl oder einen carbocyclischen oder heterocyclischen Arylrest darstellen, $R_7$, $R_8$ und $R_9$ unabhängig voneinander Wasserstoff, unsubstituiertes oder substituiertes Alkyl oder einen carbocyclischen oder heterocyclischen Arylrest bedeuten, worin $R_1$ und $R_2$ zusammen oder $R_1$ und $R_3$ zusammen unsubstituiertes oder substituiertes Niederalkylen, worin ein Kohlenstoffatom gegebenenfalls durch ein Heteroatom ersetzt ist, bedeuten können, worin $R_4$ und $R_5$ zusammen, ebenso wie $R_5$ und $R_6$ zusammen und/oder $R_8$ und $R_9$ zusammen, unabhängig voneinander unsubstituiertes oder substituiertes

Niederalkylen, worin ein Kohlenstoffatom durch ein Heteroatom ersetzt sein kann, bedeuten können, optischen Isomeren von Verbindungen der Formel I, Mischungen dieser optischen Isomere und Salzen von solchen Verbindungen, die eine salzbildende Gruppierung aufweisen, dadurch gekennzeichnet, dass man

a) eine Verbindung der Formel II

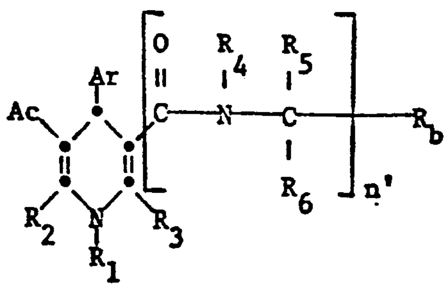

(II),

worin n' für 0, 1 oder 2 steht und $R_b$ eine gegebenenfalls aktivierte Carboxygruppe bedeutet, mit der Massgabe, dass $R_b$ von einem Rest C(=O)—Z verschieden ist, wenn n' für 1 oder 2 steht, mit einer Verbindung der Formel III

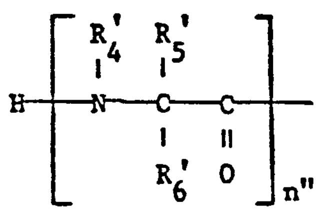

(III) ,

worin n" für 1, 2 oder 3 steht, und $R_4'$, $R_5'$ und $R_6'$ die unter Formel I für $R_4$, $R_5$ und $R_6$ angegebene Bedeutung haben, jedoch von den Resten $R_4$, $R_5$ bzw. $R_6$ in Formel II verschieden sein können, umsetzt, mit der Massgabe, dass die Summe aus n' und n" nicht grösser als 3 ist, oder

a') in einer Verbindung der Formel IIa

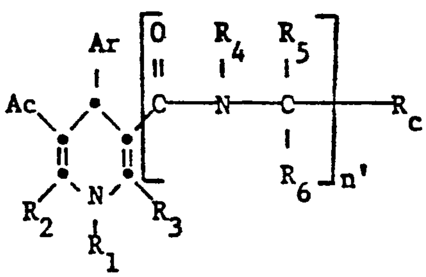

(IIa),

worin n' für 1, 2 oder 3 steht und $R_c$ eine in den Rest C(=O)-Z überführbare Gruppe ist, diese in den Rest C(=O)-Z überführt, oder

b) eine Verbindung der Formel IV, oder ein reaktionsfähiges Derivat davon, mit einer Verbindung der Formel V, oder einem Tautomeren davon, und mit einer Verbindung der Formel VI umsetzt,

(IV)

(VI) 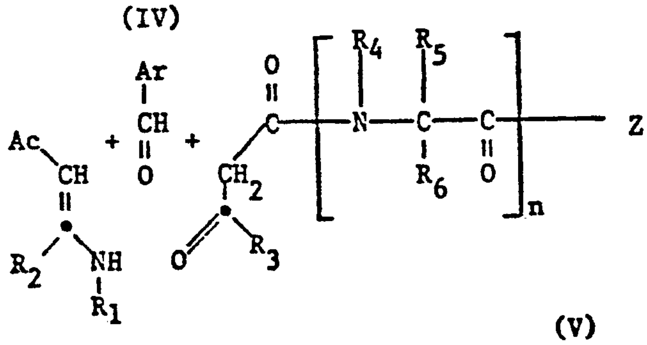

(V)

b') völlig analog zu Verfahren b), eine Verbindung der Formel Va mit einer Verbindung der Formel VI umsetzt,

(VI) 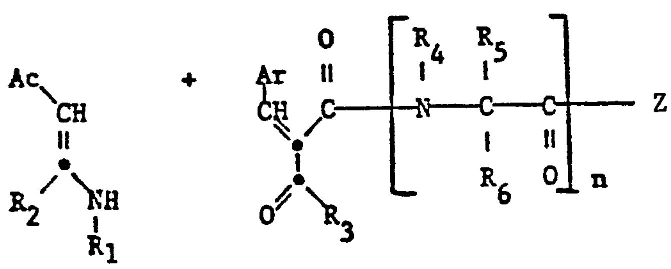 (Va)

c) eine Verbindung der Formel VII

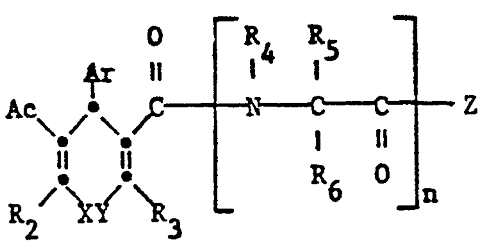 (VII) ,

worin einer der Reste X und Y für die Gruppe der Formel $-NH-R_1$ steht und der andere Hydroxy ist oder beide die Gruppe der Formel $-NH-R_1$ bedeuten, oder ein Tautomeres davon oder ein entsprechendes Tautomerengemisch ringschliesst, oder

d) eine Verbindung der Formel Ar-CHO (IV) oder ein reaktionsfähiges funktionelles Derivat davon mit einer Verbindung der Formel VIII

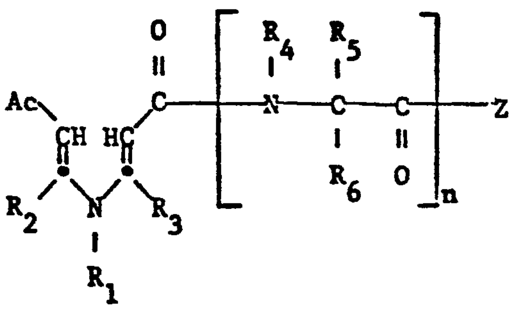

(VIII)

oder einem Tautomeren davon oder einem entsprechenden Tautomerengemisch umsetzt, oder

e) in einer Verbindung der Formel IX

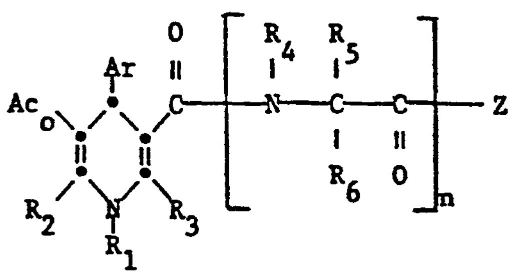

(IX) ,

worin $Ac_o$ einen in die Gruppe Ac überführbaren Rest darstellt, diesen in die Gruppe Ac überführt,

wobei in den obigen Ausgangsstoffen der Formeln II bis IX, IIa und Va, die, sofern sie salzbildende Eigenschaften aufweisen, auch in Form ihrer Salze verwendet werden können, die Symbole n, Ar, Ac, $R_1$, $R_2$, $R_3$, $R_4$, $R_5$, $R_6$ und Z die unter Formel I gegebenen Bedeutungen haben,

und, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt, und/oder, wenn erwünscht, ein erhaltenes Gemisch von Isomeren oder Racematen in die einzelnen Isomere oder Racemate auftrennt, und/oder, wenn erwünscht, erhaltene Racemate in die optischen Antipoden aufspaltet.

2. Verfahren gemäss Anspruch 1, dadurch gekennzeichnet, dass man Verbindungen der Formel I, worin n für 1, 2 oder 3 steht, Ar für einen mono- oder bicyclischen, carbocyclischen Arylrest oder für einen fünf- oder sechsgliedrigen, ein bis und mit vier Ringstickstoffatome, ein Ringsauerstoff- oder Ringschwefelatom, oder ein oder zwei Ringstickstoffatome zusammen mit einem Ringsauerstoff oder einem Ringschwefelatom als Ringglieder enthaltenden, monocyclischen Heteroarylrest, der gegebenenfalls einen ankondensierten Benzoring enthält, steht, und insbesondere Phenyl, Naphthyl, Pyrryl, Pyrazolyl, Imidazolyl, Triazolyl, Tetrazolyl, Furyl, Thienyl, Isoxazolyl, Oxazolyl, Oxadiazolyl, Isothiazolyl, Thiazolyl, Thiadiazolyl, Pyridyl, Pyridazinyl, Pyrimidinyl, Pyrazinyl, Triazinyl, Indolyl, Isoindolyl, Benzoxadiazolyl, Benzthiadiazolyl, Benzimidazolyl, Benzofuranyl, Benzothienyl, Chinolinyl oder Isochinolinyl bedeutet, wobei in diesen Resten Ringkohlenstoffatome gegebenenfalls durch Niederalkyl, Niederalkenyl, Niederalkinyl, Niederalkylen, Cycloalkyl, Phenyl, Phenylniederalkyl, Phenylniederalkoxy und/oder Phenylniederalkylthio, wobei Niederalkyl, Phenyl, Phenylniederalkyl, Phenylniederalkoxy und/oder Phenylniederalkylthio gegebenenfalls Hydroxy, Niederalkoxy, Halogenniederalkoxy, Niederalkenyloxy, Niederalkinyloxy, Niederalkylendioxy, Niederalkanoyloxy, Halogen, Carboxy, Niederalkoxycarbonyl, Carbamoyl, N-Niederalkylcarbamoyl, N,N-Diniederalkyl-carbamoyl und/oder Cyan,

die cyclischen Reste auch Niederalkyl, das seinerseits wie angegeben substituiert sein kann, als Substituenten enthalten können, und/oder durch Hydroxy, Niederalkoxy, Halogenniederalkoxy, Niederalkenyloxy, Niederalkinyloxy, Niederalkylendioxy, Niederalkanoyloxy, Halogen, Nitro, Amino, Niederalkylamino, Diniederalkylamino, N-Niederalkyl-N-phenylniederalkylamino, Niederalkylenamino, Oxaniederalkylenamino, Thianiederalkylenamino und/oder Azaniederalkylenamino, worin das Azastickstoffatom durch Niederalkyl, Phenyl oder Phenylniederalkyl substituiert sein kann, wobei diese Substituenten Hydroxy, Niederalkoxy, Halogenniederalkoxy, Niederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkylcarbamoyl und/oder Cyan als Substituenten enthalten können, und/oder durch Niederalkanoylamino, Azido, Carboxy, Niederalkoxycarbonyl, Carbamoyl, N-Niederalkylcarbamoyl, N,N-Diniederalkyl-carbamoyl, Cyan, Sulfo, Aminosulfonyl, Niederalkylthio, Niederalkylsulfinyl und/oder Niederalkylsulfonyl, und/oder Ringstickstoffatome gegebenenfalls durch Niederalkoxycarbonyl oder durch Niederalkyl, das als Substituenten gegebenenfalls Hydroxy, Niederalkoxy, Halogenniederalkoxy, Niederalkenyloxy, Niederalkinyloxy, Niederalkylendioxy, Niederalkanoyloxy, Halogen, Carboxy, Niederalkoxycarbonyl, Carbamoyl, N-Niederalkylcarbamoyl, N,N-Diniederalkyl-carbamoyl oder Cyan enthalten kann, oder durch Hydroxy oder Oxido substituiert sein können, der Rest Ac für Niederalkanoyl, unsubstituiertes oder durch Niederalkyl, Niederalkoxy, Nitro und/oder Halogen substituiertes Benzoyl, Niederalkylsulfonyl, unsubstituiertes oder durch Niederalkyl, Niederalkoxy, Nitro und/oder Halogen substituiertes Phenylsulfonyl, Niederalkoxycarbonyl, Hydroxyniederalkoxycarbonyl, Niederalkoxyniederalkoxycarbonyl, Aminoniederalkoxycarbonyl, Niederalkylaminoniederalkoxycarbonyl, N,N-Diniederalkylaminoniederalkoxycarbonyl, N-Niederalkyl-N-phenylniederalkyl-aminoniederalkoxycarbonyl, N,N-Nieder-

alkylenaminoniederalkoxycarbonyl, Morpholinoniederalkoxycarbonyl, Thiomorpholinoniederalkoxycarbonyl, Piperazinoniederalkoxycarbonyl, 4-Niederalkyl-piperazino-niederalkoxycarbonyl, unsubstituiertes oder durch Niederalkyl, Niederalkoxy, Nitro und/oder Halogen substituiertes Phenyl-, Thienyl-, Furyl-, Pyrryl- oder Pyridyl-niederalkoxycarbonyl, Niederalkenyloxy- oder Niederalkinyloxycarbonyl, Carbamoyl, N-Niederalkyl-carbamoyl, N,N-Diniederalkylcarbamoyl, N-Hydroxycarbamoyl, N,N-Niederalkylencarbamoyl, Morpholinocarbonyl, Thiomorpholinocarbonyl, Piperazinocarbonyl, 4-Niederalkylpiperazinocarbonyl, 5-Tetrazolyl, unsubstituiertes oder durch Niederalkyl oder Phenyl substituiertes 4,5-Dihydro-2-oxazolyl oder 5,6-Dihydro-4H-1,3-oxazin-2-yl steht, Z einen Rest $-OR_7$ oder $-NR_8R_9$ bedeutet, $R_1$ Wasserstoff; Niederalkyl, das gegebenenfalls durch Niederalkylamino, Diniederalkylamino, Niederalkylenamino, Morpholino, Thiomorpholino, Piperazino, welches an einem Stickstoffatom gegebenenfalls Niederalkyl oder Niederalkanoyl als Substituenten trägt, Carboxy, funktionell abgewandeltes Carboxy, Niederalkoxy, Niederalkoxy-niederalkoxy oder Phenyl, das seinerseits gegebenenfalls Niederalkyl, Niederalkoxy, Halogen und/oder Nitro als Substituenten enthält, oder N-Pyrrolyl, N-Imidazolyl, N-Pyrazolyl, N-Indolyl oder N-Isoindolyl substituiert ist; Phenyl, das gegebenenfalls wie ein Phenyl-niederalkyl-Rest $R_1$ substituiert ist; Hydroxy, Niederalkoxy oder Phenylniederalkoxy darstellt, $R_2$ und $R_3$ unabhängig voneinander Wasserstoff, Niederalkyl, das gegebenenfalls durch Hydroxy, Niederalkoxy, das gegebenenfalls Amino , Niederalkylamino, Diniederalkylamino oder Acylamino als Substituenten enthält, Acyloxy oder Phenyl substituiert ist, Formyl, Diniederalkylacetal oder -dithioacetal, Niederalkylenacetal oder -dithioacetal, funktionell abgewandeltes Carboxy; Phenyl, das gegebenenfalls Niederalkyl, Hydroxy, Niederalkoxy, Niederalkylendioxy, Halogen, Nitro, Amino, Niederalkylamino, Diniederalkylamino, Niederalkanoylamino, Carboxy, funktionell abgewandeltes Carboxy und/oder Niederalkylthio als Substituenten enthält; Pyrryl, Furyl, Thienyl oder Pyridyl, wobei diese Reste gegebenenfalls wie ein Phenylrest $R_2$ oder $R_3$ substituiert sind;

Amino, Niederalkylamino oder Diniederalkylamino bedeuten, $R_4$ Wasserstoff oder Niederalkyl bedeutet, $R_5$ Wasserstoff, unsubstituiertes oder durch freies oder veräthertes Hydroxy, freies oder veräthertes Mercapto, das gegebenenfalls oxidiert sein kann, durch Carboxy, funktionell abgewandeltes Carboxy, durch Amino, das gegebenenfalls seinerseits Niederalkyl, Carboxy, funktionell abgewandeltes Carboxy oder Acyl als Substituenten enthält, durch einen mono- oder bicyclischen carbocyclischen Arylrest oder einen fünf- oder sechsgliedrigen Heteroarylrest gemäss der obigen Definition für Ar substituiertes Niederalkyl, unsubstituiertes oder durch freies oder veräthertes Hydroxy, Niederalkyl, Nitro, Amino, Niederalkylamino, Diniederalkylamino, Niederalkanoylamino und/oder Halogen substituiertes Phenyl oder einen gegebenenfalls in gleicher Weise substituierten monocyclischen fünf- oder sechsgliedrigen Heteroarylrest gemäss der obigen Definition für Ar bedeutet, $R_6$ Wasserstoff, Niederalkyl, unsubstituiertes oder durch freies oder veräthertes Hydroxy, Niederalkyl, Nitro, Amino, Niederalkylamino, Diniederalkylamino, Niederalkanoylamino und/oder Halogen substituiertes Phenylniederalkyl, unsubstituiertes oder wie ein Phenylniederalkyl-Rest $R_6$ substituiertes Phenyl oder einen gegebenenfalls in gleicher Weise substituierten monocyclischen fünf- oder sechsgliedrigen Heteroarylrest bedeutet, und $R_7$, $R_8$ und $R_9$ unabhängig voneinander Wasserstoff; Alkyl, das gegebenenfalls durch Amino, Niederalkylamino, Diniederalkylamino, Niederalkylenamino, Oxa-, Thia- oder Azaniederalkylenamino, worin der Aza-Stickstoff gegebenenfalls Niederalkyl oder Niederalkanoyl als Substituenten trägt, Acylamino, freies oder veräthertes Hydroxy oder einen carbocyclischen Arylrest oder Heteroarylrest wie oben für die Gruppe Ar definiert substituiert ist; oder einen carbocyclischen Arylrest oder Heteroarylrest wie oben für die Gruppe Ar definiert bedeuten, worin $R_1$ und $R_2$ zusammen

oder $R_1$ und $R_3$ zusammen $C_3$-$C_5$-Niederalkylen bedeuten können, in dem das mit dem C2- bzw. C6-Kohlenstoffatom des 1,4-Dihydropyridinrings direkt verbundene Kohlenstoffatom gegebenenfalls durch ein Sauerstoff-, Schwefel- oder ein Stickstoffatom, das durch Wasserstoff oder Niederalkyl substituiert ist, ersetzt ist, worin $R_4$ und $R_5$ zusammen unsubstituiertes oder durch freies oder veräthertes Hydroxy, Amino, Niederalkylamino, Diniederalkylamino und/oder Halogen, am Aza-Stickstoff auch durch Niederalkyl, substituiertes Niederalkylen, Oxa-, Thia- oder Azaniederalkylen bedeuten können, worin $R_5$ und $R_6$ zusammen Niederalkylen, Aza-, Oxa- oder Thianiederalkylen bedeuten können, wobei diese Reste gegebenenfalls an Kohlenstoffatomen durch freies oder veräthertes Hydroxy, Amino, Niederalkylamino, Diniederalkylamino, Halogen, Carboxy und/oder funktionell abgewandeltes Carboxy und am Aza-Stickstoff gegebenenfalls durch Niederalkyl substituiert sind, und worin $R_8$ und $R_9$ zusammen Niederalkylen, Aza-, Oxa- oder Thianiederalkylen bedeuten können, wobei diese Reste gegebenenfalls an Kohlenstoffatomen durch freies oder veräthertes Hydroxy, Amino, Niederalkylamino, Diniederalkylamino, Halogen, Carboxy, funktionell abgewandeltes Carboxy oder einen fünf- oder sechsgliedrigen Monoaza-, Diaza- oder Triaza-Heteroarylrest, der gegebenenfalls ganz oder partiell gesättigt und gegebenenfalls an Kohlenstoffatomen durch Oxo substituiert ist und an den Azastickstoffatomen gegebenenfalls durch Niederalkyl oder Phenyl, welches seinerseits Niederalkyl, Halogen, Niederalkoxy und/oder Nitro als Substituenten enthalten kann, substituiert ist; und am Aza-Stickstoff gegebenenfalls durch Niederalkyl, das seinerseits gegebenenfalls einen carbocyclischen Arylrest oder Heteroarylrest wie oben für die Gruppe Ar definiert und/-oder freies oder veräthertes Hydroxy, Acyloxy, Amino, Niederalkylamino und/oder Diniederalkylamino als Substituenten enthält; durch Acyl oder Phenyl, welches seinerseits Niederalkyl, Halogen, Niederalkoxy und/oder Nitro als Substituenten enthalten kann, substituiert sind, optische Isomere von Verbindungen der Formel I, Mischungen dieser optischen Isomere und Salze von solchen Verbindungen mit salzbildenden Gruppen herstellt.

3. Verfahren gemäss Anspruch 1, dadurch gekennzeichnet, dass man Verbindungen der Formel I, worin n für 1, 2 oder 3 steht, Ar Phenyl, das gegebenenfalls durch Niederalkyl, Phenyl, Phenylniederalkyl, Phenylniederalkoxy und/oder Phenylniederalkylthio, wobei solche Reste ihrerseits Hydroxy, Niederalkoxy, Niederalkylendioxy, Halogen, Carboxy, Niederalkoxycarbonyl und/oder Cyan, die cyclischen Reste auch Niederalkyl als Substituenten enthalten können, und/oder durch Hydroxy, Niederalkoxy, Halogen-niederalkoxy, Niederalkylendioxy, Halogen, Nitro, Amino, Niederalkylamino, Diniederalkylamino, Niederalkanoylamino, Carboxy, Niederalkoxycarbonyl, Carbamoyl, Cyan, Sulfo, Aminosulfonyl, Niederalkylthio, Niederalkylsulfinyl und/oder Niederalkylsulfonyl substituiert ist, oder Pyrryl, Furyl, Thienyl, Pyridyl, Benzoxadiazolyl oder Benzthiadiazolyl, wobei diese Reste gegebenenfalls wie ein Phenylrest Ar substituiert sind, und insbesondere Niederalkyl, Niederalkoxy, Halogen und/oder gegebenenfalls durch Niederalkyl, Niederalkoxy, Halogen und/oder Nitro substituiertes Phenyl als Substituenten enthalten, bedeutet, der Rest Ac für Niederalkanoyl, Niederalkylsulfonyl, Niederalkoxycarbonyl, Hydroxy-niederalkoxycarbonyl, Niederalkoxyniederalkoxycarbonyl, N,N-Diniederalkylamino-niederalkoxycarbonyl, N-Niederalkyl-N-phenylniederalkyl-amino-niederalkoxycarbonyl, N,N-Niederalkylenamino-niederalkoxycarbonyl, (4-Morpholino)-niederalkoxycarbonyl, Niederalkenyloxy- oder Niederalkinyloxycarbonyl, Carbamoyl, N-Niederalkyl-carbamoyl, N,N-Diniederalkyl-carbamoyl, N,N-Niederalkylencarbamoyl, 4-Morpholinocarbonyl oder 4-Niederalkyl-1-piperazino-carbonyl steht, Z einen Rest $-OR_7$ oder $-NR_8R_9$ bedeutet, $R_1$ für Wasserstoff, Niederalkyl, Diniederalkylaminoniederalkyl, Niederalkylenaminoniederalkyl, (4-Morpholino)-niederalkyl, Carboxy-niederalkyl, Niederalkoxycarbonyl-niederalkyl oder Niederalkoxyniederalkoxy-niederalkyl, wobei Diniederalkylamino, Niederalkylenamino bzw. 4-Morpholino durch mindestens zwei Kohlenstoffatome vom Ringstickstoffatom getrennt sind, oder für Phenyl-

niederalkyl, Phenyl, Hydroxy oder Niederalkoxy steht, $R_2$ und $R_3$ unabhängig voneinander Niederalkyl, das gegebenenfalls durch Hydroxy, Niederalkoxy, das gegebenenfalls Amino, Niederalkylamino oder Diniederalkylamino als Substituenten enthält, Niederalkanoyloxy oder Phenyl substituiert ist, Formyl, Diniederalkylacetal oder -dithioacetal, Niederalkylenacetal oder -dithioacetal, Cyan, Phenyl, Thienyl oder Amino bedeuten, $R_4$ Wasserstoff oder Niederalkyl bedeutet, $R_5$ Wasserstoff, unsubstituiertes oder durch Hydroxy, Niederalkoxy, Mercapto, Niederalkylthio, Carboxy, Carbamoyl, Amino, Niederalkanoylamino, Carbamoylamino, Guanidino, durch Phenyl, welches seinerseits durch Hydroxy und/oder Halogen substituiert sein kann, Imidazolyl oder Indolyl substituiertes Niederalkyl, unsubstituiertes oder durch Hydroxy, Niederalkoxy, Niederalkyl, Nitro, Amino, Niederalkylamino, Diniederalkylamino und/oder Halogen substituiertes Phenyl oder unsubstituiertes oder in gleicher Weise wie für einen Phenylrest $R_5$ angegeben substituiertes Pyrryl, Pyrazolyl, Imidazolyl, Triazolyl, Tetrazolyl, Furyl, Thienyl, Isoxazolyl, Oxazolyl, Oxadiazolyl, Isothiazolyl, Thiazolyl, Thiadiazolyl, Pyridyl, Pyridazinyl, Pyrimidinyl, Pyrazinyl oder Triazinyl bedeutet, $R_6$ Wasserstoff, Niederalkyl, Phenylniederalkyl, unsubstituiertes oder durch Hydroxy, Niederalkoxy, Niederalkyl, Nitro, Amino, Niederalkylamino, Diniederalkylamino und/oder Halogen substituiertes Phenylniederalkyl oder einen unsubstituierten oder in gleicher Weise wie für einen Phenylniederalkylrest $R_6$ angegeben substituierten Rest aus der Gruppe Phenyl, Pyrryl, Pyrazolyl, Imidazolyl, Triazolyl, Tetrazolyl, Furyl, Thienyl, Isoxazolyl, Oxazolyl, Oxadiazolyl, Isothiazolyl, Thiazolyl, Thiadiazolyl, Pyridyl, Pyridazinyl, Pyrimidinyl, Pyrazinyl und Triazinyl bedeutet, und $R_7$, $R_8$ und $R_9$ unabhängig voneinander Wasserstoff, Niederalkyl oder durch Amino, Niederalkylamino, Diniederalkylamino, Niederalkylenamino, Oxa-, Thia- oder Azaniederalkylenamino, worin der Aza-Stickstoff gegebenenfalls durch Niederalkyl substituiert ist, Niederalkanoylamino, Hydroxy, Niederalkoxy oder durch Phenyl, welches unsubstituiert oder seinerseits durch Amino, Niederalkylamino, Diniederalkylamino, Niederalkylenamino, Hydroxy, Niederalkoxy, Halogen

und/oder Nitro substituiert sein kann, oder durch Pyrryl, Furyl, Thienyl oder Pyridyl, wobei diese Gruppen in gleicher Weise wie Phenyl substituiert sein können, substituiertes Niederalkyl oder Phenyl, Pyrryl, Furyl, Thienyl oder Pyridyl, wobei diese Gruppen in gleicher Weise wie ein Phenyl-niederalkyl-Rest $R_7$, $R_8$ oder $R_9$ substituiert sein können, bedeuten, worin $R_1$ und $R_2$ zusammen oder $R_1$ und $R_3$ zusammen $C_3$-$C_5$-Niederalkylen bedeuten können, in dem das mit dem C2 bzw. C6-Kohlenstoffatom des 1,4-Dihydropyridinrings direkt verbundene Kohlenstoffatom gegebenenfalls durch ein Sauerstoff-, Schwefel- oder ein Stickstoffatom, welches durch Wasserstoff oder Niederalkyl substituiert ist, ersetzt ist, worin $R_4$ und $R_5$ zusammen unsubstituiertes oder hydroxysubstituiertes $C_3$-$C_5$-Niederalkylen, $C_2$-$C_4$-Oxa- oder $C_2$-$C_4$-Thianiederalkylen bedeuten können, worin $R_5$ und $R_6$ zusammen Niederalkylen mit 2 bis 5 Kettenkohlenstoffatomen oder Azaniederalkylen mit 3 oder 4 Kettenkohlenstoffatomen bedeuten können, wobei diese Reste unsubstituiert oder durch Hydroxy, Niederalkoxy, Amino, Niederalkylamino oder Diniederalkylamino substituiert sein können, und worin $R_8$ und $R_9$ zusammen $C_2$-$C_7$-Niederalkylen, $C_3$-$C_4$-Aza-, $C_3$-$C_4$-Oxa- oder $C_3$-$C_4$-Thianiederalkylen bedeuten können, wobei diese Reste gegebenenfalls an Kohlenstoffatomen durch Hydroxy, Niederalkoxy, Amino, Niederalkylamino, Diniederalkylamino oder fünf- oder sechsgliedriges Monoaza- oder Diazaheterocyclyl, das gegebenenfalls an Kohlenstoffatomen durch Oxo substituiert ist und an den Azastickstoffatomen gegebenenfalls durch Niederalkyl oder Phenyl, welches seinerseits Niederalkyl, Halogen, Niederalkoxy und/oder Nitro als Substituenten enthalten kann, substituiert ist; und am Aza-Stickstoff gegebenenfalls durch Niederalkyl, das seinerseits durch Phenyl, Pyrryl, Furyl, Thienyl und/oder Pyridyl, wobei diese Reste selbst gegebenenfalls Niederalkyl, Hydroxy, Niederalkoxy, Halogen und/oder Nitro als Substituenten enthalten, und oder durch Hydroxy, Niederalkoxy, Niederalkanoyloxy, Amino, Niederalkylamino und/oder Diniederalkylamino substituiert sein kann; durch Benzoyl, Niederalkanoyl, Furanylcarbonyl, Thienylcarbonyl, Pyrrylcarbonyl,

Pyridinylcarbonyl oder Phenyl, welches seinerseits Niederalkyl, Halogen, Niederalkoxy und/oder Nitro als Substituenten enthalten kann, substituiert sind, optische Isomere von Verbindungen der Formel I, Mischungen dieser optischen Isomere und Salze von solchen Verbindungen mit salzbildenden Gruppen, herstellt.

4. Verfahren gemäss Anspruch 1, dadurch gekennzeichnet, dass man Verbindungen der Formel I, worin n für 1 oder 2 steht, Ar für Phenyl, Thienyl, Furyl oder Benzoxadiazolyl steht, wobei diese Gruppen gegebenenfalls durch Niederalkyl, Niederalkoxy, Halogenniederalkoxy, Phenylniederalkoxy, Phenylniederalkylthio, Niederalkylendioxy, Halogen, Trifluormethyl, Nitro, Niederalkanoylamino und/oder Cyan mono-, di- oder trisubstituiert sind,der Rest Ac Niederalkanoyl, Niederalkylsulfonyl, Niederalkoxycarbonyl, 2-Niederalkoxy-niederalkoxycarbonyl, N,N-Diniederalkylamino-niederalkoxycarbonyl, N-Niederalkyl-N-phenylniederalkylamino-niederalkoxycarbonyl, Carbamoyl, N-Niederalkyl--carbamoyl, N,N-Diniederalkyl-carbamoyl, N,N-Niederalkylen-carbamoyl oder 4-Morpholinocarbonyl bedeutet, Z für einen Rest $-OR_7$ oder $-NR_8R_9$ steht, $R_1$ Wasserstoff, Niederalkyl, 2-(Diniederalkylamino)-niederalkyl, 2-(Niederalkylenamino)-niederalkyl, 2-(4-Morpholino)-niederalkyl, Carboxyniederalkyl oder Niederalkoxyniederalkoxy-niederalkyl bedeutet, $R_2$ und $R_3$ unabhängig voneinander Niederalkyl, Hydroxyniederalkyl, Cyan oder Amino bedeuten, $R_4$ Wasserstoff oder Niederalkyl darstellt, $R_5$ Wasserstoff, unsubstituiertes oder durch Hydroxy, Niederalkoxy, Mercapto, Niederalkylthio, Carboxy, Carbamoyl, Amino, Carbamoylamino, Guanidino, Phenyl, welches seinerseits durch Hydroxy substituiert sein kann, oder Imidazol-4-yl oder Indol-3-yl substituiertes Niederalkyl oder Phenyl, Thiazolyl, Imidazolyl, Furyl, Thienyl, Pyridyl oder Pyrimidinyl, wobei diese Reste gegebenenfalls durch Hydroxy, Niederalkoxy und/oder Amino substituiert sind, bedeutet, $R_6$ Wasserstoff, Niederalkyl, Phenylniederalkyl, welches unsubstituiert oder durch Hydroxy, Niederalkoxy und/oder Amino substituiert ist, oder Phenyl, Thiazolyl, Imidazolyl,

Furyl, Thienyl, Pyridyl oder Pyrimidinyl, wobei diese Reste gegebenenfalls wie ein Phenylniederalkyl-Rest $R_6$ substituiert sind, bedeutet, und $R_7$, $R_8$ und $R_9$ unabhängig voneinander Wasserstoff oder unsubstituiertes oder durch Amino, Niederalkylamino, Diniederalkylamino, Niederalkylenamino, Oxa- oder Azaniederalkylenamino, worin der Aza-Stickstoff gegebenenfalls durch Niederalkyl substituiertist, Hydroxy, Niederalkoxy, Phenyl, welches unsubstituiert oder seinerseits durch Amino, Hydroxy, Niederalkoxy und/oder Halogen substituiert sein kann, oder durch Thienyl oder Pyridyl, wobei diese Gruppen in gleicher Weise wie Phenyl substituiert sein können, substituiertes Niederalkyl bedeuten, worin $R_4$ und $R_5$ zusammen $C_3$-$C_4$-Niederalkylen bedeuten können, worin $R_5$ und $R_6$ zusammen $C_2$-$C_5$-Niederalkylen oder $C_4$-Azaniederalkylen bedeuten können und worin $R_8$ und $R_9$ zusammen $C_4$-$C_5$-Niederalkylen, das gegebenenfalls durch 2-Imidazolidinon-1-yl, welches in 3-Stellung Phenyl oder Niederalkyl als Substituenten enthalten kann, substituiert ist, $C_4$-Oxa- oder $C_4$-Azaniederalkylen bedeuten können, wobei der Aza-Stickstoff durch Niederalkyl, das seinerseits unsubstituiert oder durch Phenyl, welches Niederalkyl, Niederalkoxy, Halogen und/oder Nitro als Substituenten enthalten kann, Thienyl und/oder Pyridyl mono- oder disubstituiert ist,oder durch Benzoyl, Furanylcarbonyl, Phenyl oder Niederalkoxyphenyl substituiert sein kann, optische Isomere von Verbindungen der Formel I, Mischungen dieser optischen Isomere und pharmazeutisch verwendbare Salze von solchen Verbindungen mit salzbildenden Gruppen herstellt.

5. Verfahren gemäss Anspruch 1, dadurch gekennzeichnet, dass man Verbindungen der Formel I, worin n für 1 oder 2 steht, Ar für unsubstituiertes oder durch Niederalkyl, Niederalkoxy, Halogenniederalkoxy, Benzyloxy, Benzylthio, Halogen, Trifluormethyl, Nitro und/oder Cyan mono- oder disubstituiertes Phenyl, 2- oder 3-Thienyl oder 2,1,3-Benzoxadiazol-4-yl steht, der Rest Ac Niederalkylsulfonyl oder Niederalkoxycarbonyl darstellt, Z für einen Rest $-OR_7$ oder $-NR_8R_9$ steht, $R_1$ Wasserstoff,

ferner 2-(4-Morpholino)-äthyl bedeutet, $R_2$ Niederalkyl, Hydroxymethyl, Cyan oder Amino bedeutet, $R_3$ für Niederalkyl steht, $R_4$ Wasserstoff oder Niederalkyl darstellt, $R_5$ Wasserstoff, unsubstituiertes oder durch Phenyl, welches seinerseits durch Hydroxy substituiert sein kann, substituiertes Niederalkyl, Phenyl oder unsubstituiertes oder Amino-substituiertes Thiazolyl bedeutet, $R_6$ Wasserstoff, Niederalkyl oder Phenyl bedeutet, $R_7$ für Wasserstoff, Niederalkyl oder Niederalkyl, das durch Phenyl, Amino, Niederalkylamino, Diniederalkylamino, 4-Morpholino, 1-Piperazino, welches seinerseits am 4-Stickstoff Niederalkyl als Substituenten enthalten kann, oder Niederalkoxy substituiert ist, steht, $R_8$ und $R_9$ unabhängig voneinander Wasserstoff, unsubstituiertes oder durch Niederalkoxy, Phenyl oder Pyridyl substituiertes Niederalkyl bedeuten, worin $R_4$ und $R_5$ zusammen 1,3-Propylen bedeuten können, worin $R_5$ und $R_6$ zusammen für 1,5-Pentylen stehen können, und worin $R_8$ und $R_9$ zusammen 1,5-Pentylen, 3-(3-Phenyl-2-imidazolidinon-1-yl)-1,5-pentylen, 3-(2-Imidazolidinon-1-yl)-1,5-pentylen oder 3-Oxa- oder 3-Aza-1,5-pentylen bedeuten können, wobei der Aza-Stickstoff gegebenenfalls durch Niederalkyl, Benzyl, Diphenylmethyl, wobei die Reste Benzyl oder Diphenylmethyl gegebenenfalls Halogen, Niederalkyl und/oder Niederalkoxy als Substituenten enthalten, Benzoyl, Furanylcarbonyl, Phenyl oder Niederalkoxyphenyl, substituiert ist, optische Isomere von Verbindungen der Formel I, Mischungen dieser optischen Isomere und pharmazeutisch verwendbare Salze von solchen Verbindungen mit salzbildenden Gruppen herstellt.

6. Verfahren gemäss Anspruch 1, dadurch gekennzeichnet, dass man Verbindungen der Formel I, worin n für 1 steht,
Ar 2- oder 3-Nitro-phenyl, 2- oder 3-Difluormethoxy-phenyl, 2- oder 3-Methyl-phenyl, 2- oder 3-Trifluormethyl-phenyl, 2,3-Dimethyl-phenyl, 2,3-Dichlor-phenyl, 2- oder 3-Benzyloxy-phenyl oder 2- oder 3-Benzylthiophenyl, bedeutet, Ac Niederalkoxycarbonyl darstellt, Z für einen Rest $-OR_7$ oder $-NR_8R_9$ steht, $R_1$ Wasserstoff ist, $R_2$ und $R_3$ unabhängig

voneinander Niederalkyl bedeuten, $R_4$ Wasserstoff ist, $R_5$ Niederalkyl oder Phenyl bedeutet, $R_6$ Wasserstoff darstellt, $R_7$ unsubstituiertes oder durch 4-Morpholino, 1-Piperazino oder 4-Niederalkyl-1-Piperazino substituiertes Niederalkyl bedeutet, $R_8$ und $R_9$ unabhängig voneinander für Wasserstoff, unsubstituiertes oder durch Phenyl oder Pyridyl substituiertes Niederalkyl stehen, und worin $R_8$ und $R_9$ zusammen 3-Aza-1,5-Pentylen, in dem der Aza-Stickstoff gegebenenfalls durch Benzyl oder Diphenylmethyl substituiert ist, bedeuten können, optische Isomere von Verbindungen der Formel I, Mischungen dieser optischen Isomere und pharmazeutisch verwendbare Salze von solchen Verbindungen mit salzbildenden Gruppen, herstellt.

7. Verfahren gemäss Anspruch 1, dadurch gekennzeichnet, dass man Verbindungen der Formel I, worin am $C_4$-Kohlenstoff des 1,4-Dihydropyridingerüsts die folgende Konfiguration vorliegt, bei der die Kohlenstoffatome C3, C4 und C5 in der Papierebene, der Rest Ar vor und das Wasserstoffatom hinter der Papierebene liegt, herstellt:

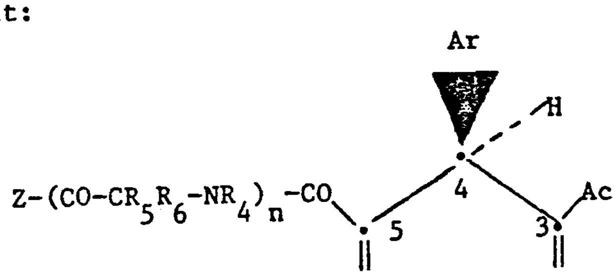

8. Verfahren gemäss Anspruch 1, dadurch gekennzeichnet, dass man (-)-(4R)-1,4-Dihydro-2,6-dimethyl-3-methoxycarbonyl-4-(3-nitrophenyl)-pyridin-5-carbonsäure-N-[(1S)-1-äthoxycarbonyl-2-methyl-1-propyl]-amid oder pharmazeutisch annehmbare Salze davon herstellt.

9. Verfahren gemäss Anspruch 1, dadurch gekennzeichnet, dass man (-)-(4R)-1,4-Dihydro-2,6-dimethyl-3-isopropoxycarbonyl-4-(3-nitro-phenyl)-pyridin-5-carbonsäure-N-[(1S)-1-äthoxycarbonyl-2-methyl-1-propyl)-amid oder pharmazeutisch annehmbare Salze davon herstellt.

10. Verfahren gemäss Anspruch 1, dadurch gekennzeichnet, dass man (-)-(4R)-1,4-Dihydro-2,6-dimethyl-3-methoxycarbonyl-4-(3-nitrophenyl)-pyridin-5-carbonsäure-N-[(1S)-1-(n-butoxycarbonyl)-2-methyl-1-propyl]-amid oder pharmazeutisch annehmbare Salze davon herstellt.

11. Verfahren gemäss Anspruch 1, dadurch gekennzeichnet, dass man (4R,4S)-1,4-Dihydro-2,6-dimethyl-3-methoxycarbonyl-4-(3-nitrophenyl)-pyridin-5-carbonsäure-N-[(1S,2S)-1-methoxycarbonyl-2-methyl-1-butyl]-amid, pharmazeutisch annehmbare Salze oder die optischen Isomere davon herstellt.

12. Verfahren gemäss Anspruch 1, dadurch gekennzeichnet, dass man (4R)-1,4-Dihydro-2,6-dimethyl-3-methoxycarbonyl-4-(3-nitrophenyl)-pyridin-5-carbonsäure-N-[(1S,2S)-1-äthoxycarbonyl-2-methyl-1-butyl]-amid oder pharmazeutisch annehmbare Salze davon herstellt.

13. Verfahren gemäss Anspruch 1, dadurch gekennzeichnet, dass man (4S,4R)-1,4-Dihydro-2,6-dimethyl-3-methoxycarbonyl-4-(3-nitrophenyl)-pyridin-5-carbonsäure-N-{(1S)-1-[2-(4-morpholino)-1-äthoxy]-carbonyl-2-methyl-1-propyl}-amid, pharmazeutisch annehmbare Salze oder die optischen Isomere davon herstellt.

14. Verfahren gemäss Anspruch 1, dadurch gekennzeichnet, dass man 1,4-Dihydro-2,6-dimethyl-3-methoxycarbonyl-4-(3-nitrophenyl)-pyridin-5-carbonsäure-N-[1-(4-benzyl-1-piperazinyl)-carbonyl-2-methyl-1-propyl]-amid, pharmazeutisch annehmbare Salze oder die optischen Isomere davon herstellt.

15. Verfahren gemäss Anspruch 1, dadurch gekennzeichnet, dass man (-)-(4R)-1,4-Dihydro-2,6-dimethyl-3-äthoxycarbonyl-4-(3-nitrophenyl)-pyridin-5-carbonsäure-N-[(1S)-1-äthoxycarbonyl-2-methyl-1-propyl]-amid oder pharmazeutisch annehmbare Salze davon herstellt.

16. Verfahren gemäss Anspruch 1, dadurch gekennzeichnet, dass man (4S,4R)-1,4-Dihydro-2,6-dimethyl-3-methoxycarbonyl-4-(3-nitrophenyl)-pyridin-5-carbonsäure-N-[(1S)-1-(4-diphenylmethyl-1-piperazinyl)-carbonyl-2-methyl-1-propyl]-amid, pharmazeutisch annehmbare Salze oder die optischen Isomere davon herstellt.

17. Verfahren zur Herstellung von Verbindungen der Formel I gemäss Anspruch 1, worin n für 1, 2 oder 3 steht, Ar einen carbocyclischen oder heterocyclischen Arylrest bedeutet, Ac den Acylrest einer Säure darstellt, Z für einen Rest $-OR_7$ steht, $R_1$ und $R_7$ Wasserstoff oder unsubstituiertes oder substituiertes Niederalkyl bedeuten, $R_2$ und $R_3$ Niederalkyl bedeuten, $R_4$ Wasserstoff oder Niederalkyl bedeutet, $R_5$ Wasserstoff, unsubstituiertes oder substituiertes Niederalkyl, unsubstituiertes oder substituiertes Phenyl oder einen heterocyclischen Arylrest bedeutet, und $R_6$ Wasserstoff, Niederalkyl, gegebenenfalls im Phenylring substituiertes Phenylniederalkyl, unsubstituiertes oder substituiertes Phenyl oder einen heterocyclischen Arylrest bedeutet, worin $R_4$ und $R_5$ zusammen unsubstituiertes oder substituiertes Niederalkylen, Oxaniederalkylen oder Thianiederalkylen sein können und worin $R_5$ und $R_6$ zusammen unsubstituiertes oder substituiertes Niederalkylen, worin ein Kohlenstoffatom durch ein Heteroatom ersetzt sein kann, sein können, und Salzen von solchen Verbindungen, die eine salzbildende Gruppierung aufweisen, dadurch gekennzeichnet, dass man

a) eine Verbindung der Formel II

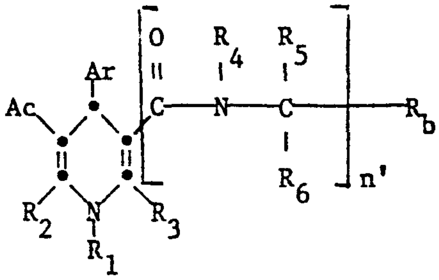

(II),

worin n' für 0, 1 oder 2 steht und $R_b$ eine gegebenenfalls aktivierte Carboxygruppe bedeutet, mit der Massgabe, dass $R_b$ von einem Rest $C(=O)-OR_7$ verschieden ist, wenn n' für 1 oder 2 steht, mit einer Verbindung der Formel III

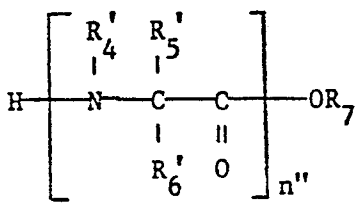

(III) ,

worin n" für 1, 2 oder 3 steht, und $R_4'$, $R_5'$ und $R_6'$ die unter Formel I für $R_4$, $R_5$ und $R_6$ angegebene Bedeutung haben, jedoch von den Resten $R_4$, $R_5$ bzw. $R_6$ in Formel II verschieden sein können, umsetzt, mit der Massgabe, dass die Summe aus n' und n" nicht grösser als 3 ist, oder

a') in einer Verbindung der Formel IIa

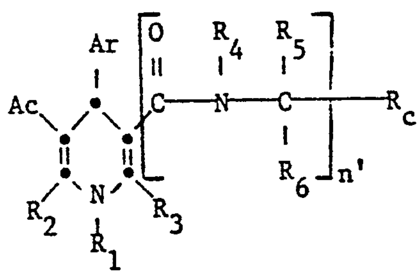

(IIa),

worin n' für 1, 2 oder 3 steht und $R_c$ eine in den Rest $C(=O)-OR_7$ überführbare Gruppe ist, diese in den Rest $C(=O)-OR_7$ überführt, oder

b) eine Verbindung der Formel IV, oder ein reaktionsfähiges Derivat davon, mit einer Verbindung der Formel V, oder einem Tautomeren davon, und mit einer Verbindung der Formel VI umsetzt,

(IV)

(VI) 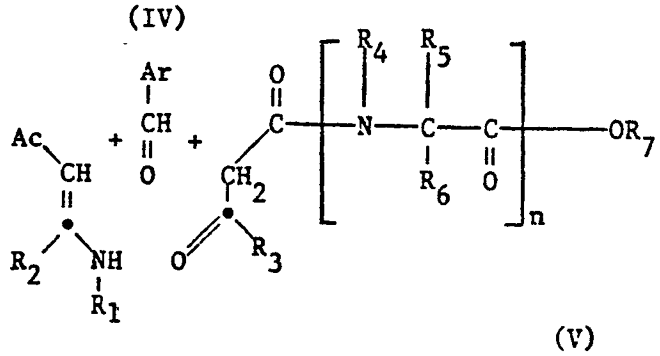

(V)

oder

c) eine Verbindung der Formel VII

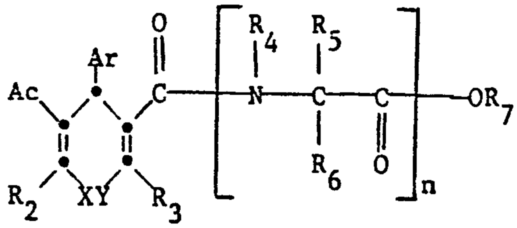 (VII) ,

worin einer der Reste X und Y für die Gruppe der Formel $-NH-R_1$ steht und der andere Hydroxy ist oder beide die Gruppe der Formel $-NH-R_1$ bedeuten, oder ein Tautomeres davon oder ein entsprechendes Tautomerengemisch ringschliesst, oder

d) eine Verbindung der Formel Ar-CHO (IV) oder ein reaktionsfähiges funktionelles Derivat davon mit einer Verbindung der Formel VIII

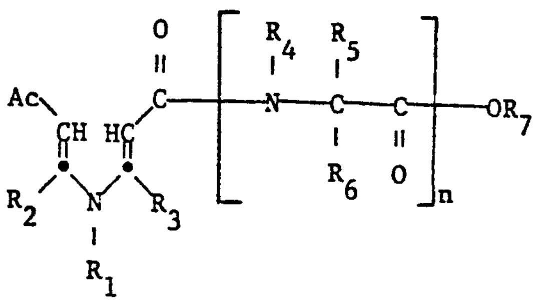 (VIII)

oder einem Tautomeren davon oder einem entsprechenden Tautomerengemisch umsetzt, oder

e) in einer Verbindung der Formel IX

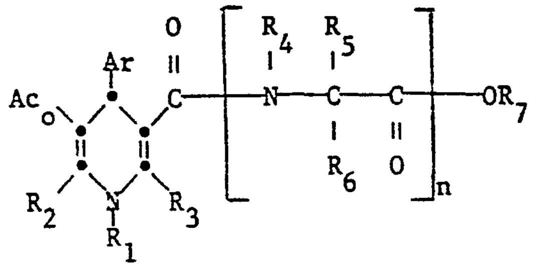

(IX) ,

worin $Ac_o$ einen in die Gruppe Ac überführbaren Rest darstellt, diesen in die Gruppe Ac überführt,

wobei in den obigen Ausgangsstoffen der Formeln II bis IX und IIa, die, sofern sie salzbildende Eigenschaften aufweisen, auch in Form ihrer Salze verwendet werden können, die Symbole n, Ar, Ac, $R_1$, $R_2$, $R_3$, $R_4$, $R_5$, $R_6$ und $R_7$ die unter der Formel I gegebenen Bedeutungen haben, und, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt, und/oder, wenn erwünscht, ein erhaltenes Gemisch von Stereoisomeren in die einzelnen Stereoisomeren auftrennt.

18. Verfahren gemäss Anspruch 17, dadurch gekennzeichnet, dass man Verbindungen der Formel I, worin n für 1, 2 oder 3 steht, Ar für Phenyl, das gegebenenfalls durch Niederalkyl, Phenyl und/oder Phenylniederalkyl, wobei solche Reste ihrerseits Hydroxy, Niederalkoxy, Niederalkylendioxy, Halogen, Carboxy, Niederalkoxycarbonyl und/oder Cyan, die cyclischen Reste auch Niederalkyl als Substituenten enthalten können, und/oder durch Hydroxy, Niederalkoxy, Halogen-niederalkoxy, Niederalkylendioxy, Halogen, Nitro, Amino, N-Niederalkylamino, N,N-Diniederalkylamino, Niederalkanoylamino, Carboxy, Niederalkoxycarbonyl, Carbamoyl, Cyan, Sulfo, Aminosulfonyl, Niederalkylthio, Niederalkylsulfinyl und/oder Niederalkylsulfonyl substituiert sein kann, oder für Pyrryl, Furyl, Thienyl oder Pyridyl, die gegebenenfalls wie ein Phenylrest Ar substituiert sein können, und insbesondere Niederalkyl, Niederalkoxy, Halogen und/oder gegebenenfalls durch Niederalkyl, Niederalkoxy, Halogen und/oder Nitro substituiertes Phenyl als Substituenten enthalten, steht, der Rest Ac für Niederalkanoyl, Niederalkylsulfonyl, Niederalkoxycarbonyl, Hydroxyniederalkoxycarbonyl, Niederalkoxyniederalkoxycarbonyl, N,N-Diniederalkylaminoniederalkoxycarbonyl, N-Niederalkyl-N-phenylniederalkyl-amino-niederalkoxycarbonyl, N,N-Niederalkylenaminoniederalkoxycarbonyl, (4-Morpholino)-niederalkoxycarbonyl, Carbamoyl, N-Niederalkyl-carbamoyl, N,N-Diniederalkyl-carbamoyl, N-N-Niederalkylencarbamoyl, 4-Niederalkyl-piperazino-carbonyl oder 4-Morpholinocarbonyl steht, Z für einen Rest $-OR_7$ steht, $R_1$ Wasserstoff, Niederalkyl, Diniederalkylaminoniederalkyl, Niederalkylenaminoniederalkyl, (4-Morpholino)-niederalkyl, Carboxy-niederalkyl oder Niederalkoxyniederalkoxy-niederalkyl darstellt, wobei Diniederalkylamino, Niederalkylenamino bzw. 4-Morpholino durch mindestens zwei Kohlenstoffatome vom Ringstickstoffatom getrennt sind, $R_2$ und $R_3$ Niederalkyl bedeuten, $R_4$ Wasserstoff oder Niederalkyl bedeutet, $R_5$ Wasserstoff, unsubstituiertes oder durch Hydroxy, Niederalkoxy, Mercapto, Niederalkylthio, Carboxy, Carbamoyl, Amino, Niederalkanoylamino, Carbamoylamino, Guanidino, durch Phenyl, welches seinerseits durch Hydroxy und/oder Halogen substituiert sein kann, Imidazolyl oder Indolyl substituiertes Niederalkyl, unsubstituiertes oder durch Hydroxy, Niederalkoxy, Amino, N-Niederalkylamino oder N,N-Diniederalkylamino substituiertes Phenyl oder unsubstituiertes oder in gleicher Weise wie für Phenyl angegeben substituiertes Pyrryl, Pyrazolyl, Imidazolyl, Triazolyl, Tetrazolyl, Furyl, Thienyl, Isoxazolyl, Oxazolyl, Oxadiazolyl, Isothiazolyl, Thiazolyl, Thiadiazolyl, Pyridyl, Pyridazinyl, Pyrimidinyl, Pyrazinyl oder Triazinyl bedeutet, $R_6$ Wasserstoff, Niederalkyl, Phenylniederalkyl, unsubstituiertes oder durch Hydroxy, Niederalkoxy, Amino,

N-Niederalkylamino oder N,N-Diniederalkylamino substituiertes Phenyl oder unsubstituiertes oder in gleicher Weise wie für Phenyl angegeben substituiertes Pyrryl, Pyrazolyl, Imidazolyl, Triazolyl, Tetrazolyl, Furyl, Thienyl, Isoxazolyl, Oxazolyl, Oxadiazolyl, Isothiazolyl, Thiazolyl, Thiadiazolyl, Pyridyl, Pyridazinyl, Pyrimidinyl, Pyrazinyl oder Triazinyl bedeutet, und $R_7$ Wasserstoff oder unsubstituiertes oder durch Amino, N-Niederalkylamino, N,N-Diniederalkylamino, Niederalkylenamino, Oxa-, Thia- oder gegebenenfalls azasubstituiertes Azaniederalkylenamino, Acylamino oder durch Phenyl, welches unsubstituiert oder seinerseits durch unsubstituiertes, mono- oder disubstituiertes Amino, Hydroxy, Niederalkoxy oder Halogen substituiert sein kann, substituiertes Niederalkyl bedeutet, worin $R_4$ und $R_5$ zusammen unsubstituiertes oder hydroxy-substituiertes Niederalkylen, Oxa- oder Thianiederalkylen bedeuten können und worin $R_5$ und $R_6$ zusammen Niederalkylen oder Azaniederalkylen sein können, wobei diese Reste unsubstituiert oder durch Hydroxy, Niederalkoxy, Amino, N-Niederalkylamino oder N,N-Diniederalkylamino substituiert sein können, wobei Niederalkyl, Niederalkoxy und Niederalkanoyl bis und mit 4 Kohlenstoffatome, Niederalkylen 3, 4 oder 5 Kettenkohlenstoffatome enthalten, und Salze von solchen Verbindungen mit salzbildenden Gruppen herstellt.

19. Verfahren gemäss Anspruch 17, dadurch gekennzeichnet, dass man Verbindungen der Formel I, worin n für 1 oder 2 steht, Ar für Phenyl, Thienyl oder Furyl steht, das gegebenenfalls durch Niederalkyl, Hydroxy, Niederalkoxy, Halogen-niederalkoxy, Niederalkylendioxy, Halogen, Trifluormethyl, Nitro, Niederalkanoylamino und/oder Cyan mono-, di- oder trisubstituiert sein kann, der Rest Ac Niederalkanoyl, Niederalkylsulfonyl, Niederalkoxycarbonyl, 2-Niederalkoxy-niederalkoxycarbonyl, Diniederalkylamino-niederalkoxycarbonyl, N-Niederalkyl-N-phenylniederalkyl-amino-niederalkoxycarbonyl, Carbamoyl, N-Niederalkyl-carbamoyl, N,N-Diniederalkylcarbamoyl, N,N-Niederalkylenamino-carbonyl oder 4-Morpholino-carbonyl bedeutet, Z für

einen Rest $-OR_7$ steht, $R_1$ Wasserstoff, Niederalkyl, 2-(Diniederalkylamino)-niederalkyl, 2-(Niederalkylenamino)-niederalkyl, 2-(Morpholino)-niederalkyl, Carboxyniederalkyl oder Niederalkoxyniederalkoxyniederalkyl bedeutet, $R_2$ und $R_3$ Niederalkyl bedeuten, $R_4$ Wasserstoff darstellt, $R_5$ Wasserstoff, unsubstituiertes oder durch Hydroxy, Mercapto, Niederalkylthio, Carboxy, Carbamoyl, Amino, Carbamoylamino, Guanidino, durch Phenyl, welches seinerseits durch Hydroxy substituiert sein kann, Imidazol-4-yl oder Indol-3-yl substituiertes Niederalkyl oder unsubstituiertes oder durch Hydroxy, Niederalkoxy oder Amino substituiertes Phenyl, Thiazolyl, Imidazolyl, Furyl, Thienyl, Pyridyl oder Pyrimidinyl bedeutet, $R_6$ Wasserstoff, Niederalkyl, Phenylniederalkyl oder unsubstituiertes oder durch Hydroxy, Niederalkoxy oder Amino substituiertes Phenyl, Thiazolyl, Imidazolyl, Furyl, Thienyl, Pyridyl oder Pyrimidinyl bedeutet, und $R_7$ Wasserstoff oder unsubstituiertes oder durch Amino, N-Niederalkylamino, N,N-Diniederalkylamino oder Phenyl, welches unsubstituiert oder seinerseits durch Amino, Hydroxy, Niederalkoxy oder Halogen substituiert sein kann, substituiertes Niederalkyl bedeutet, worin $R_4$ und $R_5$ zusammen Niederalkylen bedeuten können und worin $R_5$ und $R_6$ zusammen Niederalkylen oder Azaniederalkylen bedeuten können, wobei Niederalkyl, Niederalkoxy und Niederalkanoyl bis und mit 4 Kohlenstoffatome, Niederalkylen 3, 4 oder 5 Kettenkohlenstoffatome enthalten, und Salze von solchen Verbindungen mit salzbildenden Gruppen herstellt.

20. Verfahren gemäss Anspruch 17, dadurch gekennzeichnet, dass man Verbindungen der Formel I, worin n für 1 oder 2 steht, Ar für 2- oder 3-Nitro-phenyl, 2- oder 3-Difluormethoxy-phenyl, 2,3-Dimethyl-phenyl oder 2- oder 3-Trifluormethyl-phenyl steht, der Rest Ac Niederalkoxycarbonyl darstellt, $R_1$ Wasserstoff bedeutet, $R_2$ und $R_3$ Methyl bedeuten, $R_4$ Wasserstoff darstellt, $R_5$ Wasserstoff, unsubstituiertes oder durch Phenyl, welches seinerseits durch Hydroxy substituiert sein kann, substituiertes Niederalkyl oder Phenyl bedeu-

tet, $R_6$ Wasserstoff oder Niederalkyl bedeutet, und $R_7$ für Wasserstoff, Niederalkyl oder Phenylniederalkyl steht, worin $R_4$ und $R_5$ zusammen 1,3-Propylen bedeuten können, und worin $R_5$ und $R_6$ zusammen für 1,5-Pentylen stehen können, wobei Niederalkyl, Niederalkoxy und Niederalkanoyl bis und mit 4 Kohlenstoffatome enthalten, und Salze von solchen Verbindungen mit salzbildenden Gruppen herstellt.

21. Verfahren gemäss Anspruch 17, dadurch gekennzeichnet, dass man (-)-(4R)-1,4-Dihydro-2,6-dimethyl-3-methoxycarbonyl-4-(3-nitrophenyl)-pyridin-5-carbonsäure-N-[(1S)-1-äthoxycarbonyl-2-methyl-1-propyl]-amid oder pharmazeutisch annehmbare Salze davon herstellt.

22. Verfahren gemäss Anspruch 17, dadurch gekennzeichnet, dass man (-)-(4R)-1,4-Dihydro-2,6-dimethyl-3-isopropoxycarbonyl-4-(3-nitro-phenyl)-pyridin-5-carbonsäure-N-[(1S)-1-äthoxycarbonyl-2-methyl-1-propyl)-amid oder pharmazeutisch annehmbare Salze davon herstellt.

23. Verfahren gemäss Anspruch 17, dadurch gekennzeichnet, dass man (-)-(4R)-1,4-Dihydro-2,6-dimethyl-3-methoxycarbonyl-4-(3-nitro-phenyl)-pyridin-5-carbonsäure-N-[(1S)-1-(n-butoxycarbonyl)-2-methyl-1-propyl]-amid oder pharmazeutisch annehmbare Salze davon herstellt.

24. Verfahren gemäss Anspruch 17, dadurch gekennzeichnet, dass man (4R,4S)-1,4-Dihydro-2,6-dimethyl-3-methoxycarbonyl-4-(3-nitro-phenyl)-pyridin-5-carbonsäure-N-[(1S,2S)-1-methoxycarbonyl-2-methyl-1-butyl]-amid, pharmazeutisch annehmbare Salze oder die optischen Isomere davon herstellt.

25. Verfahren gemäss Anspruch 17, dadurch gekennzeichnet, dass man (4R)-1,4-Dihydro-2,6-dimethyl-3-methoxycarbonyl-4-(3-nitrophenyl)-pyridin-5-carbonsäure-N-[(1S,2S)-1-äthoxycarbonyl-2-methyl-1-butyl]-amid oder pharmazeutisch annehmbare Salze davon herstellt.

26. Verfahren zur Herstellung von pharmazeutischen Präparaten, dadurch gekennzeichnet, dass man eine nach dem Verfahren gemäss Anspruch 1 erhaltene Verbindung der Formel I oder ein pharmazeutisch verwendbares Salz einer solcher Verbindung mit einem pharmazeutisch verwendbaren Trägermaterial mischt.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

0145956

Nummer der Anmeldung

EP 84113706.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP - A1 - 0 069 033 (RHONE-POULENC) * Ansprüche 1,2,7 * -- | 1,25 | C 07 D 211/90<br>C 07 D 401/06<br>C 07 D 401/12<br>C 07 D 405/12<br>C 07 D 417/12<br>A 61 K 31/44 |
| D,A | EP - A1 - 0 011 706 (BAYER) * Ansprüche 6,9 * -- | 1,24 | |
| A | EP - A1 - 0 052 300 (BAYER) * Ansprüche 1,4c,6; Beispiel 21 * ---- | 1,21, 25 | |

**RECHERCHIERTE SACHGEBIETE (Int. Cl.4)**

C 07 D 211/00
C 07 D 401/00
C 07 D 405/00
C 07 D 417/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-25

Unvollständig recherchierte Patentansprüche: -

Nicht recherchierte Patentansprüche: 26

Grund für die Beschränkung der Recherche:

Unklarheiten

| Recherchenort | Abschlußdatum der Recherche | Prüfer |
|---|---|---|
| WIEN | 31-01-1985 | HOCHHAUSER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 08.82